# EUROPEAN PATENT APPLICATION

(11) **EP 4 516 896 A2**
(43) Date of publication of application: **05.03.2025**
(21) Application number: 24207574.5
(22) Date of filing: 09.08.2018
(51) Int. Cl.: C12N 5/0783

(54) **METHODS AND COMPOSITIONS FOR PREPARING GENETICALLY ENGINEERED CELLS**

(30) Priority: 09.08.2017 US 201762543359 P
(62) Divisional of application: 18760160.4
(71) Applicant: Juno Therapeutics, Inc., Seattle, WA 98109 (US)
(72) Inventor: BONYHADI, Mark L., Seattle, WA 98109 (US)
(74) Representative: Carpmaels & Ransford LLP

(57) **Abstract**

Provided are methods for preparing T cells for cell therapy, compositions produced by the methods, and methods of administering the cells to subjects. In particular, the disclosure relates to preparation of engineered T cells, such as those expressing genetically engineered receptors, such as genetically engineered antigen receptors such as engineered (recombinant) TCRs and chimeric antigen receptors (CARs), or other recombinant chimeric receptors. Features of the methods include producing a more consistent and/or predictable T cell product and/or lower toxicity compared with other methods. The provided methods include incubating cells under stimulating conditions to induce expansion or proliferation of naive-like T cells compared to non-naive like T cells in the stimulated composition, which in turn can result in preferential transduction of cells derived from the naïve-like T cells. Features of the methods can also include reduction in costs, numbers of steps, and resource expenditure compared with other methods.

## Description

### Cross-Reference to Related Applications

This application claims priority from U.S. provisional application No. 62/543,359, filed August 9, 2017, entitled "METHODS AND COMPOSITIONS FOR PREPARING GENETICALLY ENGINEERED CELLS," the contents of which are incorporated by reference in their entirety.

### Incorporation by Reference of Sequence Listing

The present application is being filed along with a Sequence Listing in electronic format. The Sequence Listing is provided as a file entitled 735042010540SEQLIST.txt, created July 11, 2018, which is 35,434 kilobytes in size. The information in the electronic format of the Sequence Listing is incorporated by reference in its entirety.

### Field

The present disclosure relates to methods for preparing T cells for cell therapy, compositions produced by the methods, and methods of administering the cells to subjects. In particular, the disclosure relates to preparation of engineered T cells, such as those expressing genetically engineered receptors, such as genetically engineered antigen receptors such as engineered (recombinant) TCRs and chimeric antigen receptors (CARs), or other recombinant chimeric receptors. Features of the methods include producing a more consistent and/or predictable T cell product and/or lower toxicity compared with other methods. The provided methods include incubating cells under stimulating conditions to induce expansion or proliferation of naïve-like T cells compared to non-naive like T cells in the stimulated composition, which in turn can result in preferential transduction of cells derived from the naive-like T cells. Features of the methods can also include reduction in costs, numbers of steps, and resource expenditure compared with other methods.

### Background

Various methods are available for preparing cells for therapeutic use and administering the cells. For example, methods are available for preparing cells, including T cells, for engineering and cell therapy, including methods involving depletion of or enrichment for certain sub-populations. Improved methods are needed, for example, to reduce toxicity associated with certain adoptive cell therapy administrations, to improve the manufacturing process, to allow improved administration, and/or to reduce cost or other resources. Provided are methods, cells, compositions, kits, and systems that meet such needs.

### Summary

Provided herein are methods for genetically engineering T cells, the method including incubating an input composition, under stimulating conditions, for between 2 and 6 days, said input composition comprising a population of T cells comprising naïve-like T cells and non-naive-like T cells, wherein the stimulating conditions comprises a stimulatory reagent capable of activating one or more intracellular signaling domains of one or more components of a TCR complex and/or one or more intracellular signaling domains of one or more costimulatory molecules, thereby generating a stimulated composition; and introducing a nucleic acid encoding a genetically engineered recombinant receptor into the stimulated composition of T cells, wherein the introducing is carried out during at least a portion of the incubating.

Provided herein are methods for genetically engineering T cells, the method comprising incubating an input composition, under stimulating conditions, for between 2 and 6 days, said input composition comprising a population of T cells comprising naïve-like T cells and non-naive-like T cells, wherein the stimulating conditions comprise the presence of a stimulatory reagent capable of activating one or more intracellular signaling domains of one or more components of a TCR complex and/or one or more intracellular signaling domains of one or more costimulatory molecules, thereby generating a stimulated composition; and the incubating the input composition under stimulating conditions is performed prior to, during and/or subsequent to introducing a nucleic acid encoding a genetically engineered recombinant receptor. In some embodiments, the incubating is carried out for at least 3 days. In some cases, the incubating is carried out for at least 4 days. In some embodiments, the incubating is carried out for at least 5 days. In some embodiments,the incubating is carried out for at least 6 days.

Provided herein are methods for stimulating T cells including (a) incubating, under stimulating conditions, an input composition containing T cells containing a culture-initiating amount of naïve-like T cells or a CD8+ T cell subset thereof, thereby producing a stimulated composition; and (b) introducing into the stimulated cell composition a nucleic acid encoding a genetically engineered recombinant receptor, wherein the method thereby generates an output composition containing T cells expressing the genetically engineered recombinant receptor. In some embodiments, the T cells contain naïve-like T cells and non-naive-like T cells, wherein the stimulating conditions preferentially induces expansion or proliferation of the naïve-like T cells compared to the non-naive like T cells in the stimulated composition. In some embodiments, the introducing is carried out during at least a portion of the incubating or is carried out subsequent to the incubating.

In some embodiments, the culture-initiating amount of naïve-like T cells or a CD8+ T cell subset thereof is from or from about 0.1 × 10⁸ to 5 × 10⁸, from or from about 0.1 × 10⁸ to 4 × 10⁸, from or from about 0.1 × 10⁸ to 2 × 10⁸, from or from about 0.1 × 10⁸ to 1 × 10⁸, from or from about 1 × 10⁸ to 5 × 10⁸ from or from about 1 × 10⁸ to 4 × 10⁸, from or from about 1 × 10⁸ to 2 × 10⁸, from or from about 2 × 10⁸ to 5 × 10⁸, from or from about 2 × 10⁸ to 4 × 10⁸ of the naïve-like T cells or a CD8+ T cell subset thereof. In some cases, the culture-initiating amount of naïve-like T cells or a CD8+ T cell subset thereof is at least or at least about or is or is about 0.5 × 10⁸, 0.75 × 10⁸, 1 × 10⁸, 1.5 × 10⁸, 2 × 10⁸, or 4 × 10⁸ of the naïve-like T cells or a CD8+ T cell subset thereof. In some instances, the culture-initiating amount of naïve-like T cells or a CD8+ T cell subset thereof is at least or at least about or is or is about 2 × 10⁸ of the naïve-like T cells or a CD8+ T cell subset thereof.

Provided are methods for stimulating T cells, the method including incubating, under stimulating conditions, an input composition including T cells containing a culture-initiating amount of naive-like T cells or a CD8+ T cell subset thereof of from or from about 1 × 10⁸ to 4 × 10⁸ naïve-like T cells or a CD8+ T cell subset thereof, thereby producing a stimulated composition. In some aspects, the T cells contain naïve-like T cells and non-naive-like T cells, wherein the stimulating conditions preferentially induces expansion or proliferation of the naïve-like T cells compared to the non-naive like T cells in the stimulated composition.

In some embodiments, the culture-initiating amount of naïve-like T cells or a CD8+ T cell subset thereof is at least or at least about or is or is about 2 × 10⁸ of the naïve-like T cells or a CD8+ T cell subset thereof. In some aspects, the culture initiating amount is an amount of naïve-like CD8+ T cells.

In some of any such embodiments, the naive-like T cells or naïve-like CD8+ T cells are surface positive for a T cell activation marker selected from the group consisting of CD45RA, CD27, CD28, and CCR7; and/or are surface negative for a marker selected from the group consisting of CD25, CD45RO, CD56, CD62L, KLRG1; and/or have low expression of CD95; and/or are negative for intracellular expression of a cytokine selected from the group consisting of IL-2, IFN-γ, IL-4, IL-10. In some embodiments, the naïve-like cells or the naive-like CD8+ cells are surface positive for a T cell activation marker selected from the group consisting of CD45RA, CD27, CD28, and CCR7; and/or are surface negative for a marker selected from the group consisting of CD45RO, CD56, KLRG1; and/or have low expression of CD95. In some embodiments, the naïve-like T cells or the naive-like CD8+ cells are CD45RA+, CD27+, CCR7+, CD62-, and/or CD45RO-.

In some of any such embodiments, the non-naive-like T cells are surface negative for a T cell activation marker selected from the group consisting of CD45RA, CD27, CD28, and CCR7; and/or are surface positive for a marker selected from the group consisting of CD25, CD45RO, CD56, CD62L, KLRG1, and perforin; and/or are positive intracellular expression of a cytokine selected from the group consisting of IL-2, IFN-γ, IL-4, IL-10; and/or have high expression of CD95. In some aspects, the non-naive-like T cells are CD45RA-, CD27-, CCR7-, CD62+, and/or CD45RO+.

In some embodiments, the cells of the input composition have not been and are not, prior to the incubation, subjected to a selection step based on an endogenous T cell surface marker that differentiates between naïve-like and non-naive-like T cells.

In some embodiments, the method further includes introducing a genetically engineered recombinant receptor into the stimulated cells, wherein the method thereby generates an output composition including T cells expressing the genetically engineered recombinant receptor. In some cases, the incubating the composition under stimulating conditions is performed prior to, during and/or subsequent to introducing a nucleic acid encoding a genetically engineered recombinant receptor.

In some embodiments, the recombinant receptor is capable of binding to a target antigen that is associated with, specific to, and/or expressed on a cell or tissue of a disease, disorder or condition. In some examples, the disease, disorder or condition is an infectious disease or disorder, an autoimmune disease, an inflammatory disease, or a tumor or a cancer. In some instances, the target antigen is a tumor antigen. In some embodiments, the target antigen is selected from among αvβ6 integrin (avb6 integrin),, B cell maturation antigen (BCMA), carbonic anhydrase 9 (CAIX), Her2/neu (receptor tyrosine kinase erbB2), L1-CAM, B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), and hepatitis B surface antigen, anti-folate receptor, a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), epithelial glycoprotein 2 (EPG-2), epithelial glycoprotein 40 (EPG-40), ephrinB2, ephrin receptor A2 (EPHa2), erb-B2, erb-B3, erb-B4, erbB dimers, type III epidermal growth factor receptor mutation (EGFR vIII), folate binding protein (FBP), Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glypican-3 (GPC3), G Protein Coupled Receptor 5D (GPRC5D), Her2/neu (receptor tyrosine kinase erbB2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha (IL-22R-alpha), IL-13R-alpha2 (IL-13Rα2), kinase insert domain receptor (kdr), kappa light chain, Lewis Y, L1-cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), folate receptor-a, 8H9, dual antigen, glycoprotein 100 (gp100), cular endothelial growth factor receptor (VEGFR), vascular endothelial growth factor receptor 2 (VEGF-R2), estrogen receptor, progesterone receptor, Wilms Tumor 1 (WT-1), a pathogen-specific or pathogen-expressed antigen and an antigen associated with a universal tag.

In some embodiments, the recombinant receptor is or contains a functional non-TCR antigen receptor or a TCR or antigen-binding fragment thereof. In some embodiments, the recombinant receptor is a chimeric antigen receptor (CAR).

In some of any such embodiments, the recombinant receptor contains an extracellular domain containing an antigen-binding domain, optionally wherein the antigen-binding domain specifically binds the target antigen. In some cases, the antigen-binding domain is or contains an antibody or an antibody fragment thereof, which optionally is a single chain fragment. In some aspects, the fragment contains antibody variable regions joined by a flexible linker. In some instances, the fragment contains an scFv.

In some embodiments, the recombinant receptor further contains a spacer and/or a hinge region. In some aspects, the recombinant receptor contains an intracellular signaling region. In some examples, the intracellular signaling region contains an intracellular signaling domain. In some aspects, the intracellular signaling domain is or contains a primary signaling domain, a signaling domain that is capable of inducing a primary activation signal in a T cell, a signaling domain of a T cell receptor (TCR) component, and/or a signaling domain containing an immunoreceptor tyrosine-based activation motif (ITAM). In some embodiments, the intracellular signaling domain is or contains an intracellular signaling domain of a CD3 chain, optionally a CD3-zeta (CD3ζ) chain, or a signaling portion thereof.

In some embodiments, the recombinant receptor further contains a transmembrane domain disposed between the extracellular domain and the intracellular signaling region. In some aspects, the intracellular signaling region further contains a costimulatory signaling region. In some embodiments, the costimulatory signaling region contains an intracellular signaling domain of a T cell costimulatory molecule or a signaling portion thereof. In some cases, the costimulatory signaling region contains an intracellular signaling domain of a CD28, a 4-1BB or an ICOS or a signaling portion thereof. In some embodiments, the CAR comprises an scFv specific for the antigen, a transmembrane domain, a cytoplasmic signaling domain derived from a costimulatory molecule, which optionally is or comprises a 4-1BB, and a cytoplasmic signaling domain derived from a primary signaling ITAM-containing molecule, which optionally is or comprises a CD3zeta signaling domain and optionally further comprises a spacer between the transmembrane domain and the scFv; the CAR comprises, in order, an scFv specific for the antigen, a transmembrane domain, a cytoplasmic signaling domain derived from a costimulatory molecule, which optionally is or comprises a 4-1BB signaling domain, and a cytoplasmic signaling domain derived from a primary signaling ITAM-containing molecule, which optionally is a CD3zeta signaling domain; or the CAR comprises, in order, an scFv specific for the antigen, a spacer, a transmembrane domain, a cytoplasmic signaling domain derived from a costimulatory molecule, which optionally is a 4-1BB signaling domain, and a cytoplasmic signaling domain derived from a primary signaling ITAM-containing molecule, which optionally is or comprises a CD3zeta signaling domain.

In some embodiments, the costimulatory signaling region is between the transmembrane domain and the intracellular signaling region.

In some of any such embodiments, the stimulating condition includes incubation with a stimulatory reagent capable of activating T cells, CD4+ T cells and/or CD8+ T cells; is capable of inducing a signal through a TCR complex; and/or is capable of inducing proliferation of T cells, CD4+ T cells and/or CD8+ T cells. In some embodiments, the stimulating condition include incubation with a stimulatory reagent capable of activating one or more intracellular signaling domains of one or more components of a TCR complex and/or one or more intracellular signaling domains of one or more costimulatory molecules. In some cases, the stimulatory reagent contains a primary agent that specifically binds to a member of a TCR complex, optionally that specifically binds to CD3. In some embodiments, the primary agent is an antibody or an antigen-binding fragment.

In some examples, the stimulatory agent further includes a secondary agent that specifically binds to a T cell costimulatory molecule, optionally wherein the costimulatory molecule is selected from the group consisting of CD28, CD137 (4-1-BB), OX40, or ICOS. In some embodiments, the primary agent is an antibody or an antigen-binding fragment. In some embodiments, the primary and secondary agents include antibodies, optionally wherein the one or more stimulating agent includes incubation with an anti-CD3 antibody and an anti-CD28 antibody.

In some embodiments, the primary and/or secondary are present on the surface of a solid support. In some cases, the solid support is or contains a bead. In some embodiments, the bead contains a diameter of greater than or greater than about 3.5 µm but no more than about 9 µm or no more than about 8 µm or no more than about 7 µm or no more than about 6 µm or no more than about 5 µm. In some examples, the bead contains a diameter of or about 4.5 µm. In some aspects, the bead contains a diameter that is or is about the same size as a lymphocyte or an antigen presenting cell.

In some embodiments, the bead is inert. In some cases, the bead is or contains a polystyrene surface, and optionally contains a magnetic or superparamagnetic core.

In some embodiments, the stimulating condition includes incubating the cells with a ratio of beads to cells that is from or from about 1:1 to 10:1, from or from about 1:1 to 8:1, from or from about 1:1 to 6:1, from or from about 1:1 to 4:1, from or from about 1:1 to 3:1, from or from about 2:1 to 4:1, from or from about 2:1 to 3:1, from or from about 1:1 to 2:1, from or from about 4:1 to 10:1, from or from about 4:1 to 8:1, from or from about 4:1 to 6:1, from or from about 6:1 to 10:1, from or from about 6:1 to 8:1, from or from about 8:1 to 10:1, from or from about 1:1 to 1:10, from or from about 1:1 to 1:8, from or from about 1:1 to 1:6, from or from about 1:1 to 1:4, from or from about 1:2 to 1:3. In some examples, the ratio of beads to cells is from or from about 3:1. In some embodiments, the ratio of beads to cells is from or from about 1:1.

Provided herein are methods for genetically engineering T cells including incubating an input composition, under stimulating conditions, for between 2 and 6 days, said input composition comprising a population of T cells comprising naïve-like T cells and non-naive-like T cells, wherein the stimulating conditions comprises a stimulatory reagent comprising an anti-CD3 antibody and a secondary agent that is an anti-CD28 antibody that is attached to a bead, wherein the ratio of beads to cells during the incubating is from or from about 1:1 to 4:1; and introducing a nucleic acid encoding a genetically engineered recombinant receptor into the stimulated composition of T cells, wherein the introducing is carried out during at least a portion of the incubating.

Provided herein are methods for genetically engineering T cells, the method comprising incubating an input composition, under stimulating conditions, for between 2 and 6 days, said input composition comprising a population of T cells comprising naïve-like T cells and non-naive-like T cells, wherein the stimulating conditions comprises a stimulatory reagent comprising an anti-CD3 antibody and a secondary agent that is an anti-CD28 antibody that is attached to a bead, wherein the ratio of beads to cells during the incubating is from or from about 1:1 to 4:1; and the incubating the input composition under stimulating conditions is performed prior to, during and/or subsequent to introducing a nucleic acid encoding a genetically engineered recombinant receptor.

In some of any such embodiments, the T cells are from a biological sample, optionally wherein the biological sample is from a human subject. In some cases, the biological sample is or contains a whole blood sample, a buffy coat sample, a peripheral blood mononuclear cells (PBMC) sample, an unfractionated T cell sample, a lymphocyte sample, a white blood cell sample, an apheresis product, or a leukapheresis product.

In some embodiments, the T cells contain CD4+ and/or CD8+ cells. In some embodiments, the T cells include CD4+ and CD8+ T cells and the ratio of CD4+ to CD8+ T cells is between at or about 2:1 and at or about 1:5. In some cases, a ratio of the CD4+ cells to the CD8+ cells is or is about 1:1, 1:2, 2: 1, 1:3, or 3:1. In some embodiments, the naïve-like T cells include naïve-like CD4+ T cells and/or naïve-like CD8+ T cells.

In some of any such embodiments, the naive-like T cells are polyclonal. In some cases, the clonality of the naïve-like T cells is determined by clonal sequencing, optionally next-generation sequencing, or spectratype analysis.

In some embodiments, the presence, amount, number or percentage of naïve-like T cells is detected by flow cytometry.

In some of any such embodiments, the stimulating condition does not include N-acetylcysteine (NAC). In some embodiments, the stimulating condition does not contain IL-15 and/or IL-7. In some embodiments, the stimulating condition results or induces death or the non-naïve like T cells or a subpopulation thereof. In some aspects, the stimulation condition results in activation-induced cell death (AICD) of non-naive like T cells or a subpopulation thereof.

In some embodiments, the method further includes adding DNAase during the incubation and/or to the stimulated composition.

In some embodiments, the incubation is carried out for greater than or about 1, 2, 3, 4, 5, 6, 7, 8, 9, 10, 11, 12, 13, 14, 15, or 16 days. In some embodiments, the percent of cells, in the stimulated composition, derived from the naïve-like T cells is increased greater than or greater than about 1.5-fold, 2-fold, 3-fold, 4-fold, 5-fold, 6-fold, 7-fold, 8-fold, 9-fold, 10-fold, 50-fold, 100-fold compared to the percent of naïve-like cells in the input composition. In some embodiments, the ratio, in the stimulated composition, of cells derived from the naïve-like T cells compared to cells derived from the non-naive-like T cells is increased greater than or greater than about 1.5-fold, 2-fold, 3-fold, 4-fold, 5-fold, 6-fold , 7-fold, 8-fold, 9-fold, 10-fold, 50-fold, 100-fold compared to the ratio of the naïve-like T cells compared to non-naive-like T cells in the input composition.

In some cases, the stimulated composition contains greater than 75%, 85%, 90%, 91%, 92%, 93%, 94%, 95%, 96%, 97%, 98%, 99% of cells that are derived from naïve-like T cells of the input composition. In some embodiments, the stimulated composition contains less than 10% of cells derived from the non-naive like T cells. In some examples, the stimulated composition contains less than 9%, 8%, 7%, 6%, 5%, 4%, 3%, 2%, 1%, 0.5% or 0.1% cells derived from the non-naive T cells.

In some embodiments, of the cells in the input composition, a greater percentage of the naïve-like T cells, as compared to the non-naive-like T cells, are induced to proliferate and/or become activated. In some aspects, a greater percentage of the T cells that were naïve-like in the input composition, as compared to the percentage of the T cells that were non-naïve-like in the input composition, are dividing at day 1, 2, 3, 4, 5, 6, 7, 8, 9, or 10 following initiation of said incubation. In some cases, the stimulating conditions are capable of inducing proliferation of a greater percentage of cells of a human naïve-like T cell population, as compared to human non-naive-like T cells, at day 1, 2, 3, 4, 5, 6, 7, 8, 9, or 10 following initiation of incubation under the conditions.

In some of any such embodiments, the non-naive-like T cells are selected from the group consisting of effector T (T_{EFF}) cells, memory T cells, central memory T cells (T_{CM}), effector memory T (T_{EM}) cells, and combinations thereof; or the non-naive-like T cells are a plurality of T cells including or consisting of effector T (T_{EFF}) cells and/or memory T cells, the memory T cells optionally containing central memory T cells (T_{CM}) and/or effector memory T (T_{EM}) cells.

In some embodiments, the percentage of naïve-like T cells in the input composition is less than the percentage of engineered cells in the stimulated composition derived from naïve-like T cells in the input composition. In some embodiments, a greater percentage of the cells introduced with the nucleic acid are, or are derived from the proliferation of, naïve-like T cells in the input composition, compared to non-naive-like T cells in the input composition.

In some of any such embodiments, the introduction is by transduction. In some embodiments, the nucleic acid contains a viral vector. In some cases, the viral vector is a retroviral vector. In some aspects, the viral vector is a lentiviral vector or a gammaretroviral vector. In some embodiments, the introduction is by transposition of a transposon containing the nucleic acid molecule. In some embodiments, the ratio of naïve-like T cells compared to non-naive-like T cells in the stimulated composition is increased greater than or greater than about 1.5-fold, 2-fold, 3-fold, 4-fold, 5-fold, 6-fold, 7-fold, 8-fold, 9-fold, 10-fold, 50-fold, 100-fold compared to the ratio of the naïve-like T cells compared to non-naive-like T cells in the input composition. In some embodiments, the stimulated composition is more polyclonal or multiclonal compared to the input composition. In some embodiments, the method is performed in vitro or ex vivo.

Provided are output compositions produced by any of the methods provided herein. Also provided are pharmaceutical compositions containing the output compositions described. In some embodiments, the pharmaceutical composition further contains a pharmaceutical carrier.

Provided are methods of treatment including administering to a mammalian subject an output composition produced by the any of the described methods or any of the pharmaceutical compositions described. In some embodiments, the cells are derived from the subject to which the cells are administered.

### Detailed Description

Provided herein are methods for incubating (e.g., stimulating) cells in the process of preparing cells for adoptive cell therapy, and compositions and cells produced by the methods. In some embodiments, the cells include T cells, which in some aspects express genetically engineered antigen receptors. In some embodiments, the genetically engineered antigen receptors include genetically engineered or recombinant T cell receptors (TCRs) and functional non-TCR antigen receptors such as chimeric antigen receptors (CARs).

In some embodiments, provided are methods for incubating (e.g., stimulating) T cells. Methods available have made use of anti-CD3 and anti CD28 for the stimulation of T cells. However, the methods currently available have not focused on obtaining specific target populations of cells through the incubating process and producing a more desirable population of T cells and the beneficial results thereof. The methods available also do not result in elimination of an undesired and specific T cell population from an input T cell population in the incubating process nor the beneficial results thereof. Moreover, the methods available are not designed to eliminate, from a T cell population during the incubation process, a subpopulation that will be used for generating an output composition comprising T cells expressing a genetically engineered recombinant receptor. The available methods also do not describe benefits including producing a more consistent and/or predictable population of T cells that is beneficial for genetic engineering, dosing, and/or clinical response.

In some aspects, the provided embodiments provide methods to generate an increased number of more uniform and/or predictable composition of T cells compared to other stimulation methods and, in some aspects, further provides for the diminution or elimination of undesired populations of T cells, e.g., cells whose presence may give the final product such heterogeneity that is difficult to predict potency, efficacy and safety. In some embodiments, the provided methods address problems related to the generation of genetically engineered T cells in which a large number or majority of such genetically engineered cells are derived from non-naïve-like T cells. In some aspects, lack of persistence, exhaustion and or/or problems related to toxicity can be associated with adoptive T cell therapy involving a composition containing genetically engineered cells derived from non-naive-like T cells and/or in which the percentage or number of genetically engineered cells derived from non-naive-like T cells is a above a certain threshold. In some embodiments, methods resulting in a genetically engineered T cell composition that is enriched for cells derived from naïve-like cells can exhibit features related to an overall increase in the percentage of healthy cells and/or in cells in the composition that exhibit increased persistence compared to other methods in which such naïve-like cells are not so enriched.

Accordingly, among the methods for preparing engineered T cells for adoptive therapy provided herein are those using conditions that result in preferential expansion or proliferation or genetic engineering of cells derived from naïve-like T cells (or derived from CD27+, CD45RA+, CCR7+, CD62L- or CD45RO- T cells) compared with those derived from non-naive-like T cells (or derived from CD27-, CD45RA-, CCR7-, CD62L+ or CD45RO+ T cells). In some embodiments, the methods involve incubating T cells in an input composition containing naïve-like T cells under stimulating conditions that preferentially produces a stimulated composition containing cells derived from naïve-like T cells of the input composition. In some aspects, the T cell population contains a mixture of both naïve-like T cells and non-naive-like T cells. In some embodiments, the stimulating conditions preferentially induce a response in non-naive-like T cells as compared to naïve-like T cells. In some cases, the response includes preferential activation of the non-naive-like T cells, which, in some embodiments, can lead to cell death.

In some embodiments, the method includes incubating cells under stimulating conditions including incubation with a stimulatory agent capable of activating one or more intracellular signaling domains of one or more components of a TCR complex and/or one or more intracellular signaling domains of one or more costimulatory molecules. In some cases, the primary agent specifically binds to CD3 and/or the costimulatory molecule is selected from the group consisting of CD28, CD137 (4-1-BB), OX40, or ICOS. For example, in some embodiments, the primary agent is or comprises anti-CD3 and the secondary agent is or comprises anti-CD28. In some cases, the primary and secondary agents comprise antibodies and/or are present on the surface of a solid support. In some examples, the solid support is a bead.

In some embodiments, the stimulating condition includes incubating the cells of the input composition with such a stimulatory reagent, e.g. anti-CD3/anti-CD28 beads, in which the ratio of beads to cells that is from or from about 1:1 to 10:1, from or from about 1:1 to 8:1, from or from about 1:1 to 6:1, from or from about 1:1 to 4:1, from or from about 1:1 to 3:1, from or from about 4:1 to 10:1, from or from about 4:1 to 8:1, from or from about 4:1 to 6:1, from or from about 6:1 to 10:1, from or from about 6:1 to 8:1, from or from about 8:1 to 10:1, from or from about 1:1 to 1:10, from or from about 1:1 to 1:8, from or from about 1:1 to 1:6, from or from about 1:1 to 1:4, from or from about 1:2 to 1:3. In some specific examples, the ratio of beads to cells is from or from about 3:1. In some cases, the ratio of beads to cells is from or from about 1:3.

The methods generally further include steps for genetic engineering of the stimulated composition of T cells. The genetic engineering may be carried out or initiated on the stimulated composition, following or at any point after the initiation of the initial incubation, and/or simultaneously with the incubation. In some embodiments, the cells are incubated with nucleic acids encoding such genetically engineered molecules, such that the nucleic acids are introduced and the genetically engineered molecules expressed in cells in the composition, thereby generating an output composition. Among the genetically engineered molecules are proteins, such as genetically engineered antigen receptors, including chimeric antigen receptors (CARs), and other recombinant receptors, such as chimeric receptors with ligand-binding extracellular portions and intracellular signaling portions.

Also provided are culture-initiating compositions, stimulated compositions, and output compositions, used in or generated by the described methods. Also provided are methods involving administration of such compositions and cells to subjects in need thereof, including cancer patients. Further provided are kits including the compositions and/or cells produced by any of the methods described herein.

The methods can be advantageous and produce a more desirable product. In some embodiments, the provided methods allow for a more consistent manufacturing process. In some embodiments, the provided methods produce cells that result in more uniform transduction and/or expansion in the later steps of engineering the cells. In some embodiments, the more uniform transduction and/or expansion can lead to a T cell product that is more predictable throughout the manufacturing process. In some cases, the T cell product can be more consistently dosed for administration to a subject. Such features may in some contexts reduce or prevent potential toxicity and/or associated outcomes and symptoms in subjects following adoptive cell therapy.

All publications, including patent documents, scientific articles and databases, referred to in this application are incorporated by reference in their entirety for all purposes to the same extent as if each individual publication were individually incorporated by reference. If a definition set forth herein is contrary to or otherwise inconsistent with a definition set forth in the patents, applications, published applications and other publications that are herein incorporated by reference, the definition set forth herein prevails over the definition that is incorporated herein by reference.

The section headings used herein are for organizational purposes only and are not to be construed as limiting the subject matter described.

### Brief Description of the Drawings

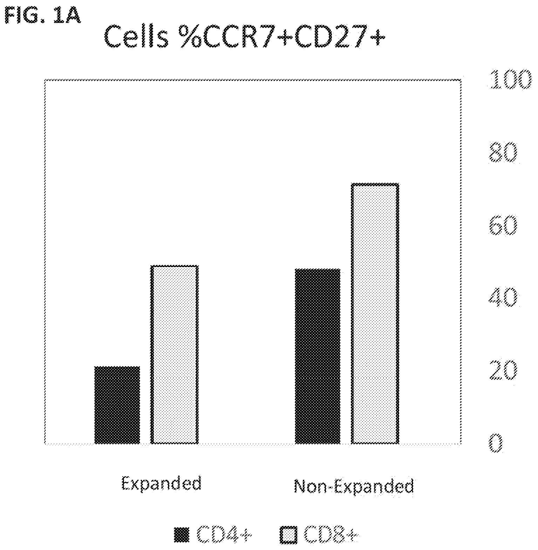

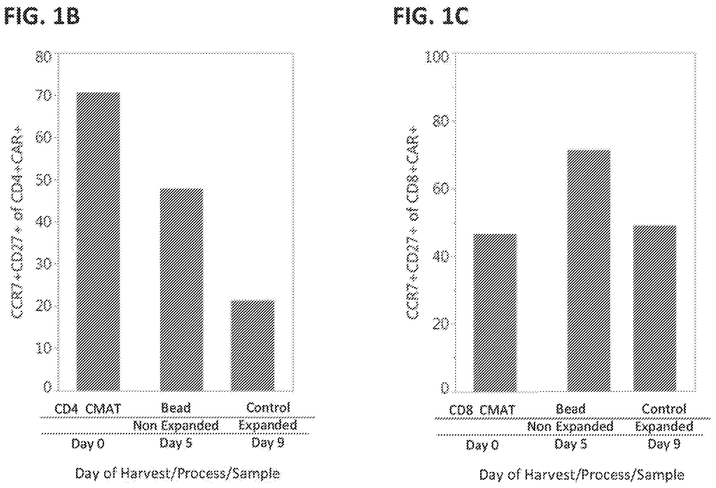

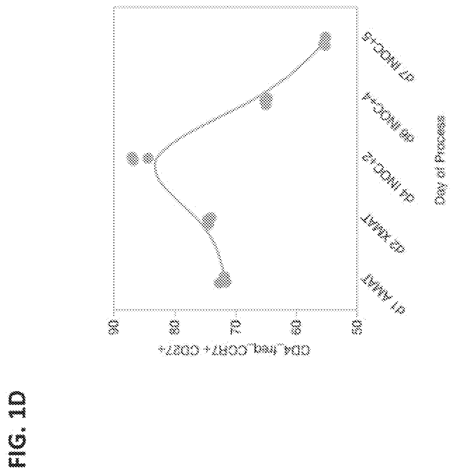

**FIGS. 1A-1D** depict results from CAR+ T cell compositions generated from an expanded and non-expanded processes involving a bead-based stimulatory reagent (Bead), a bead based stimulatory reagent and incubation in basal media (Bead-Basal Media), or an oligomeric stimulatory reagent (Oligomer). FIG. 1A depicts the percentage of CD4+ and CD8+ T cells positive for both CCR7 and CD27. FIG. 1B depicts the percentage of CCR7+CD27+cells for CD4+CAR+T cells. FIG. 1C depicts the percentage of CCR7+CD27+cells for CD8+CAR+T cells. FIG. 1D displays the percentage of CCR7+ CD27+ cells generated from a representative donor from an expanded process at various days during the process of manufacture, including activation at day 1(d1 AMAT), transduction at day 2 (d2 XMAT), and at various timed after initiation of cultivation (d4 INOC+2, d6 INOC+4, d7 INOC+5).

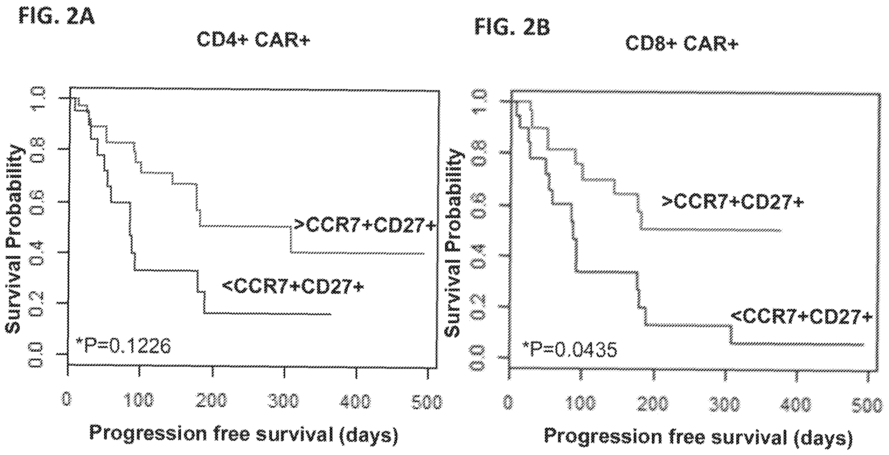

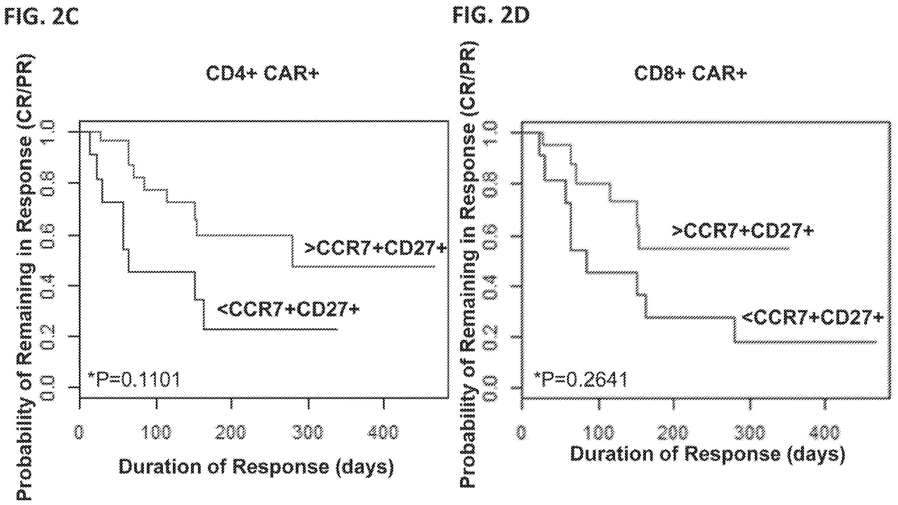

**FIGS. 2A-2D** show the Kaplan-Meier survival curves for subjects who were administered CAR⁺ T cell compositions, divided into groups that were administered compositions containing a percentage of CCR7⁺CD27⁺ CAR⁺ T cells among CD4⁺ CAR⁺ T cells (FIG. 2A for progression free survival, FIG. 2C for duration of response) and among CD8⁺ CAR⁺ T cells (FIG. 2B for progression free survival, FIG. 2D for duration of response) that is above or below a certain threshold level.

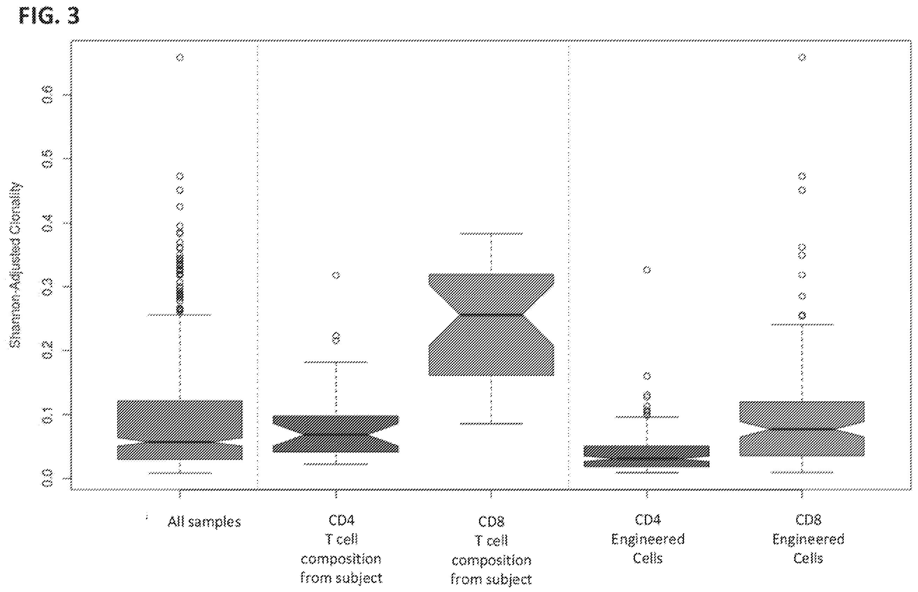

**FIG.** 3 shows the T cell clonality of the isolated CD4+ and CD8+ T cell compositions before engineering (CMAT) and of the CD4+ and CD8+ therapeutic CAR+T cell compositions after engineering (Shannon index applied).

### I. METHOD FOR INCUBATING (E. G., STIMULATING) T CELLS

Provided herein are methods for incubating (e.g., stimulating) cells in the process of preparing cells for adoptive cell therapy, and compositions and cells produced by the methods. In some embodiments, the cells typically include T cells, which in some embodiments express genetically engineered antigen receptors, such as genetically engineered or recombinant T cell receptors (TCRs) and functional non-TCR antigen receptors such as chimeric antigen receptors (CARs).

In some embodiments, provided is a method for incubating (e.g., stimulating) T cells including incubating, under stimulating conditions, an input composition containing a population of T cells comprising naïve-like T cells and non-naive-like T cells, said input composition containing a culture-initiating amount of the naive-like T cells or a CD8+ T cell subset thereof, thereby producing a stimulated composition. In some aspects, the stimulating conditions preferentially induce expansion or proliferation of the naïve-like T cells compared to the non-naive like T cells in the stimulated composition. In some embodiments, the method further includes introducing into the stimulated cell composition a nucleic acid encoding a genetically engineered recombinant receptor, wherein the method thereby generates an output composition comprising T cells expressing the genetically engineered recombinant receptor. In some aspects, the introducing is carried out during at least a portion of the incubating or is carried out subsequent to the incubating. In some embodiments, the cells of the input composition have not been and are not, prior to the incubation, subjected to a selection step based on an endogenous T cell surface marker that differentiates between naïve-like and non-naïve-like T cells.

In some embodiments of the methods provided, the introducing of a nucleic acid encoding a genetically engineered recombinant receptor is performed prior to, during and/or subsequent to introducing a nucleic acid encoding a genetically engineered recombinant receptor. In some embodiments, the method includes incubating the composition under stimulating conditions prior to introducing a nucleic acid encoding a genetically engineered recombinant receptor. In some cases, the method includes incubating the composition under stimulating conditions during the introducing of a nucleic acid encoding a genetically engineered recombinant receptor. In some embodiments, the method includes incubating the composition under stimulating conditions subsequent to introducing a nucleic acid encoding a genetically engineered recombinant receptor.

In certain embodiments, the stimulating condition comprises a surface, such as a magnetic bead, having attached thereto one or more agents that bind a cell surface moiety. In one embodiment, the surface has attached thereto at least anti-CD3 antibodies. In another embodiment, the surface has attached thereto anti-CD3 and/or anti-CD28 antibodies. In some embodiments, at least a substantial portion of at least one population of T cells in the input composition is deleted after about the incubating. In one embodiment, the ratio of cells to beads in the stimulating conditions is from about 50:1 to about 5:1. In certain embodiments, the ratio is from about 100:1 to about 1:1. In some embodiments, the ratio is from about 1:1 to 1:50. In certain embodiments, the ratio is at least about 40:1, 35:1, 30:1, 25:1, 20:1, 15:1, 14:1, 13:1, 12:1, 11:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, or 1:10. In one particular embodiment the ratio is about 3:1. In one particular embodiment, the ratio is about 1:3.

In some embodiments of the methods provided herein, the culture conditions preferentially induce proliferation, stimulation, and/or activation of non-naïve-like T cells compared to naïve-like T cells. In some embodiments, the methods of incubating (e.g., stimulating) generates a desired output composition comprised of a desired number of cells derived from naïve-like T cells of the input composition. In some embodiments of using the method of stimulating T cells described herein, of the cells in the input composition, a greater percentage of the naïve-like T cells, as compared to the non-naive-like T cells, are induced to proliferate and/or are expanded. In some aspects, the stimulated composition resulting from the methods of stimulating described herein contains less than 10% of cells derived from non-naive like T cells. In some examples, the stimulated composition contains less than 9%, 8%, 7%, 6%, 5%, 4%, 3%, 2%, 1%, 0.5% or 0.1% cells derived from non-naive T cells. In some embodiments, a greater percentage of the T cells that were naïve-like in the input composition, as compared to the percentage of the T cells that were non-naive-like in the input composition, are dividing at day 1, 2, 3, 4, 5, 6, 7, 8, 9, or 10 following initiation of said incubation. In some cases, the stimulating conditions of the method for stimulating T cells induces cell death in particular subpopulations of cells. In particular examples, the stimulating conditions of the method induce activation of non-naïve-like T cells, thereby inducing activation-induced cell death (AICD).

In one aspect, the method preferentially activates non-naive-like T cells of the input composition, thereby inducing cell death of non-naïve-like T cells of the input composition, thereby eliminating the T cells derived from non-naive-like cells of the input composition from the stimulated composition. At the same time, the desired cells that remain, e.g., those cells that are derived from the naïve-like T cells of the input composition, are activated, survive, and stimulated to expand, thereby resulting in a population of activated cells from which at least a substantial portion of unwanted subpopulations of T cells have been eliminated. Furthermore, the incubating (e.g., stimulation) conditions provided by the methods described herein restores polyclonality to the population of T cells with respect to expressed TCR genes as indicated by spectratype analysis or other methods of quantifying clonality. In some embodiments, the signature of the cells in the output composition derived from naïve-like T cells of the input composition is indicated by the expression of specific markers.

### A. Input Composition

In the provided methods for incubating (e.g., stimulating) T cells, the method includes incubating, under stimulating conditions, an input composition comprising a population of T cells. The input composition, in some aspects, comprises a population of T cells, such as comprising naïve-like T cells and non-naive-like T cells. In some embodiments, the population of T cells comprises CD4+ and/or CD8+ cells. In some embodiments, the input composition comprises a culture-initiating amount of cells. In some cases, the culture-initiating amount of cells is based on the amount of naïve-like T cells or a CD8+ T cell subset thereof in the input composition. In some aspects, specific subsets of T cells, such as naïve-like T cells and/or non-naïve-like T cells can be identified using specific markers or signatures. In some specific examples, expression of cell surface markers is used to assess and/or identify subsets of T cells. In some aspects, clonality of the input composition can be characterized.

In some aspects, the composition of cells being incubated and/or engineered, such as the input composition, has not been and is not subjected to previous selection of naïve-like T cells. In some embodiments, the method does not comprise positive or negative selection or enrichment of the naïve-like cells of the input composition. In some aspects, the composition of cells being stimulated and/or engineered, such as the input composition and/or the stimulated composition, has not been and is not subjected to such selection prior to the incubation (e.g., stimulation). In some embodiments, the cells have not been and/or are not, prior to the incubation, subjected to a selection step based on the level or presence of a T cell surface marker that differentiates between naïve-like and non-naive-like T cells, such as CD27, CD28, CD45RA, CD45RO, CD56, CD62L, CD95, KLRG1, or CCR7. For example, in some aspects, the cells in the input composition have not been subjected to selection based on expression of or based on surface expression of such a marker, prior to the incubation under the stimulating conditions. In some aspects, the cells in the stimulated composition are not subjected to selection based on expression of or based on surface expression of such a marker, prior to genetic engineering, e.g., incubation with the nucleic acid. In some aspects, the incubating of the input composition is carried out without selection based on surface expression of a marker to enrich for naive-like T cells.

In some embodiments, the methods comprise fewer selection steps compared to other methods, e.g., do not involve selection of subsets of T cells, and thus are simpler and associated with cost- and/or resource-saving advantages compared to multi-step selection methods. In some embodiments, the methods do not include enrichment based on expression of markers characteristic to memory T cells or subset thereof and/or naïve-like T cells. In some aspects, the composition of cells being incubated (e.g., stimulated), such as the input composition and/or the stimulated composition, has not been and is not subjected to such selection.

In some examples, the methods do not comprise positive selection based on a marker that is characteristic of non-naive-like T cells or that distinguishes specific subpopulations of T cells, such as CD62L, CCR7, CD27, CD28, CD56, CD3, CD122, CD95, CD25, IL7-Rα and/or CD 127. In some examples, the methods do not comprise positive selection or enrichment based on expression of CD62L, CCR7, CD27, CD28, CD56, CD3, CD122, CD95, CD25, IL7-Rα and/or CD127. In some embodiments, the methods do not use samples or compositions having been enriched or positively selected based on such markers or to enrich for specific sub-types.

In some embodiments, the methods do not comprise affinity-based selection steps designed to separate or distinguish between naïve-like and non-naive-like T cells. In some examples, the methods do not comprise positive selection based on a marker that is characteristic of naïve-like or non-naive-like cells, or distinguishes one from the other, such as CD27, CD28, CD45RO, CD45RA, CD56, CCR7, CD95, KLRG1, and/or CD62L. In some examples, the methods do not comprise positive selection or enrichment based on expression of such markers. In some embodiments, the methods do not use samples or compositions having been enriched or positively selected based on such markers or to enrich for such sub-types.

### Cells

In some embodiments, the methods provided herein include one or more steps for preparing an input composition of cells in which is contained a culture-initiating amount of naïve-like T cells, such as in connection with a method including one or more steps of stimulation, expansion, proliferation and/or genetic engineering, e.g. transduction, of cells. The cells generally are eukaryotic cells, such as mammalian cells, and typically are human cells. The input composition can be produced or generated by various methods involving isolation or selection of cells from a biological sample. In some embodiments, input composition contains CD4+ and/or CD8+ cells derived from a biological sample, such as obtained or derived from one or more isolation, selection or enrichment step. In some specific examples, a ratio of the CD4+ cells to the CD8+ cells of the input culture is or is about 1:1, 1:2, 2:1, 1:3, or 3:1. In some embodiments, the input composition containing isolated CD4+ and/or CD8+ T cells contains a mixture of naïve-like T cells and non-naive-like T cells.

In some embodiments, the cells of the input composition are derived from the blood, bone marrow, lymph, or lymphoid organs, are cells of the immune system, such as cells of the innate or adaptive immunity, including from samples containing myeloid or lymphoid cells, including lymphocytes, typically T cells. The cells typically are primary cells, such as those isolated directly from a subject and/or isolated from a subject and frozen. In some embodiments, the cells include one or more subsets of T cells or other cell types, such as whole T cell populations, CD4+ cells, CD8+ cells, and subpopulations thereof, such as those defined by function, activation state, maturity, potential for differentiation, expansion, recirculation, localization, and/or persistence capacities, antigen-specificity, type of antigen receptor, presence in a particular organ or compartment, marker or cytokine secretion profile, and/or degree of differentiation. With reference to the subject to be treated, the cells may be allogeneic and/or autologous. Among the methods include off-the-shelf methods. In some embodiments, the methods include isolating cells from the subject, preparing, processing, culturing, and/or engineering them, as described herein, and re-introducing them into the same patient, before or after cryopreservation.

Among the sub-types and subpopulations of T cells and/or of CD4+ and/or of CD8+ T cells are naive T (T_{N}) cells, effector T cells (T_{EFF}), memory T cells and sub-types thereof, such as stem cell memory T (T_{SCM}), central memory T (T_{CM}), effector memory T (T_{EM}), or terminally differentiated effector memory T cells, tumor-infiltrating lymphocytes (TIL), immature T cells, mature T cells, helper T cells, cytotoxic T cells, mucosa-associated invariant T (MAIT) cells, naturally occurring and adaptive regulatory T (Treg) cells, helper T cells, such as TH1 cells, TH2 cells, TH3 cells, TH17 cells, TH9 cells, TH22 cells, follicular helper T cells, alpha/beta T cells, and delta/gamma T cells. In some embodiments, the cell is a regulatory T cell (Treg). In some embodiments, the cell further comprises a recombinant FOXP3 or variant thereof.

In some embodiments, preparation of the engineered cells includes one or more culture and/or preparation steps. The cells for engineering may be isolated from a sample, such as a biological sample, e.g., one obtained from or derived from a subject. In some embodiments, the subject from which the cell is isolated is one having the disease or condition or in need of a cell therapy or to which cell therapy will be administered. The subject in some embodiments is a human in need of a particular therapeutic intervention, such as the adoptive cell therapy for which cells are being isolated, processed, and/or engineered.

Accordingly, the cells in some embodiments are primary cells, e.g., primary human cells. The samples include tissue, fluid, and other samples taken directly from the subject, as well as samples resulting from one or more processing steps, such as separation, centrifugation, genetic engineering (e.g. transduction with viral vector), washing, and/or incubation. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some aspects, the sample from which the cells are derived or isolated is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, e.g., adoptive cell therapy, samples from autologous and allogeneic sources.

In some embodiments, the cells are derived from cell lines, *e.g.,* T cell lines. The cells in some embodiments are obtained from a xenogeneic source, for example, from mouse, rat, non-human primate, or pig.

In some embodiments, isolation of the cells includes one or more preparation and/or non-affinity based cell separation steps. In some examples, cells are washed, centrifuged, and/or incubated in the presence of one or more reagents, for example, to remove unwanted components, enrich for desired components, lyse or remove cells sensitive to particular reagents. In some examples, cells are separated based on one or more property, such as density, adherent properties, size, sensitivity and/or resistance to particular components.

In some examples, cells from the circulating blood of a subject are obtained, e.g., by apheresis or leukapheresis. The samples, in some aspects, contain lymphocytes, including T cells, monocytes, granulocytes, B cells, other nucleated white blood cells, red blood cells, and/or platelets, and in some aspects contain cells other than red blood cells and platelets.

In some embodiments, the blood cells collected from the subject are washed, e.g., to remove the plasma fraction and to place the cells in an appropriate buffer or media for subsequent processing steps. In some embodiments, the cells are washed with phosphate buffered saline (PBS). In some embodiments, the wash solution lacks calcium and/or magnesium and/or many or all divalent cations. In some aspects, a washing step is accomplished a semi-automated "flow-through" centrifuge (for example, the Cobe 2991 cell processor, Baxter) according to the manufacturer's instructions. In some aspects, a washing step is accomplished by tangential flow filtration (TFF) according to the manufacturer's instructions. In some embodiments, the cells are resuspended in a variety of biocompatible buffers after washing, such as, for example, Ca⁺⁺/Mg⁺⁺ free PBS. In certain embodiments, components of a blood cell sample are removed and the cells directly resuspended in culture media.

In some embodiments, the methods include density-based cell separation methods, such as the preparation of white blood cells from peripheral blood by lysing the red blood cells and centrifugation through a Percoll or Ficoll gradient.

In some embodiments, the isolation methods include the separation of different cell types based on the expression or presence in the cell of one or more specific molecules, such as surface markers, e.g., surface proteins, intracellular markers, or nucleic acid. In some embodiments, any known method for separation based on such markers may be used. In some embodiments, the separation is affinity- or immunoaffinity-based separation. For example, the isolation in some aspects includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

Such separation steps can be based on positive selection, in which the cells having bound the reagents are retained for further use, and/or negative selection, in which the cells having not bound to the antibody or binding partner are retained. In some examples, both fractions are retained for further use. In some aspects, negative selection can be particularly useful where no antibody is available that specifically identifies a cell type in a heterogeneous population, such that separation is best carried out based on markers expressed by cells other than the desired population.

The separation need not result in 100% enrichment or removal of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells.

In some examples, multiple rounds of separation steps are carried out, where the positively or negatively selected fraction from one step is subjected to another separation step, such as a subsequent positive or negative selection. In some examples, a single separation step can deplete cells expressing multiple markers simultaneously, such as by incubating cells with a plurality of antibodies or binding partners, each specific for a marker targeted for negative selection. Likewise, multiple cell types can simultaneously be positively selected by incubating cells with a plurality of antibodies or binding partners expressed on the various cell types.

For example, in some aspects, specific subpopulations of T cells, such as cells positive or expressing high levels of one or more surface markers, e.g., CD28⁺, CD62L⁺, CCR7⁺, CD27⁺, CD127⁺, CD4⁺, CD8⁺, CD56⁺, CD45RA⁺, CD95^{hi}, and/or CD45RO⁺ T cells, are isolated by positive or negative selection techniques.

For example, CD3⁺, CD28⁺ T cells can be positively selected using anti-CD3/anti-CD28 conjugated magnetic beads (e.g., DYNABEADS^{®} M-450 CD3/CD28 T Cell Expander).

In some embodiments, isolation is carried out by enrichment for a particular cell population by positive selection, or depletion of a particular cell population, by negative selection. In some embodiments, positive or negative selection is accomplished by incubating cells with one or more antibodies or other binding agent that specifically bind to one or more surface markers expressed or expressed (marker⁺) at a relatively higher level (marker^{high}) on the positively or negatively selected cells, respectively.

In some embodiments, T cells are separated from a PBMC sample by negative selection of markers expressed on non-T cells, such as B cells, monocytes, or other white blood cells, such as CD14. In some aspects, a CD4⁺ or CD8⁺ selection step is used to separate CD4⁺ helper and CD8⁺ cytotoxic T cells. Such CD4⁺ and CD8⁺ populations can be further sorted into sub-populations by positive or negative selection for markers expressed or expressed to a relatively higher degree on one or more naive, memory, and/or effector T cell subpopulations.

In some embodiments, CD8⁺ cells are further enriched for or depleted of naive, central memory, effector memory, and/or central memory stem cells, such as by positive or negative selection based on surface antigens associated with the respective subpopulation. In some embodiments, enrichment for central memory T (T_{CM}) cells is carried out to increase efficacy, such as to improve long-term survival, expansion, and/or engraftment following administration, which in some aspects is particularly robust in such sub-populations. *See* Terakura et al. (2012) Blood. 1:72-82; Wang et al. (2012) J Immunother. 35(9):689-701. In some embodiments, combining T_{CM}-enriched CD8⁺ T cells and CD4⁺T cells further enhances efficacy.

In embodiments, memory T cells are present in both CD62L⁺ and CD62L⁻ subsets of CD8⁺ peripheral blood lymphocytes. PBMC can be enriched for or depleted of CD62L⁻CD8⁺ and/or CD62L⁺CD8⁺ fractions, such as using anti-CD8 and anti-CD62L antibodies.

In some embodiments, the enrichment for central memory T (T_{CM}) cells is based on positive or high surface expression of CD45RO, CD62L, CCR7, CD28, CD3, and/or CD 127; in some aspects, it is based on negative selection for cells expressing or highly expressing CD45RA and/or granzyme B. In some aspects, isolation of a CD8⁺ population enriched for T_{CM} cells is carried out by depletion of cells expressing CD4, CD14, CD45RA, and positive selection or enrichment for cells expressing CD62L. In one aspect, enrichment for central memory T (T_{CM}) cells is carried out starting with a negative fraction of cells selected based on CD4 expression, which is subjected to a negative selection based on expression of CD14 and CD45RA, and a positive selection based on CD62L. Such selections in some aspects are carried out simultaneously and in other aspects are carried out sequentially, in either order. In some aspects, the same CD4 expression-based selection step used in preparing the CD8⁺ cell population or subpopulation, also is used to generate the CD4⁺ cell population or subpopulation, such that both the positive and negative fractions from the CD4-based separation are retained and used in subsequent steps of the methods, optionally following one or more further positive or negative selection steps.

In a particular example, a sample of PBMCs or other white blood cell sample is subjected to selection of CD4⁺ cells, where both the negative and positive fractions are retained. The negative fraction then is subjected to negative selection based on expression of CD14 and CD45RA or ROR1, and positive selection based on a marker characteristic of central memory T cells, such as CD62L or CCR7, where the positive and negative selections are carried out in either order.

CD4⁺ T helper cells are sorted into naive, central memory, and effector cells by identifying cell populations that have cell surface antigens. CD4⁺ lymphocytes can be obtained by standard methods. In some embodiments, naive CD4⁺ T lymphocytes are CD45RO⁻, CD45RA⁺, CD62L⁻, CD4⁺ T cells. In some embodiments, central memory CD4⁺ cells are CD62L⁺ and CD45RO⁺. In some embodiments, effector CD4⁺ cells are CD62L⁻ and CD45RO⁻.

In one example, to enrich for CD4⁺ cells by negative selection, a monoclonal antibody cocktail typically includes antibodies to CD14, CD20, CD11b, CD16, HLA-DR, and CD8. In some embodiments, the antibody or binding partner is bound to a solid support or matrix, such as a magnetic bead or paramagnetic bead, to allow for separation of cells for positive and/or negative selection. For example, in some embodiments, the cells and cell populations are separated or isolated using immunomagnetic (or affinitymagnetic) separation techniques (reviewed in Methods in Molecular Medicine, vol. 58: Metastasis Research Protocols, Vol. 2: Cell Behavior In vitro and In vivo, p 17-25 Edited by: S. A. Brooks and U. Schumacher © Humana Press Inc., Totowa, NJ).

In some aspects, the sample or composition of cells to be separated is incubated with small, magnetizable or magnetically responsive material, such as magnetically responsive particles or microparticles, such as paramagnetic beads (e.g., such as Dynalbeads or MACS beads). The magnetically responsive material, e.g., particle, generally is directly or indirectly attached to a binding partner, *e.g.,* an antibody, that specifically binds to a molecule, *e.g.,* surface marker, present on the cell, cells, or population of cells that it is desired to separate, e.g., that it is desired to negatively or positively select.

In some embodiments, the magnetic particle or bead comprises a magnetically responsive material bound to a specific binding member, such as an antibody or other binding partner. In some aspects, magnetically responsive materials used in magnetic separation methods can be used. Suitable magnetic particles include those described in Molday, U.S. Pat. No. 4,452,773, and in European Patent Specification EP 452342 B, which are hereby incorporated by reference. Colloidal sized particles, such as those described in Owen U.S. Pat. No. 4,795,698, and Liberti et al., U.S. Pat. No. 5,200,084 are other examples.

The incubation generally is carried out under conditions whereby the antibodies or binding partners, or molecules, such as secondary antibodies or other reagents, which specifically bind to such antibodies or binding partners, which are attached to the magnetic particle or bead, specifically bind to cell surface molecules if present on cells within the sample.

In some aspects, the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained. In some aspects, a combination of positive and negative selection is performed during the same selection step, where the positive and negative fractions are retained and further processed or subject to further separation steps.

In certain embodiments, the magnetically responsive particles are coated in primary antibodies or other binding partners, secondary antibodies, lectins, enzymes, or streptavidin. In certain embodiments, the magnetic particles are attached to cells via a coating of primary antibodies specific for one or more markers. In certain embodiments, the cells, rather than the beads, are labeled with a primary antibody or binding partner, and then cell-type specific secondary antibody- or other binding partner (e.g., streptavidin)-coated magnetic particles, are added. In certain embodiments, streptavidin-coated magnetic particles are used in conjunction with biotinylated primary or secondary antibodies.

In some embodiments, the magnetically responsive particles are left attached to the cells that are to be subsequently incubated, cultured and/or engineered; in some aspects, the particles are left attached to the cells for administration to a patient. In some embodiments, the magnetizable or magnetically responsive particles are removed from the cells. Methods for removing magnetizable particles from cells can include, e.g., the use of competing non-labeled antibodies, magnetizable particles or antibodies conjugated to cleavable linkers, etc. In some embodiments, the magnetizable particles are biodegradable.

In some embodiments, the affinity-based selection is via magnetic-activated cell sorting (MACS) (Miltenyi Biotec, Auburn, CA). Magnetic Activated Cell Sorting (MACS) systems are capable of high-purity selection of cells having magnetized particles attached thereto. In certain embodiments, MACS operates in a mode wherein the non-target and target species are sequentially eluted after the application of the external magnetic field. That is, the cells attached to magnetized particles are held in place while the unattached species are eluted. Then, after this first elution step is completed, the species that were trapped in the magnetic field and were prevented from being eluted are freed in some manner such that they can be eluted and recovered. In certain aspects, the non-target cells are labelled and depleted from the heterogeneous population of cells.

In certain embodiments, the isolation or separation is carried out using a system, device, or apparatus that carries out one or more of the isolation, cell preparation, separation, processing, incubation, culture, and/or formulation steps of the methods. In some aspects, the system is used to carry out each of these steps in a closed or sterile environment, for example, to minimize error, user handling and/or contamination. In one example, the system is a system as described in International PCT Publication No. WO2009/072003, or US 20110003380 A1.

In some embodiments, the system or apparatus carries out one or more, e.g., all, of the isolation, processing, engineering, and formulation steps in an integrated or self-contained system, and/or in an automated or programmable fashion. In some aspects, the system or apparatus includes a computer and/or computer program in communication with the system or apparatus, which allows a user to program, control, assess the outcome of, and/or adjust various aspects of the processing, isolation, engineering, and formulation steps.

In some aspects, the separation and/or other steps is carried out using CliniMACS system (Miltenyi Biotec), for example, for automated separation of cells on a clinical-scale level in a closed and sterile system. Components can include an integrated microcomputer, magnetic separation unit, peristaltic pump, and various pinch valves. The integrated computer in some aspects controls all components of the instrument and directs the system to perform repeated procedures in a standardized sequence. The magnetic separation unit in some aspects includes a movable permanent magnet and a holder for the selection column. The peristaltic pump controls the flow rate throughout the tubing set and, together with the pinch valves, ensures the controlled flow of buffer through the system and continual suspension of cells.

The CliniMACS system in some aspects uses antibody-coupled magnetizable particles that are supplied in a sterile, non-pyrogenic solution. In some embodiments, after labelling of cells with magnetic particles the cells are washed to remove excess particles. A cell preparation bag is then connected to the tubing set, which in turn is connected to a bag containing buffer and a cell collection bag. The tubing set consists of pre-assembled sterile tubing, including a pre-column and a separation column, and are for single use only. After initiation of the separation program, the system automatically applies the cell sample onto the separation column. Labelled cells are retained within the column, while unlabeled cells are removed by a series of washing steps. In some embodiments, the cell populations for use with the methods described herein are unlabeled and are not retained in the column. In some embodiments, the cell populations for use with the methods described herein are labeled and are retained in the column. In some embodiments, the cell populations for use with the methods described herein are eluted from the column after removal of the magnetic field, and are collected within the cell collection bag.

In certain embodiments, separation and/or other steps are carried out using the CliniMACS Prodigy system (Miltenyi Biotec). The CliniMACS Prodigy system in some aspects is equipped with a cell processing unity that permits automated washing and fractionation of cells by centrifugation. The CliniMACS Prodigy system can also include an onboard camera and image recognition software that determines the optimal cell fractionation endpoint by discerning the macroscopic layers of the source cell product. For example, peripheral blood may be automatically separated into erythrocytes, white blood cells and plasma layers. The CliniMACS Prodigy system can also include an integrated cell cultivation chamber which accomplishes cell culture protocols such as, e.g., cell differentiation and expansion, antigen loading, and long-term cell culture. Input ports can allow for the sterile removal and replenishment of media and cells can be monitored using an integrated microscope. See, e.g., Klebanoff et al. (2012) J Immunother. 35(9): 651-660, Terakura et al. (2012) Blood. 1:72-82, and Wang et al. (2012) J Immunother. 35(9):689-701.

In some embodiments, a cell population described herein is collected and enriched (or depleted) via flow cytometry, in which cells stained for multiple cell surface markers are carried in a fluidic stream. In some embodiments, a cell population described herein is collected and enriched (or depleted) via preparative scale (FACS)-sorting. In certain embodiments, a cell population described herein is collected and enriched (or depleted) by use of microelectromechanical systems (MEMS) chips in combination with a FACS-based detection system (see, *e.g.,* WO 2010/033140, Cho et al. (2010) Lab Chip 10:1567-1573; and Godin et al. (2008) J Biophoton. 1(5):355-376. In both cases, cells can be labeled with multiple markers, allowing for the isolation of well-defined T cell subsets at high purity.

In some embodiments, the antibodies or binding partners are labeled with one or more detectable marker, to facilitate separation for positive and/or negative selection. For example, separation may be based on binding to fluorescently labeled antibodies. In some examples, separation of cells based on binding of antibodies or other binding partners specific for one or more cell surface markers are carried in a fluidic stream, such as by fluorescence-activated cell sorting (FACS), including preparative scale (FACS) and/or microelectromechanical systems (MEMS) chips, e.g., in combination with a flow-cytometric detection system. Such methods allow for positive and negative selection based on multiple markers simultaneously.

In some embodiments, the preparation methods include steps for freezing, e.g., cryopreserving, the cells, either before or after isolation, incubation, and/or engineering. In some embodiments, the freeze and subsequent thaw step removes granulocytes and, to some extent, monocytes in the cell population. In some embodiments, the cells are suspended in a freezing solution, e.g., following a washing step to remove plasma and platelets. Any of a variety of known freezing solutions and parameters in some aspects may be used. One example involves using PBS containing 20% DMSO and 8% human serum albumin (HSA), or other suitable cell freezing media. This is then diluted 1:1 with media so that the final concentration of DMSO and HSA are 10% and 4%, respectively. The cells are then frozen to -80° C. at a rate of 1° per minute and stored in the vapor phase of a liquid nitrogen storage tank.

### a. Naïve-like T cells

In some embodiments, the method comprises incubating an input composition comprising naive-like T cells or a certain threshold amount of naïve-like T cells. One aspect of the present methods provides an input composition that has been assessed for subpopulations of cells based on expression of a variety of markers, such as CD27, CD28, CD56, CD62L, CD95, KLRG1, CD45RA or CD45RO, cytokines (e.g., IL-2, IFN-γ, IL-4, IL-10), cytokine receptors (e.g., CD25), perforin, adhesion molecules (e.g., VLA-1, VLA-2, VLA-4, LPAM-1, LFA-1), and/or homing molecules (e.g., L-Selectin), prior to the incubating (e.g., stimulating). To identify naïve-like T cells, in some embodiments, various signatures of naïve-like T cells can be utilized. In some embodiments, expression of particular markers of naïve-like T cells can be assessed. For examples, in some cases, the naïve-like T cells are surface positive for a marker, including T cell activation markers, selected from the group consisting of CD27, CD28, CD45RA, CD62L, and CCR7. In some aspects, the naive-like T cells are surface negative for CD56 and/or CD45RO. In some aspects, the naïve-like T cells are surface negative for CD45RO and cell surface positive for CD27, CD45RA, and CCR7. In some cases, the naïve-like T cells are negative for intracellular expression of a cytokine such as IL-2, IFN-γ, IL-4, and/or IL-10. In some further examples, the naïve-like T cells are negative for expression of markers CD25 and/or perforin. In some cases, the naïve-like T cells are CD95^{lo}.

To assess the expression of the markers of naive-like T cells, the method in some embodiments includes detecting the markers by performing an *in vitro* assay. In some examples, the *in vitro* assay is an immunoassay, an aptamer-based assay, a histological or cytological assay, or an mRNA expression level assay. In some cases, the *in vitro* assay used can be an enzyme-linked immunosorbent assay (ELISA), immunoblotting, immunophenotyping, immunoprecipitation, radioimmunoassay (RIA), immunostaining, flow cytometry assay, surface plasmon resonance (SPR), chemiluminescence assay, lateral flow immunoassay, inhibition assay or avidity assay. In some embodiments, the expression of markers of naïve-like T cells is determined by RNA-seq.

Naïve-like T cells of the input composition can also be assessed by the clonality of the T cells. In some embodiments, assessing the clonality of the population of T cells is an assessment of clonal diversity of the population of T cells. In some embodiments, the naïve-like T cells are polyclonal or multiclonal. Polyclonality of said input composition of T cells is measured by the breadth of the response of the population to a given antigen. In some aspects, the input composition can be assessed by measuring the number of different epitopes recognized by antigen-specific cells. This can be carried out using standard techniques for generating and cloning antigen-specific T cells *in vitro.* In some embodiments, the naive-like T cells are polyclonal (or multiclonal) with no single clonotypic population predominating in the population of naïve-like T cells.

In the context of a population of T cells, such as of the input composition, in some aspects, the signature of polyclonality refers to a population of T cells that has multiple and broad antigen specificity. In some embodiments, polyclonality relates to a population of T cells that exhibits high diversity in the TCR repertoire. In some cases, diversity of the TCR repertoire is due to V(D)J recombination events that, in some respects, are triggered by selection events to self and foreign antigens. In some embodiments, a population of T cells that is diverse or polyclonal is a population of T cells in which analysis indicates the presence of a plurality of varied or different TCR transcripts or products present in the population. In some embodiments, a population of T cells that exhibits high or relatively high clonality is a population of T cells in which the TCR repertoire is less diverse. In some embodiments, T cells are oligoclonal if analysis indicates the presence of several, such as two or three, TCR transcripts or products in a population of T cells. In some embodiments, T cells are monoclonal if analysis indicates the presence of a single TCR transcript or product in a population of T cells.

The clonality of the cells in the input composition, such as the naïve-like T cells is, in some examples, determined by clonal sequencing, optionally next-generation sequencing, or spectratype analysis. In some aspects, next-generation sequencing methods can be employed, using genomic DNA or cDNA from T cells, to assess the TCR repertoire, including sequences encoding the complementarity-determining region 3 (CDR3). In some embodiments, whole transcriptome sequencing by RNA-seq can be employed. In some embodiments, single-cell sequencing methods can be used.

In some embodiments, polyclonality can be assessed or determined by spectratype analysis (a measure of the TCR Vβ, Vα, Vγ, or V6 chain hypervariable region repertoire). A population of T cells is considered polyclonal when the Vβ spectratype profile for a given TCR Vβ, Vα, Vγ, or Vδ family has multiple peaks, typically 5 or more predominant peaks and in most cases with Gaussian distribution. Polyclonality can also be defined by generation and characterization of antigen-specific clones to an antigen of interest.

In some embodiments, the methods for assessing clonality can include various features of the methods as described in International Publication Nos. WO2012/048341, WO2014/144495, WO2017/053902, WO2016044227, WO2016176322 and WO2012048340 each incorporated by reference in their entirety. In some embodiments, such methods can be used to obtain sequence information about a target polynucleotide of interest within a cell, such as a TCR. The target genes can be obtained from genomic DNA or mRNA of a cell from a sample or population of cells. The sample or population of cells can include immune cells. For example, for target TCR molecules, the genes encoding chains of a TCR can be obtained from genomic DNA or mRNA of immune cells or T cells. In some embodiments, the starting material is RNA from T cells composed of genes that encode for a chain of a TCR.

In some embodiments, the Shannon index is applied to the clonality as a threshold to filter clones ("Shannon- adjusted clonality"), see, Chaara et al. (2018) Front Immunol 9: 1038).

In some embodiments, the provided methods promote or result in an increase in the polyclonality of the population of T cells or a subset thereof from the input composition. In some embodiments, the provided methods promote or result in an increase in the diversity of the population of T cells or a subset thereof from the input composition. In some embodiments, T cells, or a CD4 or CD8 subset thereof, of an incubated or stimulated composition exhibit reduced or decreased clonality compared to T cells, or the CD4 or CD8 subset thereof, in the input composition prior to carrying out the methods. In some embodiments, the degree of clonality is decreased by greater than or greater than about 2-fold, 3-fold, 4-fold, 5-fold, 6-fold, 7-fold, 8-fold, 9-fold, 10-fold or more.

In one aspect of the methods provided, a population of T cells in the input composition is activated or stimulated to induce apoptosis as described below in the section entitled "Cell Incubation", thereby eliminating the subpopulation from the population of cells. At the same time, the desired cells that remain, e.g., the naïve-like cells, are activated and stimulated to expand, thereby resulting in a population of activated cells from which at least a substantial portion of unwanted subpopulations of T cells (such as non-naive like T cells) have been eliminated. As mentioned previously, stimulation/activation as described herein may be carried out on cells remaining following exposure of a population of cells directly to pro-apoptotic compositions. Furthermore, the subsequent stimulation and activation provided by the present invention restores polyclonality to the population of T cells with respect to expressed TCR genes as indicated by spectratype analysis or sequencing methods.

### Culture-initiating Amount

In aspects of the provided methods, the input composition includes a culture-initiating amount for the preferential activation or expansion of naïve-like T cells. In some cases, the culture-initiating amount includes or is determined or based on the number of naïve-like T cells or a CD8+ T cell subset thereof in the composition. Thus, in aspects of the provided method the incubation (e.g. stimulating) is carried out without regard to the total number or percentage of non-naive T cells so long as there is a present a threshold or culture-initiating amount of naïve-like T cells in the input composition.

In some embodiments of the methods provided herein for incubating (e.g., stimulating) cells, the culture-initiating amount of the naive-like T cells or a CD8+ T cell subset thereof is from or from about 0.1 × 10⁸ to 5 × 10⁸, from or from about 0.1 × 10⁸ to 4 × 10⁸, from or from about 0.1 × 10⁸ to 2 × 10⁸, from or from about 0.1 × 10⁸ to 1 × 10⁸, from or from about 1 × 10⁸ to 5 × 10⁸ from or from about 1 × 10⁸ to 4 × 10⁸, from or from about 1 × 10⁸ to 2 × 10⁸, from or from about 2 × 10⁸ to 5 × 10⁸, from or from about 2 × 10⁸ to 4 × 10⁸ of the naïve-like T cells. In some cases, the culture-initiating amount of the naïve-like T cells or a CD8+ T cell subset thereof is at least or at least about or is or is about 0.5 × 10⁸, 0.75 × 10⁸, 1 × 10⁸, 1.5 × 10⁸, 2 × 10⁸, or 4 × 10⁸ of the naïve-like T cells. In some examples, the culture-initiating amount of the naïve-like T cells or a CD8+ T cell subset thereof is at least or at least about or is or is about 2 × 10⁸ cells.

In some embodiments of the methods for incubating provided herein under stimulating conditions described herein, an input composition comprising a population of T cells comprising naïve-like T cells and non-naive-like T cells contains a culture-initiating amount of from or from about 1 × 10⁸ to 4 × 10⁸ naïve-like T cells or a CD8+ T cell subset thereof. In some embodiments, the methods thereby produce a stimulated composition, wherein the stimulating conditions preferentially induces expansion or proliferation of the naïve-like T cells compared to the non-naive like T cells in the stimulated composition. In some aspects, the culture-initiating amount of the naïve-like T cells or a CD8+ T cell subset thereof is at least or at least about or is or is about 2 × 10⁸ cells. In some embodiments, the amount of naïve-like T cells and non-naive like T cells of the input composition are about the same. In some cases, the culture initiating amount is an amount of naïve CD8+ T cells. In some cases, the culture initiating amount does not take into account the amount of non-naïve-like T cells in the input composition. In some aspects, the culture-initiating amount is determined based on the number of target non-naive-like T cells in the stimulated composition.

### b. Non-naïve-like T cells

In some embodiments, the method comprises incubating an input composition comprising a population of T cells comprising naïve-like T cells and non-naive-like T cells. In some embodiments, the non-naive-like T cells include effector T (T_{EFF}) cells, memory T cells, central memory T cells (T_{CM}), effector memory T (T_{EM}) cells, and combinations thereof. One aspect of the present methods provides an input composition that has been assessed for populations of cells expressing a variety of markers, such as CD27, CD28, CD56, CD62L, CD95, KLRG1, CD45RA or CD45RO, cytokines (e.g., IL-2, IFN-γ, IL-4, IL-10), cytokine receptors (e.g., CD25), perforin, adhesion molecules (e.g., VLA-1, VLA-2, VLA-4, LPAM-1, LFA-1), and/or homing molecules (e.g., L-Selectin), prior to the incubating (e.g., stimulating). To identify non-naive-like T cells, in some embodiments, various signatures of non-naïve-like T cells can be utilized. In some embodiments, expression of particular markers of non-naive-like T cells can be assessed. For example, in some cases, the non-naive-like T cells are surface negative for a marker, including T cell activation markers, such as CD27, CD28, CD45RA, and CCR7; and in some cases, the non-naive-like T cells are surface positive for a marker, including CD62L. In some aspects, the non-naive-like T cells are surface positive for CD56 and/or CD45RO. In some aspects, the non-naive-like T cells are surface positive for CD45RO and cell surface negative for CD27, CD45RA, and CCR7. In some cases, the non-naive-like T cells are positive for intracellular expression of a cytokine such as IL-2, IFN-γ, IL-4, and/or IL-10. In some further examples, the non-naive-like T cells are positive for expression of markers CD25 and/or perforin. In some cases, the non-naive-like T cells are CD95^{hi}.

To assess the expression of the markers of non-naive-like T cells, the method in some embodiments includes detecting the markers by performing an *in vitro* assay. In some examples, the *in vitro* assay is an immunoassay, an aptamer-based assay, a histological or cytological assay, or an mRNA expression level assay. In some cases, the *in vitro* assay used can be an enzyme-linked immunosorbent assay (ELISA), immunoblotting, immunoprecipitation, radioimmunoassay (RIA), immunostaining, flow cytometry assay, surface plasmon resonance (SPR), chemiluminescence assay, lateral flow immunoassay, inhibition assay or avidity assay. In some embodiments, the expression of markers of non-naive-like T cells is determined by RNA-seq.

Non-naive-like T cells of the input composition can also be assessed by the clonality of the T cells. In some embodiments, the non-naive-like T cells are monocolonal. Clonality of said input composition of T cells is measured by the breadth of the response of the population to a given antigen. In some embodiments, monoclonality refers to a population of T cells that is of low diversity. In some aspects, the input composition can be assessed by measuring the number of different epitopes recognized by antigen-specific cells. This can be carried out using standard techniques for generating and cloning antigen-specific T cells *in vitro.* In some embodiments, the non-naive-like T cells exhibit a predominance of a single TCR-gene rearrangement pattern. The clonality of the cells in the input composition, such as the non-naive-like T cells is, in some examples, determined by clonal sequencing, optionally next-generation sequencing, or spectratype analysis.

In some embodiments, assessing the clonality of the population of T cells is an assessment of clonal diversity of the population of T cells. In some embodiments, a population of T cells that is monoclonal refers to a population of T cells that exhibits low diversity.In the context of a population of T cells, such as of the input composition, monoclonality refers to a population of T cells that has a single specificity as defined by spectratype analysis (a measure of the TCR Vβ, Vα, Vγ, or V6 chain hypervariable region repertoire). A population of T cells is considered monoclonal (or mono-specific) when the Vβ, Vα, Vγ, and/or Vδ spectratype profile for a given TCR Vβ, Vα, Vγ, and/or Vδ family has a single predominant peak. Spectratype analysis distinguishes rearranged variable genes of a particular size, not sequence. Thus, it is understood that a single peak could represent a population of T cells expressing any one of a limited number of rearranged TCR variable genes (Vβ, Vα, Vγ, or Vδ) comprising any one of the 4 potential nucleotides (adenine (a), guanine (g), cytosine (c), or thymine (t)) or a combination of the 4 nucleotides at the junctional region. In certain embodiments, it may be desirable to clone and sequence a particular band to determine the sequence(s) of the rearranged variable gene(s) present in the band representing a particular length.

In some embodiments, the methods for assessing clonality can include various features of the methods as described in International Publication Nos. WO2012/048341, WO2014/144495, WO2017/053902, WO2016044227, WO2016176322 and WO2012048340 each incorporated by reference in their entirety. In some embodiments, such methods can be used to obtain sequence information about a target polynucleotide of interest within a cell, such as a TCR. The target genes can be obtained from genomic DNA or mRNA of a cell from a sample or population of cells. The sample or population of cells can include immune cells. For example, for target TCR molecules, the genes encoding chains of a TCR can be obtained from genomic DNA or mRNA of immune cells or T cells. In some embodiments, the starting material is RNA from T cells composed of genes that encode for a chain of a TCR. In some embodiments, the Shannon index is applied to the clonality as a threshold to filter clones ("Shannon- adjusted clonality"), see, Chaara et al. (2018) Front Immunol 9:1038).

Accordingly, among the methods for incubating (e.g., stimulating) T cells in preparing engineered T cells for adoptive therapy are those using conditions that preferentially induce expansion and proliferation of cells derived from naïve-like T cells (or derived from CD45RA+ or CD45RO- T cells) compared with those derived from non-naive-like T cells (or derived from CD45RA- or CD45RO+ T cells). In some embodiments, the naive-like T cells are CD45RA+, CD45RO-, CD27+, and CCR7+. In some embodiments, the non-naive-like T cells are CD45RA-, CD45RO+, CD27-, and CCR7-. In some embodiments, the methods involve incubating T cells in a culture-initiating composition under stimulating conditions that preferentially induces expansion or proliferation of naïve-like T cells compared to non-naive-like T cells, thereby generating a stimulated composition. In using the provided methods, assessment of the input composition using the markers and signatures described above may be utilized as part of the method.

### B. Cell Incubation

In some embodiments, the provided methods include cultivation, incubation, culture, and/or genetic engineering steps of cells in the input composition, such as of an input composition containing a culture-initiating amount of naïve-like T cells. For example, in some embodiments, provided are methods for incubating and/or engineering a culture-initiating amount of the provided input compositions in which is contained a threshold number of naive-like T cells as described. The incubation and/or engineering may be carried out in a culture vessel, such as a unit, chamber, well, column, tube, tubing set, valve, vial, culture dish, bag, or other container for culture or cultivating cells.

In some embodiments, the cells are incubated and/or cultured prior to or in connection with genetic engineering, such as according to any of the methods described in Section II. The incubation steps can include culture, cultivation, stimulation, activation, and/or propagation. In some embodiments, the compositions or cells are incubated in the presence of stimulating conditions or a stimulatory agent. Such conditions include those designed to induce proliferation, expansion, activation, and/or survival of cells in the population, to mimic antigen exposure, and/or to prime the cells for genetic engineering, such as for the introduction of a recombinant receptor, e.g., CAR.

The conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some embodiments, the stimulating conditions or agents include one or more agent, e.g., ligand, which is capable of activating an intracellular signaling domain of a TCR complex. In some aspects, the agent turns on or initiates TCR/CD3 intracellular signaling cascade in a T cell. Such agents can include antibodies, such as those specific for a TCR, e.g. anti-CD3. In some embodiments, the stimulating conditions include one or more agent, e.g. ligand, which is capable of stimulating a costimulatory receptor, e.g., anti-CD28. In some embodiments, such agents and/or ligands may be, bound to solid support such as a bead, and/or one or more cytokines. Optionally, the expansion method may further comprise the step of adding anti-CD3 and/or anti CD28 antibody to the culture medium (e.g., at a concentration of at least about 0.5 ng/ml).

The provided methods generally include the presence, design, and/or use of stimulating conditions that preferentially induce expansion or proliferation of the naïve-like versus non-naive-like T cells, and/or that do not preferentially induce expansion or proliferation in non-naive-like compared with naïve-like T cells. In some aspects, the conditions include agents that induce an activating signal such that only non-naive-like T cells present in the composition will become activated in a manner that induces cell death. Such preferential conditions typically are used at a stage prior to the introduction of nucleic acids encoding the engineered molecules, such as engineered antigen receptors.

The conditions include, but are not limited to, those designed to induce proliferation, expansion, activation, and/or survival of cells in the population. In some embodiments, the conditions induce a stimulating, e.g., activation, signal sufficient to activate and/or induce proliferation or division in non-naive-like T cells or a subset thereof. Naïve-like T cells generally require a minimal signal via TCR/CD3 to reach an activation threshold, for example, to become fully activated and driven into cell cycle. This minimal signal is generally higher than the signal required to induce activation/cell cycle entry in non-naive-like T cells and/or certain subsets thereof. Non-naive-like T cells generally require a much lower level of TCR/CD3 engagement to become activated and enter into cell cycle. In some aspects, stronger signals can cause or increase levels of activation-induced cell death in non-naive-like cells or certain populations thereof.

Thus, in some embodiments, where the composition includes a population of naïve-like and non-naive-like T cells, and conditions are used that induce an activating signal that is below the threshold of activation required for naïve-like T cell activation, primarily non-naïve-like T cells will become activated and be susceptible to stimulating conditions that may lead to cell death. For example, in particular stimulating conditions, cell death may result from activation-induced cell death.

In some aspects, the stimulating conditions are such that activation-induced cell death in non-naive-like cells is induced as compared to other conditions, such as standard conditions. Thus, the present invention provides methods for the elimination of at least a substantial portion of any unwanted subpopulation of T cells, e.g., non-naive-like T cells of the input composition. For the purposes of the provided methods, a substantial portion means at least 70% of the unwanted subpopulation of cells, e.g., non-naive-like T cells of the input composition. In certain embodiments, a substantial portion means 75%, 80%, 85%, 90%, 95%, 96%, 97%, 98%, 99% and higher of the unwanted subpopulation of cells, e.g., non-naive-like T cells of the input composition. Elimination of cells, such as from cell death of non-naïve-like T cells, can be measured using any number of techniques, including but not limited to flow cytometric analysis using a variety of antibodies and/or peptide-MHC tetramers and functional assays such as proliferation and chromium release assays.

In some embodiments, an enzyme is added to the input composition to remove residual cell components from the elimination of cells. For example, deoxyribonuclease (DNAse) or a recombinant human deoxyribonuclease I (Pulmozyme^{®}) is added to the input composition in some exemplary embodiments. In some aspects, the stimulating conditions include deoxyribonuclease (DNAse) or recombinant human deoxyribonuclease I (Pulmozyme^{®}).

In some cases, the stimulating conditions do not comprise culture components which are supplemented to preserve particular subsets of T cells, for example non-naive-like T cells. Therefore, in some cases, removal of components from the culture of the stimulating conditions may contribute to elimination of non-naïve-like T cells. In some aspects, the stimulatory condition is carried out, or is additionally carried out, by excluding or reducing the concentration of culture reagents that are known or likely to reduce AICD and/or that promote survival of older cells, such as non-naive cells. In some cases, the stimulatory condition does not include N-acetyl cysteine or includes N-acetyl cysteine in a reduced amount or concentration. In some cases, the stimulatory condition does not include one recombinant IL-7 and/or recombinant IL-15 or includes a reduced amount or concentration of recombinant IL-7 or IL-15. In some embodiments, culture additives could be included that additionally assist or promote the removal of non-naive cells (e.g., toxin-attached to CD45RO).

In certain embodiments, stimulation and/or expansion times may be between 2 and 15 days, between 2 and 12 days, between 2 and 10 days, between 2 and 8 days, between 2 and 6 days, between 2 and 4 days, between 4 and 12 days, between 4 and 10 days, between 4 and 8 days, between 4 and 6 days, between 6 and 12 days, between 6 and 10 days, between 6 and 8 days, between 8 and 12 days, between 8 and 10 days, or between 10 and 12 days. In some embodiments, the cells are incubated for at least 2 days, 3 days, 4 days, 5 days, 6 days, 7 days, 8 days, 9 days, 10 days, 11 days, 12 days, 13 days, 14 days, or more than 14 days. In one embodiment of the methods provided, the mixture may be cultured for 30 minutes to several hours (about 3 hours) to about 14 days or any hourly or minute integer value in between. In another embodiment, the mixture may be cultured for 21 days. In one embodiment, the beads and the T cells are cultured together for about eight days. In another embodiment, the beads and T cells are cultured together for at least or at least about 2-3 days. In another embodiment, the beads and T cells are cultured together for at least or at least about 2 days or at least or at least about 48 hours. In some aspects, several cycles of stimulation may also be desired such that culture time of T cells can be 60 days or more.

The incubation and/or engineering may be carried out in a culture vessel, such as a unit, chamber, well, column, tube, tubing set, valve, vial, culture dish, bag, or other container for culture or cultivating cells. In some embodiments, the compositions or cells are incubated in the presence of stimulating conditions or a stimulatory agent. Such conditions include those designed to induce proliferation, expansion, activation, and/or survival of cells in the population, to mimic antigen exposure, and/or to prime the cells for genetic engineering, such as for the introduction of a recombinant antigen receptor.

The provided methods generally include incubation (e.g., of T-cell containing input compositions) under stimulating conditions. In the context of stimulating T cells, the stimulating conditions generally include a primary agent capable of binding to, ligating, crosslinking, and/or inducing activation via an intracellular signaling domain of, a TCR complex or member thereof, such as CD3. In some embodiments, the stimulating conditions include a primary agent that specifically binds to a member of a TCR complex and a secondary agent that specifically binds to a T cell costimulatory molecule. In some specific examples, the primary agent specifically binds to CD3 and/or the costimulatory molecule is selected from the group consisting of CD28, CD137 (4-1-BB), OX40, or ICOS.

In some embodiments, the stimulating conditions include immobilized anti-CD3 and anti-CD28 antibodies, soluble anti-CD3 antibodies, derivatives of such antibodies, and/or other ligands that engage the TCR/CD3 complex on T cells. In some aspects, the primary agent turns on or initiates TCR/CD3 intracellular signaling cascade in a T cell. Such agents can include binding partners, such as natural ligands and/or antibodies, including antigen-binding antibody fragments, such as those specific for a TCR component such as CD3. Exemplary anti-CD3 antibodies are BC3, OKT3, and G19-4.

In some embodiments, the stimulating conditions further include incubation with a secondary agent, such as an agent capable of inducing a T cell costimulatory signal to a T cell, such as a signal that in combination with a primary signal (e.g., TCR/CD3 ligation), leads to T cell proliferation and/or activation. Exemplary secondary agents are those that specifically bind to, ligate, crosslink, and/or induce intracellular signaling events via, a T cell costimulatory or accessory molecule, such as CD28, CD137 (4-1-BB), OX40, ICOS, CD40, LFA-1, DAP10, and/or CD54. Agents include antibodies, including fragments thereof, natural ligands, and other binding partners. In some embodiments, the secondary agent binds to CD28, such as an anti-CD28 antibody, including antigen-binding antibody fragments. In some aspects, it is an anti-CD28 antibody. Exemplary anti-CD28 antibodies are B-T3 and XR-CD28.

In some embodiments, the binding partner or agent that specifically binds to a particular molecule, such as a T cell stimulatory or costimulatory molecule, include antibodies specific for such molecules, including antigen-binding antibody fragments. In some aspects, the antibody, e.g., anti-CD3 and/or anti-CD28 antibody, is included at a concentration of at least at or about 0.5 ng/mL. In some aspects, the agents include one or more other binding partner for such a molecule, such as a natural binding partner. Agents also may include natural ligands and complexes, including molecules and/or complexes on antigen presenting cells and/or superantigen *(Staphylococcus* enterotoxin A (SEA), *Staphylococcus* enterotoxin B (SEB), Toxic Shock Syndrome Toxin 1 (TSST-1)), endotoxin). Other exemplary agents are those which mimic signaling through a primary or costimulatory T cell signaling molecule, such as a mitogen including a PKC activator, phorbol myristate acetate (PMA), phytohaemagglutinin (PHA), and/or calcium ionophore, e.g., ionomycin, lipopolysaccharide (LPS), T cell mitogen, and cytokines. In some aspects, the conditions include incubation with one or more stimulatory cytokines or other factors, such as IL-2 and/or IL-15. In some aspects, the concentration of cytokine is at least about 10 units/mL.

The primary and secondary (costimulatory) agents, in some embodiments are bound to a solid surface or support, such as a particle, e.g., bead. In some embodiments, the cells can be incubated and/or contacted with a stimulatory agent that is capable of activating and/or expanding T cells. In some aspects, the primary and secondary agents are coupled to a solid surface, such as a particle, e.g., bead. In certain embodiments, the stimulatory agent comprises a particle, e.g., a bead, that is conjugated or linked to one or more agents, e.g., biomolecules, that are capable of activating and/or expanding cells, e.g., T cells. In some embodiments, the one or more agents are bound to a bead. In some embodiments, the bead is biocompatible, i.e., composed of a material that is suitable for biological use. In some embodiments, the beads are non-toxic to cultured cells, e.g., cultured T cells. In some embodiments, the beads may be any particles which are capable of attaching agents in a manner that permits an interaction between the agent and a cell.

In some embodiments, a stimulatory agent comprises one or more agents that are capable of activating and/or expanding cells, e.g., T cells, that are bound to or otherwise attached to a bead, for example to the surface of the bead. In certain embodiments, the bead is a non-cell particle. In particular embodiments, the bead may include a colloidal particle, a microsphere, nanoparticle, a magnetic bead, or the like. In some embodiments the beads are agarose beads. In certain embodiments, the beads are sepharose beads.

In particular embodiments, the stimulatory agent comprises beads that are monodisperse. In certain embodiments, beads that are monodisperse comprise size dispersions having a diameter standard deviation of less than 5% from each other.

In some embodiments, the bead contains one or more agents, such as an agent that is coupled, conjugated, or linked (directly or indirectly) to the surface of the bead. In some embodiments, an agent as contemplated herein can include, but is not limited to, RNA, DNA, proteins (e.g., enzymes), antigens, polyclonal antibodies, monoclonal antibodies, antibody fragments, carbohydrates, lipids lectins, or any other biomolecule with an affinity for a desired target. In some embodiments, the desired target is a T cell receptor and/or a component of a T cell receptor. In certain embodiments, the desired target is CD3. In certain embodiment, the desired target is a costimulatory molecule, e.g., CD28. The one or more agents may be attached directly or indirectly to the bead by a variety of methods. The attachment may be covalent, noncovalent, electrostatic, or hydrophobic and may be accomplished by a variety of attachment means, including for example, a chemical means, a mechanical means, or an enzymatic means. In some embodiments, a biomolecule (e.g., a biotinylated anti-CD3 antibody) may be attached indirectly to the bead via another biomolecule (e.g., anti-biotin antibody) that is directly attached to the bead.

In some embodiments, one or more of the agents attached to the bead is an antibody. The antibody can include a polyclonal antibody, monoclonal antibody (including full length antibodies which have an immunoglobulin Fc region), antibody compositions with polyepitopic specificity, multispecific antibodies (e.g., bispecific antibodies, diabodies, and single-chain molecules, as well as antibody fragments (e.g., Fab, F(ab')2, and Fv). In some embodiments, the stimulatory reagent is an antibody fragment (including antigen-binding fragment), e.g., a Fab, Fab'-SH, Fv, scFv, or (Fab')2 fragment. It will be appreciated that constant regions of any isotype can be used for the antibodies contemplated herein, including IgG, IgM, IgA, IgD, and IgE constant regions, and that such constant regions can be obtained from any human or animal species (e.g., murine species). In some embodiments, the agent is an antibody that binds to and/or recognizes one or more components of a T cell receptor. In particular embodiments, the agent is an anti-CD3 antibody. In certain embodiments, the agent is an antibody that binds to and/or recognizes a co-receptor. In some embodiments, the stimulatory reagent comprises an anti-CD28 antibody.

In some embodiments, the bead has a diameter of greater than about 0.001 µm, greater than about 0.01 µm, greater than about 0.1 µm, greater than about 1.0 µm, greater than about 10 µm, greater than about 50 µm, greater than about 100 µm or greater than about 1000 µm and no more than about 1500µm. In some embodiments, the bead has a diameter of about 1.0 µm to about 500 µm, about 1.0 µm to about 150 µm, about 1.0 µm to about 30 µm, about 1.0 µm to about 10 µm, about 1.0 µm to about 5.0 µm, about 2.0 µm to about 5.0 µm, or about 3.0 µm to about 5.0 µm. In some embodiments, the bead has a diameter of about 3 µm to about 5 µm. In some embodiments, the bead has a diameter of at least or at least about or about 0.001 µm, 0.01 µm, 0.1µm, 0.5µm, 1.0 µm, 1.5 µm, 2.0 µm, 2.5 µm, 3.0 µm, 3.5 µm, 4.0 µm, 4.5 µm, 5.0 µm, 5.5 µm, 6.0 µm, 6.5 µm, 7.0 µm, 7.5 µm, 8.0 µm, 8.5 µm, 9.0 µm, 9.5 µm, 10 µm, 12 µm, 14 µm, 16 µm, 18 µm or 20 µm. In certain embodiments, the bead has a diameter of or about 4.5 µm. In certain embodiments, the bead has a diameter of or about 2.8 µm.

In some embodiments, the beads have a density of greater than 0.001 g/cm³, greater than 0.01 g/cm³, greater than 0.05 g/cm³, greater than 0.1 g/cm³, greater than 0.5 g/cm³, greater than 0.6 g/cm³, greater than 0.7 g/cm³, greater than 0.8 g/cm³, greater than 0.9 g/cm³, greater than 1 g/cm³, greater than 1.1 g/cm³, greater than 1.2 g/cm³, greater than 1.3 g/cm³, greater than 1.4 g/cm³, greater than 1.5 g/cm³, greater than 2 g/cm³, greater than 3 g/cm³, greater than 4 g/cm³, or greater than 5g/cm³. In some embodiments, the beads have a density of between about 0.001 g/cm³ and about 100 g/cm³, about 0.01 g/cm³ and about 50 g/cm³, about 0.1 g/cm³ and about 10 g/cm³, about 0.1 g/cm³ and about .5 g/cm³, about 0.5 g/cm³ and about 1 g/cm³, about 0.5 g/cm³ and about 1.5 g/cm³, about 1 g/cm³ and about 1.5 g/cm³, about 1 g/cm³ and about 2 g/cm³, or about 1 g/cm³ and about 5 g/cm³. In some embodiments, the beads have a density of about 0.5 g/cm³, about 0.6 g/cm³, about 0.7 g/cm³, about 0.8 g/cm³, about 0.9 g/cm³, about 1.0 g/cm³, about 1.1 g/cm³, about 1.2 g/cm³, about 1.3 g/cm³, about 1.4 g/cm³, about 1.5 g/cm³, about 1.6 g/cm³, about 1.7 g/cm³, about 1.8 g/cm³, about 1.9 g/cm³, or about 2.0 g/cm³. In certain embodiments, the beads have a density of about 1.6 g/cm³. In particular embodiments, the beads or particles have a density of about 1.5 g/cm³. In certain embodiments, the particles have a density of about 1.3 g/cm³.

In certain embodiments, a plurality of the beads has a uniform density. In certain embodiments, a uniform density comprises a density standard deviation of less than 10%, less than 5%, or less than 1% of the mean bead density.

In some embodiments, the beads have a surface area of between about 0.001 m² per each gram of particles (m²/g) to about 1,000 m²/g, about 0.010 m²/g to about 100 m²/g, about 0.1 m²/g to about 10 m²/g, about 0.1 m²/g to about 1 m²/g, about 1 m²/g to about 10 m²/g, about 10 m²/g to about 100 m²/g, about 0.5 m²/g to about 20 m²/g, about 0.5 m²/g to about 5 m²/g, or about 1 m²/g to about 4 m²/g. In some embodiments, the particles or beads have a surface area of about 1 m²/g to about 4 m²/g.

In some embodiments, the bead contains at least one material at or near the bead surface that can be coupled, linked, or conjugated to an agent. In some embodiments, the bead is surface functionalized, i.e. comprises functional groups that are capable of forming a covalent bond with a binding molecule, e.g., a polynucleotide or a polypeptide. In particular embodiments, the bead comprises surface-exposed carboxyl, amino, hydroxyl, tosyl, epoxy, and/or chloromethyl groups. In particular embodiments, the beads comprise surface exposed agarose and/or sepharose. In certain embodiments, the bead surface comprises attached stimulatory reagents that can bind or attach binding molecules. In particular embodiments, the biomolecules are polypeptides. In some embodiments, the beads comprise surface exposed protein A, protein G, or biotin.

In some embodiments, the bead reacts in a magnetic field. In some embodiments, the bead is a magnetic bead. In some embodiments, the magnetic bead is paramagnetic. In particular embodiments, the magnetic bead is superparamagnetic. In certain embodiments, the beads do not display any magnetic properties unless they are exposed to a magnetic field.

In particular embodiments, the bead comprises a magnetic core, a paramagnetic core, or a superparamagnetic core. In some embodiments, the magnetic core contains a metal. In some embodiments, the metal can be, but is not limited to, iron, nickel, copper, cobalt, gadolinium, manganese, tantalum, zinc, zirconium or any combinations thereof. In certain embodiments, the magnetic core comprises metal oxides (e.g., iron oxides), ferrites (e.g., manganese ferrites, cobalt ferrites, nickel ferrites, etc.), hematite and metal alloys (e.g., CoTaZn). In some embodiments, the magnetic core comprises one or more of a ferrite, a metal, a metal alloy, an iron oxide, or chromium dioxide. In some embodiments, the magnetic core comprises elemental iron or a compound thereof. In some embodiments, the magnetic core comprises one or more of magnetite (Fe3O4), maghemite (yFe2O3), or greigite (Fe3S4). In some embodiments, the inner core comprises an iron oxide (e.g., Fe₃O₄).

In certain embodiments, the bead contains a magnetic, paramagnetic, and/or superparamagnetic core that is covered by a surface functionalized coat or coating. In some embodiments, the coat can contain a material that can include, but is not limited to, a polymer, a polysaccharide, a silica, a fatty acid, a protein, a carbon, agarose, sepharose, or a combination thereof. In some embodiments, the polymer can be a polyethylene glycol, poly (lactic-co-glycolic acid), polyglutaraldehyde, polyurethane, polystyrene, or a polyvinyl alcohol. In certain embodiments, the outer coat or coating comprises polystyrene. In particular embodiments, the outer coating is surface functionalized.

In some embodiments, the stimulatory reagent comprises a bead that contains a metal oxide core (e.g., an iron oxide core) and a coat, wherein the metal oxide core comprises at least one polysaccharide (e.g., dextran), and wherein the coat comprises at least one polysaccharide (e.g., amino dextran), at least one polymer (e.g., polyurethane) and silica. In some embodiments the metal oxide core is a colloidal iron oxide core. In certain embodiments, the one or more agents include an antibody or antigen-binding fragment thereof. In particular embodiments, the one or more agents include an anti-CD3 antibody and an anti-CD28 antibody. In some embodiments, the stimulatory reagent comprises an anti-CD3 antibody, anti-CD28 antibody, and an anti-biotin antibody. In some embodiments, the stimulatory reagent comprises an anti-biotin antibody. In some embodiments, the bead has a diameter of about 3 µm to about 10 µm. In some embodiments, the bead has a diameter of about 3 µm to about 5 µm. In certain embodiments, the bead has a diameter of about 3.5 µm.

In some embodiments, the stimulatory reagent comprises one or more agents that are attached to a bead comprising a metal oxide core (e.g., an iron oxide inner core) and a coat (e.g., a protective coat), wherein the coat comprises polystyrene. In certain embodiments, the beads are monodisperse, superparamagnetic beads comprising a superparamagnetic iron core, e.g., a core comprising magnetite (Fe₃O₄) and/or maghemite (γFe₂O₃) c and a polystyrene coat or coating. In some embodiments, the bead is non-porous. In some embodiments, the beads contain a functionalized surface to which the one or more agents are attached. In certain embodiments, the one or more agents are covalently bound to the beads at the surface. In some embodiments, the one or more agents include an antibody or antigen-binding fragment thereof. In some embodiments, the one or more agents include an anti-CD3 antibody and an anti-CD28 antibody. In certain embodiments, the beads have a density of about 1.5 g/cm³ and a surface area of about 1 m²/g to about 4 m²/g. In particular embodiments; the beads are monodisperse superparamagnetic beads that have a diameter of about 4.5 µm and a density of about 1.5 g/cm³. In some embodiments, the beads are monodisperse superparamagnetic beads that have a mean diameter of about 2.8 µm and a density of about 1.3 g/cm³.

To effectuate isolation of different T cell populations, exposure times to the particles may be varied. For example, in one preferred embodiment, T cells are isolated by incubation with 3×28 beads, such as DYNABEADS^{®} M-450, or Dynabeads^{®} CD3/CD28 CTS^{™}, for a time period sufficient for positive selection of the desired T cells. In one embodiment, the time period is about 30 minutes. In a further embodiment, the time period is at least 1, 2, 3, 4, 5, or 6 hours. In yet another preferred embodiment, the time period is 10 to 24 hours or more. In one preferred embodiment, the incubation time period is 24 hours. For isolation of T cells from cancer patients, use of longer incubation times, such as 24 hours, can increase cell yield.

When coupled to a surface, the agents may be coupled to the same surface (i.e., in "cis" formation) or to separate surfaces (i.e., in "trans" formation). Alternatively, one agent may be coupled to a surface and the other agent in solution. In one embodiment, the agent providing the co-stimulatory signal is bound to a cell surface and the agent providing the primary activation signal is in solution or coupled to a surface. In a preferred embodiment, the two agents are immobilized on beads, either on the same bead, i.e., "cis," or to separate beads, i.e., "trans." By way of example, the agent providing the primary activation signal is an anti-CD3 antibody and the agent providing the co-stimulatory signal is an anti-CD28 antibody; and both agents are co-immobilized to the same bead in equivalent molecular amounts. In one embodiment, a 1:1 ratio of each antibody bound to the beads for CD4+ T cell expansion and T cell growth is used. In certain aspects of the present invention, a ratio of anti CD3:CD28 antibodies bound to the beads is used such that an increase in T cell expansion is observed as compared to the expansion observed using a ratio of 1:1. In one particular embodiment an increase of from about 0.5 to about 3 fold is observed as compared to the expansion observed using a ratio of 1:1. In one embodiment, the ratio of CD3 :CD28 antibody bound to the beads ranges from 100: 1 to 1:100 and all integer values there between. In one aspect of the present invention, more anti-CD28 antibody is bound to the particles than anti-CD3 antibody, i.e., the ratio of CD3:CD28 is less than one. In certain embodiments of the invention, the ratio of anti CD28 antibody to anti CD3 antibody bound to the beads is greater than 2: 1. In one particular embodiment, a 1:200 CD3:CD28 ratio of antibody bound to beads is used. In one particular embodiment, a 1:150 CD3:CD28 ratio of antibody bound to beads is used. In one particular embodiment, a 1:100 CD3:CD28 ratio of antibody bound to beads is used. In another embodiment, a 1:75 CD3:CD28 ratio of antibody bound to beads is used. In a further embodiment, a 1:50 CD3:CD28 ratio of antibody bound to beads is used. In another embodiment, a 1:45 CD3:CD28 ratio of antibody bound to beads is used. In another embodiment, a 1:40 CD3:CD28 ratio of antibody bound to beads is used. In another embodiment, a 1:35 CD3:CD28 ratio of antibody bound to beads is used. In another embodiment, a 1:30 CD3:CD28 ratio of antibody bound to beads is used. In another embodiment, a 1:25 CD3:CD28 ratio of antibody bound to beads is used. In another embodiment, a 1:20 CD3:CD28 ratio of antibody bound to beads is used. In another embodiment, a 1:15 CD3:CD28 ratio of antibody bound to beads is used. In one embodiment, a 1:10 CD3:CD28 ratio of antibody bound to beads is used. In another embodiment, a 1:5 CD3:CD28 ratio of antibody bound to beads is used. In another embodiment, a 1:4 CD3:CD28 ratio of antibody bound to beads is used. In another embodiment, a 1:3 CD3:CD28 ratio of antibody bound to the beads is used. In yet another embodiment, a 3:1 CD3:CD28 ratio of antibody bound to the beads is used.

In some embodiments, the agent, e.g., antibody, is added in soluble form to the culture or composition. In some embodiments, one agent is included in soluble form and another agent is included coupled to the solid support. In some aspects, where two or more agents are coupled to solid support(s), two agents are coupled to the same support or particle, e.g., incubation with anti-CD3/anti-CD28 beads, the beads containing antibodies recognizing CD3 and CD28. In other aspects, the two or more agents are coupled to separate supports, such as separate beads. For example, anti-CD3 and anti-CD28 beads in some aspects are added separately to the culture.

In some embodiments, the culture conditions include artificial antigen presenting cells. For example, the surface in some aspects is an artificial antigen presenting cell loaded with an agent or agents able to potentiate a signal through a TCR complex, such as anti-CD3 and/or anti-CD28 antibodies. Exemplary antigen presenting cells are genetically modified cells such as myeloid cells (e.g., K562 or U937) engineered to express Fc receptors, such as the CD32 intermediate-affinity or the CD64 high-affinity Fc receptors. Such cells may be loaded with anti-CD3 and/or anti-CD28 antibodies recognized by the Fc receptors and incubated with the T cells to deliver the signal in the culture. Exemplary artificial APCs and ratios and methods of use thereof in culture conditions are described for example, in Suhoski et al., Molecular Therapy (2007) 15 5, 981-988; Thomas et al., Clin Immunol (2002) 105(3): 259-72; Kim et al., Nature Biotechnology 22, 403 - 410 (2004). In some embodiments, the culture conditions include antigen presenting cells, such as PBMCs, loaded with antigen, such as antigen recognized by the TCR complex or other receptor on the cells to be transduced, such as T cells specific for a particular tumor antigen.

In some embodiments, the preferential expansion or proliferation of naïve-like versus non-naive-like T cells is achieved by use of stimulatory conditions designed to induce a particular strength of signal, such as a strength of stimulatory or activating signal that is higher than a certain level, e.g., to induce cell death of non-naive-like T cells. In some aspects, a stronger signal induces cell death of non-naive-like T cells of the input composition, whereas a weaker signal, does not induce cell death of non-naive-like T cells of the input composition and/or maintains survival of non-naive-like T cells, as compared to a stronger signal. Thus, in some embodiments, a stronger signal preferentially activates or causes cell death of non-naïve-like T cells.

In some aspects, the preferential expansion or proliferation of naïve-like versus non-naïve-like T cells is achieved by a particular ratio of beads to cells. In some embodiments, any stimulatory condition that bias expansion or survival or naive-like T cells over non-naive-like T cells can be employed. In some embodiments, the stimulation conditions includes incubation in the presence of a bead reagent containing a primary and/or secondary signal for T cell activation, e.g. anti-CD3/anti-CD28 bead reagent, such as provided in an amount that favors proliferation and/or survival of naïve-like cells and/or that preferentially induces activation induced cell death (AICD) of non-naive like cells. In some cases, such bead reagents are incubated with cells at a ratio of beads to cells of 1:1 or higher, which, in some aspects, can drive toward more AICD and increasing surviving percentage of naïve-like cells, See e.g. Kalamasz et al., J Immunother. (2004) 27(5):405-418 and U.S. Patent Nos.: 7,977,095 and 9,528,088. In one embodiment, the ratio is from about 50:1 to about 5:1. In certain embodiments, the ratio is from about 100:1 to about 1:1. In one embodiment the ratio is at least about 45:1. In certain embodiments, the ratio is at least about 40:1, 35:1, 30:1, 25:1, 20:1, 15:1, 14:1, 13:1, 12:1, 11:1, 10:1, 9:1, 8:1, 7:1, 6:1, 5:1, 4:1, 3:1, 2:1 or 1:1. In some aspects, a bead to cell ratio of less than 5:1 or less than 3:1 is used. In one particular embodiment the ratio is about 3:1.

In one embodiment of the provided methods, bead:cell ratios can be tailored to obtain a desired T cell phenotype. In one particular embodiment, bead:cell ratios can be varied to selectively expand or delete subsets of T cells. In one embodiment, the particular bead:cell ratio used selectively induces cell death in non-naive-like T cells or cells derived from non-naive-like T cells of the input composition. In a further embodiment, the particular bead:cell ratio used selectively expands naïve-like T cells or cells derived from naive-like T cells. In some embodiments, a particular ratio can be used as long as the desired expansion or deletion of subsets of T cells occurs. Therefore, the compositions and methods described herein can be used to expand specific populations of T cells, or to delete specific populations of T cells, for use in any variety of immunotherapeutic settings described herein.

Also provided are stimulating conditions appropriate for the method of incubating (e.g., stimulating) T cells. Conditions appropriate for T cell culture include an appropriate media (e.g., OpTmizer^{™} (Gibco), or Minimal Essential Media or RPMI Media 1640 or, X-vivo 15, (BioWhittaker)) that may contain factors necessary for proliferation and viability, including serum (e.g., fetal bovine or human serum), serum replacement products, or interleukin-2 (IL-2), insulin, or any other additives for the growth of cells. Media can include RPMI 1640, AIM-V, DMEM, MEM, α-MEM, F-12, X-Vivo 15, and X-Vivo 20, with added amino acids and vitamins, either serum-free or supplemented with an appropriate amount of serum (or plasma), or serum replacement or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37° C.) and atmosphere (e.g., air plus 5% CO2).

In another embodiment, the time of exposure to stimulatory agents such as anti-CD3/anti-CD28 (i.e., 3×28)-coated beads may be modified or tailored in such a way to obtain a desired T cell phenotype. One may desire a greater population of helper T cells (TH), typically CD4+ as opposed to CD8+ cytotoxic or regulatory T cells, because an expansion of TH cells could improve or restore overall immune responsiveness. While many specific immune responses are mediated by CD8+ antigen-specific T cells, which can directly lyse or kill target cells, most immune responses require the help of CD4+ T cells, which express important immune-regulatory molecules, such as GM-CSF, CD40L, and IL-2, for example. Where CD4-mediated help is preferred, a method, such as that described herein, which preserves or enhances the CD4:CD8 ratio could be of significant benefit. Increased numbers of CD4+ T cells can increase the amount of cell-expressed CD40L introduced into patients, potentially improving target cell visibility (improved APC function). Similar effects can be seen by increasing the number of infused cells expressing GM-CSF, or IL-2, all of which are expressed predominantly by CD4+ T cells. Likewise, it may be desirable in certain applications to utilize a population of regulatory T cells (e.g., Autoimmun Rev. 2002 August; 1(4): 190-7; Curr Opin Immunol. 2002 December; 14(6):771-8) which can be generated and expanded using the methods described herein. Alternatively, in situations where CD4-help is needed less and increased numbers of CD8+ T cells are desirous, the XCELLERATE^{™} approaches described herein can also be utilized, by for example, pre-selecting for CD8+ cells prior to stimulation and/or culture. Such situations may exist where increased levels of IFN-γ or increased cytolysis of a target cell is preferred. One may also modify time and type of exposure to stimulatory agents to expand T cells with a desired TCR repertoire, e.g., expressing desired Vβ family genes.

Other conditions of the stimulating conditions, in some embodiments, also include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some embodiments, the cells or compositions are assessed and/or adjusted during the incubation steps. For example, assessment and/or adjustment may be at any time subsequent to the initiation of the incubation or culture, such as at a time during the incubation. Assessment can include taking one or more measurements of a composition or vessel containing the cells, such as assessing cells for proliferation rate, degree of survival, phenotype, e.g., expression of one or more surface or intracellular markers, such as proteins or polynucleotides, and/or assessing the composition or vessel for temperature, media component(s), oxygen or carbon dioxide content, and/or presence or absence or amount or relative amount of one or more factors, agents, components, and/or cell types, including subtypes. Assessment can also include assessment for an indicator or predictor of a toxic outcome, such as using an *in vitro* or *ex vivo* as described herein.

In some aspects, the assessment is performed in an automated fashion, for example, using a device as described herein, and/or is set ahead of time to be carried out at certain time-points during incubation. In some aspects, the outcome of the assessment indicates that an adjustment should be made.

Adjustment can include adjusting any cell culture factor or parameter, such as temperature, length (time) for which incubation or a step thereof will be carried out (duration of incubation), replenishment, addition and/or removal of one or more components in the composition being incubated, e.g., media or buffer or components thereof, agents, e.g., nutrients, amino acids, antibiotics, ions, and/or stimulatory factors, such as cytokines, chemokines, antigens, binding partners, fusion proteins, recombinant soluble receptors, or cells or cell types or populations of cells. In some aspects, the removal or addition of various components or other adjustment is carried out in an automated fashion, for example, using a device or system as described herein. In some embodiments, the system is programmed such that an adjustment is automatically initiated based on a certain readout from an interim assessment. For example, in some cases, a system or device is programmed to carry out one or more assessments at a particular time; the system or device in such cases can be further programmed such that a particular outcome of such an assessment, such as a particular ratio of one cell type to another, initiates a particular adjustment, such as addition of one or more of the cell types.

In some aspects, the adjustment is carried out by addition or removal in a way that does not disrupt a closed environment containing the cells and compositions, such as by input and/or removal valves, designed to add or remove components while maintaining sterility, such as in one or more device or system as described herein. Various adjustments of stimulating conditions to favor responses and/or outcomes in particular cell types are disclosed, for example, in US Patent Number 8,617,884, and in U.S. Patent Application Publication No.: US 20030235908 A1.

In some embodiments, the cells are incubated for at or about 1, 2, 3, 4, 5, 6, 7, 8, 9, 10, 11, 12, 13, or 14 days, or at or about 1, 2, 3, 4, or more weeks, either in total or prior to engineering. In some examples the incubation is carried out for greater than or about 1, 2, 3, 4, 5, 6, 7, 8, 9, 10, 11, 12, 13, 14, 15, or 16 days.

In some aspects, incubation is carried out in accordance with techniques such as those described in US Patent No. 6,040,177 to Riddell et al., Klebanoff et al.(2012) J Immunother. 35(9): 651-660, Terakura et al. (2012) Blood.1:72-82, and/or Wang et al. (2012) J Immunother. 35(9):689-701.

In some embodiments, the stimulatory conditions include the addition of feeder cells, such as non-dividing peripheral blood mononuclear cells (PBMC), (e.g., such that the resulting population of cells contains at least about 5, 10, 20, or 40 or more PBMC feeder cells for each T lymphocyte in the initial population to be expanded); and incubating the culture (e.g., for a time sufficient to expand the numbers of T cells). In some aspects, the non-dividing feeder cells can comprise gamma- irradiated PBMC feeder cells. In some embodiments, the PBMC are irradiated with gamma rays in the range of about 3000 to 3600 rads to prevent cell division. In some aspects, the feeder cells are added to culture medium prior to the addition of the populations of T cells.

In some embodiments, the stimulating conditions include temperature suitable for the growth of human T lymphocytes, for example, at least about 25 degrees Celsius, generally at least about 30 degrees, and generally at or about 37 degrees Celsius. Optionally, the incubation may further comprise adding non-dividing EBV-transformed lymphoblastoid cells (LCL) as feeder cells. LCL can be irradiated with gamma rays in the range of about 6000 to 10,000 rads. The LCL feeder cells in some aspects is provided in any suitable amount, such as a ratio of LCL feeder cells to initial T lymphocytes of at least about 10:1.

In embodiments, antigen-specific T cells, such as antigen-specific CD4+ and/or CD8+ T cells, are obtained by stimulating naive or antigen specific T lymphocytes with antigen. For example, antigen-specific T cell lines or clones can be generated to cytomegalovirus antigens by isolating T cells from infected subjects and stimulating the cells in vitro with the same antigen.

Incubation and/or engineering may be carried out in a culture vessel, such as a unit, chamber, well, column, tube, tubing set, valve, vial, culture dish, bag, or other container for culture or cultivating cells.

Also provided are culture-initiating compositions used in the method, such as those containing the T cells, e.g., human primary T cells, stimulating conditions, e.g., the various agents at designed concentrations/ratios for the preferential activation or expansion of non-naive T cells.

In some embodiments, where cells are engineered, e.g., to introduce a genetically engineered antigen receptor, the incubation in the presence of one or more stimulating agents continues during the engineering phase.

Ratios of beads to cells from 1:500 to 500:1 and any integer values in between may be used to stimulate T cells or other target cells. In some cases, the ratio of beads to cells may depend on particle size relative to the target cell. For example, small sized beads could only bind a few cells, while larger beads could bind many. In certain embodiments the ratio of cells to particles ranges from 1:100 to 100:1 and any integer values in-between and in further embodiments the ratio comprises 1:50 to 50:1 and any integer values in between. In another embodiment, the ratio of cells to particles ranges from 1:9 to 9:1 and any integer values in between, can also be used to stimulate T cells. The ratio of anti-CD3- and anti-CD28-coupled particles to T cells that result in T cell stimulation can vary as described herein, however certain preferred values include at least 1:150, 1:125, 1:100, 1:75, 1:50, 1:40, 1:30, 1:20, 1:10, 1:9, 1:8, 1:7, 1:6, 1:5, 1:4, 1:3, 1:2.5, 1:2, 1:1, 2:1, 3:1, 4:1, 5:1, 6:1, 7:1, 8:1, 9:1, 10:1, and 15:1, with one preferred ratio being at least 1:1 beads per T cell. In one particular embodiment, the preferred ratio of beads to cells is 3:1. In some cases, the ratio of beads to cells is 1:3.

In further embodiments, the ratio of particles to cells can be varied depending on the day of stimulation. For example, in one embodiment, the ratio of particles to cells is from 1:1 to 10:1 on the first day and additional particles are added to the cells every day or every other day thereafter for up to 10 days, at final ratios of from 1:1 to 1:10 (based on cell counts on the day of addition). In another embodiment, the ratio of particles to cells is at least about 1:2.5 on the first day and additional particles are added to the cells on day 5 at about 1:10, 1:25, 1:50 or 1:100, on day 7 at 1:10, 1:25, 1:50, or 1:100 and on day 9 at 1:10, 1:25, 1:50, or 1:100. In one particular embodiment, the ratio of particles to cells is 1:1 on the first day of stimulation and adjusted to 1:5 on the third and fifth days of stimulation. In another embodiment, particles are added on a daily or every other day basis to a final ratio of 1:1 on the first day, and 1:5 on the third and fifth days of stimulation. In another embodiment, the ratio of particles to cells is 2:1 on the first day of stimulation and adjusted to 1:10 on the third and fifth days of stimulation. In another embodiment, particles are added on a daily or every other day basis to a final ratio of 1:1 on the first day, and 1:10 on the third and fifth days of stimulation. In some aspects, a variety of other ratios may be suitable for use in the present invention. In particular, ratios will vary depending on particle size and on cell size and type.

One aspect of the present invention relates to the observation that using different bead (e.g. anti-CD3/anti-CD28 bead reagent) to cell ratios can lead to different outcomes with respect to expansion of antigen-specific T cells. In particular, bead to cell ratios can be varied to selectively expand or delete antigen-specific or antigen-experienced (such as memory or effector or activated) T cells, as opposed to other cell types such as naive or naive like T cells. In one embodiment, the particular bead to cell ratio used selectively deletes antigen-specific T cells. Specifically, bead to cell ratios, such as bead to cell ratios at or greater than 1:1 and/or high bead to cell ratios, such as at least or at about 3:1, 5:1, 10:1, 15:1, 20:1, 25:1, 30:1, 35:1, 40:1, 45:1, 50:1, and higher, can induce deletion of antigen-specific T cells. Without being bound by theory, it is thought that the antigen-specific T cells are sensitized to further stimulation. Thus, in some embodiments, the strength of the activation signal delivered to a T cell via the reagent or composition may impact expansion and/or death of one or more specific T cell subsets. In some aspects, selective expansion of memory T cells (antigen-specific T cells) may occur with signals via the TCR and/or coreceptors that are weak compared to others; whereas in some embodiments, selective deletion of memory T cells and/or other antigen experienced T cells, while sparing naive cells, may occur following incubation with reagents delivering signals that are relatively stronger. The quantity of the CD3/TCR (and CD28) receptors that are bound by ligands, among other factors, may in some aspects determine or contribute to determination of the signal strength. Thus, stimulation with high bead to cell ratios in some contexts may provide a high concentration of stimulating antibody (i.e., "strong" signal), leading to over-stimulation of antigen-specific T cells, causing them to die, either by apoptosis or other mechanisms. Thus, in this regard, the bead compositions described herein may in some contexts and/or with respect to certain compositions be functioning as a pro-apoptotic composition. In some aspects, the signal should not be too strong to also kill the naïve-like T cells, such as also by activation-induced cell death. In some embodiments,, the ratio of a stimulatory bead reagent, e.g. anti-CD3/anti-CD28 bead reagent, is less than 10:1.

Further, in this regard, in certain embodiments, such a reagent or composition used as pro-apoptotic conditions, e.g., with respect to certain cell types (e.g., a surface having attached thereto an agent that stimulates a cell surface moiety, such as the bead compositions described herein) is used to expand the remaining population of cells for use in any variety of immunotherapeutic settings as described herein. In a further embodiment, the particular bead to cell ratio used selectively expands naïve-like T cells. The particular ratio may be adjusted to generate desired expansion of particular T cells or deletion of particular T cells. Therefore, the compositions and methods described herein can be used to expand specific populations of T cells in the population of the input composition, or to delete specific populations of T cells in the population of the input composition, for use in any variety of immunotherapeutic settings described herein.

Further, in this regard, in certain embodiments, the same reagent or substance or composition used as a pro-apoptotic composition such as with respect to certain cell types (e.g., a surface having attached thereto an agent that stimulates a cell surface moiety, such as the bead compositions described herein) is used to expand the remaining population of cells for use in any variety of immunotherapeutic settings as described herein. Using lower bead to cell ratios provides a stimulation signal to antigen-specific T cells that does not over-stimulate, but rather induces rapid proliferation of these cells. In a further embodiment, the particular bead to cell ratio used selectively expands antigen-specific T cells. In some aspects, any ratio can be used as long as the desired expansion or deletion occurs. Therefore, the compositions and methods described herein can be used to expand specific populations of T cells, or to delete specific populations of T cells, for use in any variety of immunotherapeutic settings described herein.

Using certain methodologies it may be advantageous to maintain long-term stimulation of a population of T cells following the initial activation and stimulation, by separating the T cells from the stimulus after a period of 2 about 14 days. The rate of T cell proliferation is monitored periodically (e.g., daily) by, for example, examining the size or measuring the volume of the T cells, such as with a Coulter Counter. In this regard, a resting T cell has a mean diameter of about 6.8 microns, and upon initial activation and stimulation, in the presence of the stimulating ligand, the T cell mean diameter will increase to over 12 microns by day 4 and begin to decrease by about day 6. When the mean T cell diameter decreases to approximately 8 microns, the T cells may be reactivated and restimulated to induce further proliferation of the T cells. Alternatively, the rate of T cell proliferation and time for T cell re-stimulation can be monitored by assaying for the presence of cell surface molecules, such as, CD154, CD54, CD25, CD137, CD134, which are induced on activated T cells.

For inducing long-term stimulation of a population of CD4+ and/or CD8+ T cells, it may be necessary to reactivate and re-stimulate the T cells with a stimulatory agent such as an anti-CD3 antibody and an anti-CD28 antibody (e.g., B-T3, XR-CD28 (Diaclone, Besançon, France)) several times to produce a population of CD4+ or CD8+ cells increased in number from about 10 to about 1,000-fold the original T cell population. For example, in one embodiment of the present invention, T cells are stimulated as described for 2-3 times. In further embodiments, T cells are stimulated as described for 4 or 5 times. Using the present methodology, it is possible to achieve T cell numbers from about 100 to about 100,000-fold that have increased polyclonality as compared to prior to stimulation. Moreover, T cells expanded by the method of the present invention secrete substantial levels of cytokines (e.g., IL-2, IFN-γ, IL-4, GM-CSF and TNF-α) into the culture supernatants. For example, as compared to stimulation with IL-2, CD4+ T cells expanded by use of anti-CD3 and anti-CD28 co-stimulation secrete high levels of GM-CSF and TNF-α into the culture medium. These cytokines can be purified from the culture supernatants or the supernatants can be used directly for maintaining cells in culture. Similarly, the T cells expanded by the method of the present invention together with the culture supernatant and cytokines can be administered to support the growth of cells in vivo.

In one embodiment, T cell stimulation is performed, for example with anti-CD3 and anti-CD28 antibodies co-immobilized on beads (3×28 beads), for a period of time sufficient for the cells to return to a quiescent state (low or no proliferation) (approximately 8-14 days after initial stimulation). The stimulation signal is then removed from the cells and the cells are washed and infused back into the patient. The cells at the end of the stimulation phase are rendered "super-inducible" by the methods of the present invention, as demonstrated by their ability to respond to antigens and the ability of these cells to demonstrate a memory-like phenotype, as is evidence by the examples. Accordingly, upon re-stimulation either exogenously or by an antigen in vivo after infusion, the activated T cells demonstrate a robust response characterized by unique phenotypic properties, such as sustained CD 154 expression, increased cytokine production, etc.

In further embodiments of the present invention, the cells, such as T cells are combined with agent-coated or conjugated beads, the beads and the cells are subsequently separated, and then the cells are cultured. In an alternative embodiment, prior to culture, the agent-coated or conjugated beads and cells are not separated but are cultured together. In a further embodiment, the beads and cells are first concentrated by application of a force, resulting in cell surface moiety ligation, thereby inducing cell stimulation and/or polarization of the activation signal.

By way of example, when T cells are the target cell population, the cell surface moieties may be ligated by allowing paramagnetic beads to which anti-CD3 and anti-CD28 antibodies are attached (3 ×28 beads) to contact the T cells prepared. In one embodiment the cells (for example, 104 to 109 T cells) and beads (for example, DYNABEADS^{®} M-450 CD3/CD28 T paramagnetic beads at a ratio of 1:1) are combined in a buffer, preferably PBS (without divalent cations such as, calcium and magnesium). In some aspects, any cell concentration may be used. For example, the target cell may be very rare in the sample and comprise only 0.01% of the sample or the entire sample (i.e., 100%) may comprise the target cell of interest. Accordingly, any cell number is within the context of the present invention. In certain embodiments, it may be desirable to significantly decrease the volume in which particles and cells are mixed together (i.e., increase the concentration of cells), to ensure maximum contact of cells and particles. For example, in one embodiment, a concentration of about 2 billion cells/mL is used. In another embodiment, greater than 100 million cells/mL is used. In a further embodiment, a concentration of cells of 10, 15, 20, 25, 30, 35, 40, 45, or 50 million cells/mL is used. In yet another embodiment, a concentration of cells from 75, 80, 85, 90, 95, or 100 million cells/mL is used. In further embodiments, concentrations of 125 or 150 million cells/mL can be used. Using high concentrations can result in increased cell yield, cell activation, and cell expansion. Further, use of high cell concentrations allows more efficient capture of cells that may weakly express target antigens of interest, such as CD28-negative T cells. Such populations of cells may have therapeutic value and would be desirable to obtain. For example, using high concentration of cells allows more efficient selection of CD8+ T cells that normally have weaker CD28 expression.

In a related embodiment, it may be desirable to use lower concentrations of cells. By significantly diluting the mixture of T cells and particles, interactions between particles and cells is minimized. This selects for cells that express high amounts of desired antigens to be bound to the particles. For example, CD4+ T cells express higher levels of CD28 and are more efficiently captured and stimulated than CD8+ T cells in dilute concentrations. In one embodiment, the concentration of cells used is about 5×10⁶/mL. In other embodiments, the concentration used can be from about 1 ×10⁵/mL to about 1 ×106/mL, and any integer value in between.

The buffer that the cells are suspended in may be any that is appropriate for the particular cell type. When utilizing certain cell types the buffer may contain other components, e.g., 1-5% serum, necessary to maintain cell integrity during the process. In another embodiment, the cells and beads may be combined in cell culture media. The cells and beads may be mixed, for example, by rotation, agitation or any means for mixing, for a period of time ranging from one minute to several hours. The container of beads and cells is then concentrated by a force, such as placing in a magnetic field. Media and unbound cells are removed and the cells attached to the beads or other surface are washed, for example, by pumping via a peristaltic pump, and then resuspended in media appropriate for cell culture.

In one embodiment of the present invention, the mixture may be cultured for 30 minutes to several hours (about 3 hours) to about 14 days or any hourly or minute integer value in between. In another embodiment, the mixture may be cultured for 21 days. In one embodiment of the invention the beads and the T cells are cultured together for about eight days. In another embodiment, the beads and T cells are cultured together for 2-3 days. As described above, several cycles of stimulation may also be desired such that culture time of T cells can be 60 days or more. Conditions appropriate for T cell culture include an appropriate media (e.g., Minimal Essential Media or RPMI Media 1640 or, X-vivo 15, (BioWhittaker)) that may contain factors necessary for proliferation and viability, including serum (e.g., fetal bovine or human serum) or interleukin-2 (IL-2), insulin, or any other additives for the growth of cells. Media can include RPMI 1640, AIM-V, DMEM, MEM, α-MEM, F-12, X-Vivo 15, and X-Vivo 20, with added amino acids and vitamins, either serum-free or supplemented with an appropriate amount of serum (or plasma) or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37° C.) and atmosphere (e.g., air plus 5% CO2).

In one embodiment of the present invention, bead:cell ratios can be tailored to obtain a desired T cell phenotype. In one particular embodiment, bead:cell ratios can be varied to selectively expand or delete antigen-specific (memory) T cells. In one embodiment, the particular bead:cell ratio used selectively deletes antigen-specific T cells. In a further embodiment, the particular bead:cell ratio used selectively expands antigen-specific T cells. In some aspects, any ratio can be used as long as the desired expansion or deletion of antigen-specific T cells occurs. Therefore, the compositions and methods described herein can be used to expand specific populations of T cells, or to delete specific populations of T cells, for use in any variety of immunotherapeutic settings described herein.

In another embodiment, the time of exposure to stimulatory agents such as anti-CD3/anti-CD28 (i.e., 3 ×28)-coated beads may be modified or tailored in such a way to obtain a desired T cell phenotype. Alternatively, a desired population of T cells can be selected using any number of selection techniques, prior to stimulation. One may desire a greater population of helper T cells (TH), typically CD4+ as opposed to CD8+ cytotoxic or regulatory T cells, because an expansion of TH cells could improve or restore overall immune responsiveness. While many specific immune responses are mediated by CD8+ antigen-specific T cells, which can directly lyse or kill target cells, most immune responses require the help of CD4+ T cells, which express important immune-regulatory molecules, such as GM-CSF, CD40L, and IL-2, for example. Where CD4-mediated help is preferred, a method, such as that described herein, which preserves or enhances the CD4:CD8 ratio could be of significant benefit. Increased numbers of CD4+ T cells can increase the amount of cell-expressed CD40L introduced into patients, potentially improving target cell visibility (improved APC function). Similar effects can be seen by increasing the number of infused cells expressing GM-CSF, or IL-2, all of which are expressed predominantly by CD4+ T cells. Likewise, it may be desirable in certain applications to utilize a population of regulatory T cells (e.g., Autoimmun Rev. 2002 August; 1(4): 190-7; Curr Opin Immunol. 2002 December; 14(6):771-8) which can be generated and expanded using the methods described herein. Alternatively, in situations where CD4-help is needed less and increased numbers of CD8+ T cells are desirous, the XCELLERATE^{™} approaches described herein can also be utilized, by for example, pre-selecting for CD8+ cells prior to stimulation and/or culture. Such situations may exist where increased levels of IFN-γ or increased cytolysis of a target cell is preferred. One may also modify time and type of exposure to stimulatory agents to expand T cells with a desired TCR repertoire, e.g., expressing desired Vβ family genes.

To effectuate isolation of different T cell populations, exposure times to the particles may be varied. For example, in one preferred embodiment, T cells are isolated by incubation with 3×28 beads, such as DYNABEADS^{®} M-450, for a time period sufficient for positive selection of the desired T cells. In one embodiment, the time period is about 30 minutes. In a further embodiment, the time period is at least 1, 2, 3, 4, 5, or 6 hours. In yet another preferred embodiment, the time period is 10 to 24 hours or more. In one preferred embodiment, the incubation time period is or is about 24 hours. For isolation of T cells from cancer patients, use of longer incubation times, such as at least or at least about 24 hours, can increase cell yield.

In certain embodiments, total stimulation and/or expansion times may be between 2 and 15 days, between 2 and 12 days, between 2 and 12 days, between 2 and 8 days, between 2 and 6 days, between 2 and 4 days, between 4 and 12 days, between 4 and 10 days, between 4 and 8 days, between 4 and 6 days, between 6 and 12 days, between 6 and 10 days, between 6 and 8 days, between 8 and 12 days, between 8 and 10 days, or between 10 and 12 days, all ranges inclusive. In some embodiments, the cells are incubated and/or incubated with a stimulating reagent (for example, a particle as described herein) for at least or at least about 2 days, 3 days, 4 days, 5 days, 6 days, 7 days, 8 days, 9 days, 10 days, 11 days, 12 days, 13 days, 14 days, or more than 14 days.. When stimulation of T cells is carried out for shorter periods of time, the population of T cells may not increase in number, may not increase in number as dramatically, may decrease in number, or may remain the same in number, but the population will provide more robust and healthy activated T cells that can continue to proliferate in vivo and/or more closely resemble the natural effector T cell pool. For example, when stimulation of T cells is carried out for shorter periods of time, the population of T cells may comprise a greater percentage and/or proportion of naive or naïve-like T cells, or engineered T cells, as compared to a parallel process wherein stimulation of T cells is carried out for a longer period of time. In some embodiments, a shorter period of time may be characterized by a total incubation time, in some embodiments a total incubation time with a stimulatory agent, of fewer than or fewer than about 6 days, fewer than or fewer than about 5 days, fewer than or fewer than about 4 days, fewer than or fewer than about 3 days, or fewer than or fewer than about 2 days. As the availability of T cell help is often the limiting factor in antibody responses to protein antigens, the ability to selectively expand or selectively infuse a CD4+ rich population of T cells into a subject is extremely beneficial. Further benefits of such enriched populations are readily apparent in that activated helper T cells that recognize antigens presented by B lymphocytes deliver two types of stimuli, physical contact and cytokine production, that result in the proliferation and differentiation of B cells.

In some cases, the stimulating conditions do not comprise culture components or agents which are supplemented to preserve particular subsets of T cells, for example non-naïve-like T cells. Therefore, in some cases, removal of components or agents from the culture of the stimulating conditions may contribute to elimination of non-naïve-like T cells. In some embodiments, the stimulating conditions do not include agents, such as N-acetyl cysteine, which can be used to modulate and/or fine-tune the intensity of TCR/CD3 signaling. In some aspects, such agents are removed or reduced at amounts/ratios to increase the strength of the activating signal. In some aspects, the stimulatory condition is carried out, or is additionally carried out, by excluding or reducing the concentration of culture reagents that are known or likely to reduce AICD and/or that promote survival of older cells, such as non-naive cells. In some cases, the stimulatory condition does not include N-acetyl cysteine or includes N-acetyl cysteine in a reduced amount or concentration. In some cases, the stimulatory condition does not include one recombinant IL-7 and/or recombinant IL-15 or includes a reduced amount or concentration of recombinant IL-7 or IL-15. In some embodiments, culture additives could be included that additionally assist or promote the removal of non-naive cells (e.g., toxin-attached to CD45RO).

### C. Stimulated Composition

Also provided is a stimulated composition produced by the methods of incubating (e.g., stimulating) described. In some embodiments, cells of the stimulated composition are further engineered, e.g., to introduce a genetically engineered antigen receptor, after the incubation of the input composition. In some embodiments, the method further includes incubating in the presence of one or more stimulating agents during the genetic engineering phase.

The cells of the stimulated composition that have not undergone apoptosis using the methods for incubating (e.g., stimulating) described herein, can increase polyclonality of said remaining population of T cells as measured by the breadth of the response of the population to a given antigen. Restoration or increase in polyclonality can be measured by determining the breadth of response to a particular antigen of interest, for example by measuring the number of different epitopes recognized by antigen-specific cells. This can be carried out using standard techniques for generating and cloning antigen-specific T cells in vitro.

In some embodiments of the methods provided herein, the culture conditions preferentially induce expansion, proliferation, and/or survival non-naïve-like T cells compared to naive-like T cells. Preferential expansion, proliferation, and/or survival of a first cell type or population as compared to a second cell type or population means that the relative expansion, proliferation, and/or survival is greater for the first type or population than the second. This can include a scenario in which the percentage of the first cell type or population expanded, proliferated, and/or survived is greater, and/or that the degree (e.g., overall or average degree among the cells of the population or type) to which the cells are expanded, proliferated, and/or survived is greater for the first population or type than the second.

In some embodiments, the preferential expansion, proliferation, and/or survival is expressed by comparing percentage of naive-like cells in the input composition with the percentage of engineered cells in the stimulated composition that are derived from naïve-like cells in the original input composition. In some examples, the percentage of engineered cells in the stimulated composition that are derived from naive-like cells in the original input composition are generally larger than the former under the simulating conditions described herein. For example, in one embodiment, the percentage of cells in the stimulated composition that are derived from naïve-like cells in the original input composition is greater than the percentage of naive cells in the culture-initiating composition. In one aspect, the input composition (or T cells therein) includes at or about 70% naïve-like T cells and 30% non-naïve-like T cells, whereas greater than 50 %, e.g., at least 70%, of the engineered cells in the resulting stimulated composition are derived from naïve-like cells in the input composition. In some cases, using other conditions, such as stimulation with agent(s) that induce a strong signal via the TCR complex, would result in fewer than 50 %, e.g., at or about 5-10 % of the cells in the stimulated composition being of non-naïve-like origin. In some embodiments, the simulating conditions induce a strong signal that induces activation-induced cell death in non-naïve-like T cells of the input composition.

In some embodiments, the percent of naïve-like T cells or cells derived from the naïve-like T cells of the input composition in the stimulated composition is increased greater than or greater than about 1.5-fold, 2-fold, 3-fold, 4-fold, 5-fold, 6-fold, 7-fold, 8-fold, 9-fold, 10-fold, 50-fold, 100-fold compared to the input composition. In some cases, the ratio of cells derived from the naïve-like T cells of the input composition compared to cells derived from the non-naïve-like T cells of the input composition or the ratio of naïve-like T cells compared to non-naïve-like T cells in the stimulated composition is increased greater than or greater than about 1.5-fold, 2-fold, 3-fold, 4-fold, 5-fold, 6-fold, 7-fold, 8-fold, 9-fold, 10-fold, 50-fold, 100-fold compared to the ratio of the naïve-like T cells compared to non-naive-like T cells in the input composition. In some embodiments, the stimulated composition comprises greater than 75%, 85%, 90%, 91%, 92%, 93%, 94%, 95%, 96%, 97%, 98%, 99% of cells that are derived from naive-like T cells of the input composition.

In some embodiments of using the method of stimulating T cells described herein, of the cells in the input composition, a greater percentage of the naïve-like T cells, as compared to the non-naive-like T cells, are induced to proliferate and/or become activated. In some aspects, the stimulated composition resulting from the methods of stimulating described herein contains less than 10% of cells derived from non-naive like T cells. In some cases, the stimulated composition contains less than 9%, 8%, 7%, 6%, 5%, 4%, 3%, 2%, 1%, 0.5% or 0.1% cells derived from non-naive T cells. In some embodiments, a greater percentage of the T cells that were naïve-like in the input composition, as compared to the percentage of the T cells that were non-naïve-like in the input composition, are dividing at day 1, 2, 3, 4, 5, 6, 7, 8, 9, or 10 following initiation of said incubation. In some cases, the stimulating conditions induce cell death. In particular examples, the stimulating conditions of the method induce activation of non-naïve-like T cells thereby inducing activation-induced cell death (AICD).

In some embodiments, the method includes stimulating conditions that are capable of inducing proliferation of a greater percentage of cells of the naïve-like T cell population, as compared to the non-naïve-like T cells, at day 1, 2, 3, 4, 5, 6, 7, 8, 9, or 10 following initiation of incubation under the conditions.

In some embodiments, the stimulating conditions produce a stimulated composition, wherein the stimulating conditions preferentially induces expansion of naïve-like T cells such that a target amount of T cells of the stimulated composition derived from naïve-like T cells of the input composition are produced. In some embodiments, the stimulated composition is further adjusted to achieve a preferred CD4:CD8 ratio of T cells. In some cases, particular cell populations can be removed from the stimulated composition to achieve a preferred CD4:CD8 ratio.

In some embodiments, the stimulating conditions, e.g., under which input compositions are incubated, preferentially induce expansion, proliferation, and/or survival of naïve-like T cells or a subset thereof as compared to non-naïve-like T cells or a subset thereof. In some aspects, the stimulating conditions are strongly activate non-naïve-like T cells of the input composition, thereby activating cell death particularly in non-naïve-like cells T of the input composition. In some aspects, this thereby preferentially favors survival of naïve-like T cells. In some aspects, such stimulating conditions lead to a reduced level of toxicity and/or toxicity-associated outcome(s) or symptom(s) following administration to subjects of the cells and compositions produced by the methods.

Whether a particular response has been induced or is preferentially induced in one population over another may be measured by a variety of methods. For example, whether a cell is induced to enter cell cycle may be measured by flow cytometry-based methods, including those involving the use of dyes and other agents, such as CFSE and other interchelating agents to assess cell division followed by flow cytometric assessment, assessment of incorporation of tritium (H3) labeled thymidine and similar agents, and/or cell counts. Comparisons may be made by assessing various pure test populations, such as by comparing naïve and non-naive T cell populations under particular conditions, separately. Activation can be measured, for example, by the secretion of various cytokines and/or upregulation or expression of various activation markers including CD25, CD69, and/or cell size (e.g., forward scatter as measured by flow cytometry). Survival and/or apoptosis may be assessed by various methods, including flow-cytometric methods, the incorporation of various dyes including propidium iodide, and staining with agents such as staining with Annexin V and similar agents.

In some embodiments, the percentage of naïve-like T cells in the input composition is less than the percentage of T cells in the stimulated composition derived from naïve-like T cells in the input composition. In some embodiments, the methods provided herein produce a greater percentage of the cells introduced with the nucleic acid are, or are derived from the proliferation of, naïve-like T cells in the input composition, compared to non-naïve-like T cells in the input composition. Various adjustments of stimulating conditions to favor responses and/or outcomes in particular cell types are disclosed, for example, in US Patent Number 8,617,884, and in U.S. Patent Application Publication No.: US 20030235908 A1.

In some embodiments, the stimulated composition contains cells that express or are derived from cells that express particular markers of naïve-like T cells. For example, the stimulated composition produced by the methods provided are derived from populations of cells that are surface positive for a T cell activation marker selected from the group consisting of CD27, CD28, CD45RA, and CCR7. In some cases, the stimulated composition produced by the methods provided are derived from populations of cells that are surfacenegative for a T cell activation marker such as CD62L. In some aspects, the cells of the stimulated composition are or are derived from cells that are surface negative for CD56 and/or CD45RO. In some particular embodiments, the stimulated composition produced by the methods provided are derived from populations of cells that are CD27+, CD45RA+, CD45RO-, and CCR7+. In some cases, the cells of the stimulated composition are or are derived from cells that are negative for intracellular expression of a cytokine such as IL-2, IFN-γ, IL-4, and/or IL-10. In some further examples, the cells of the stimulated composition are or are derived from cells that are negative for expression of markers CD25 and/or perforin. In some cases, the cells of the stimulated composition are or are derived from cells that are CD95^{lo}.

In some cases, the stimulated composition contains cells that are derived from at least 40 %, at least 50 %, at least 60 %, at least 70 %, at least 80 %, or at least 90 % of T cells that are surface positive for a T cell activation marker such as CD27, CD28, CD45RA, and CCR7 and surface negative for CD62L. In some embodiments, the stimulated composition contains cells that are derived from at least 40 %, at least 50 %, at least 60 %, at least 70 %, at least 80 %, or at least 90 % of the T cells that are surface negative for CD56 and/or CD45RO. In some aspects, the stimulated composition contains cells that are derived from at least 40 %, at least 50 %, at least 60 %, at least 70 %, at least 80 %, or at least 90 % of the T cells that are surface negative for CD45RO and cell surface positive for CD27, CD45RA, and CCR7. In some examples, the stimulated composition contains cells that are derived from at least 40 %, at least 50 %, at least 60 %, at least 70 %, at least 80 %, or at least 90 % of the T cells that are negative for intracellular expression of a cytokine such as IL-2, IFN-γ, IL-4, IL-10. In some aspects, the stimulated composition contains cells that are derived from at least 40 %, at least 50 %, at least 60 %, at least 70 %, at least 80 %, or at least 90 % of T cells that are negative for expression of markers CD25 and/or perforin. In some cases, the stimulated composition contains cells that are derived from at least 40 %, at least 50 %, at least 60 %, at least 70 %, at least 80 %, or at least 90 % of T cells that are CD95^{lo}.

In certain embodiments, the stimulated composition is more polyclonal or multiclonal compared to the input composition. In some embodiments, the stimulated composition is more diverse compared to the input composition. In some embodiments, this increase in polyclonality comprises a shift from mono to oligoclonality or to polyclonality of the T cell population as measured by a Vβ, Vα, Yγ, or Vδ spectratype profile of at least one Vβ, Vα, Vγ, or V6 family gene. The stimulation and activation of the remaining cells that have survived, expanded, proliferated using provided methods, can increase polyclonality of said remaining T cells of the stimulated composition as measured by the breadth of the response of the population to a given antigen. Restoration or increase in polyclonality of the stimulated composition can be measured by determining the breadth of response to a particular antigen of interest, for example by measuring the number of different epitopes recognized by antigen-specific cells. This can be carried out using standard techniques for generating and cloning antigen-specific T cells *in vitro.*

### II. METHODS OF GENETICALLY ENGINEERING CELLS

In some embodiments, the method further includes introducing a genetically engineered recombinant receptor, e.g. chimeric receptor, such as chimeric antigen receptor (CAR), into the stimulated composition of T cells, generating an output composition comprising T cells expressing the genetically engineered recombinant receptor. In some cases, incubating the input composition under stimulating conditions is performed prior to, during and/or subsequent to introducing a nucleic acid encoding a genetically engineered recombinant receptor. In some examples, the introduction is by transduction. In some embodiments, the provided methods produce a stimulated composition that can be uniformly transduced. In some aspects, the nucleic acid contains a viral vector. In some cases, the viral vector is a retroviral vector. In some examples, the viral vector is a lentiviral vector or a gammaretroviral vector. The method, in some embodiments, including the introducing, is performed *in vitro* or *ex vivo.*

Thus, the methods provided herein include one or more steps for preparing cells for genetic engineering. In certain embodiments, the one or more steps include isolating cells from a biological sample, stimulating an input composition of cells, and preparing a composition of cells to be genetically engineered. Also provided are populations of such cells, compositions containing such cells and/or enriched for such cells, such as in which cells expressing the recombinant receptor, e.g. chimeric receptor, make up at least 50, 60, 70, 80, 90, 91, 92, 93, 94, 95, 96, 97, 98, 99, or more percent of the total cells in the composition or cells of a certain type such as T cells or CD8+ or CD4+ cells. Among the compositions are pharmaceutical compositions and formulations for administration, such as for adoptive cell therapy. Also provided are methods for engineering, producing or generating such cells, methods for administering the cells and compositions to subjects, e.g., patients, and methods for detecting, selecting, isolating or separating such cells. Thus, provided are genetically engineered cells expressing the recombinant receptors e.g., CARs.

In some embodiments, the cells include one or more nucleic acids introduced via genetic engineering, and thereby express recombinant or genetically engineered products of such nucleic acids. In some embodiments, the nucleic acids are heterologous, i.e., normally not present in a cell or sample obtained from the cell, such as one obtained from another organism or cell, which for example, is not ordinarily found in the cell being engineered and/or an organism from which such cell is derived. In some embodiments, the nucleic acids are not naturally occurring, such as a nucleic acid not found in nature, including one comprising chimeric combinations of nucleic acids encoding various domains from multiple different cell types.

### A. Genetic Engineering

### 1. Recombinant Antigen Receptors

In some embodiments, engineered cells, such as T cells, are provided that express a CAR with specificity for a particular antigen (or marker or ligand), such as an antigen expressed on the surface of a particular cell type. In some embodiments, the antigen is a polypeptide. In some embodiments, it is a carbohydrate or other molecule. In some embodiments, the antigen is selectively expressed or overexpressed on cells of the disease or condition, e.g., the tumor or pathogenic cells, as compared to normal or non-targeted cells or tissues. In other embodiments, the antigen is expressed on normal cells and/or is expressed on the engineered cells.

In particular embodiments, the recombinant receptor, such as chimeric receptor, contains an intracellular signaling region, which includes a cytoplasmic signaling domain (also interchangeably called an intracellular signaling domain), such as a cytoplasmic (intracellular) region capable of inducing a primary activation signal in a T cell, for example, a cytoplasmic signaling domain of a T cell receptor (TCR) component (e.g. a cytoplasmic signaling domain of a zeta chain of a CD3-zeta (CD3ζ) chain or a functional variant or signaling portion thereof) and/or that comprises an immunoreceptor tyrosine-based activation motif (ITAM).

In some embodiments, the chimeric receptor further contains an extracellular ligand-binding domain that specifically binds to a ligand (e.g. antigen) antigen. In some embodiments, the chimeric receptor is a CAR that contains an extracellular antigen-recognition domain that specifically binds to an antigen. In some embodiments, the ligand, such as an antigen, is a protein expressed on the surface of cells. In some embodiments, the CAR is a TCR-like CAR and the antigen is a processed peptide antigen, such as a peptide antigen of an intracellular protein, which, like a TCR, is recognized on the cell surface in the context of a major histocompatibility complex (MHC) molecule.

Exemplary antigen receptors, including CARs, and methods for engineering and introducing such receptors into cells, include those described, for example, in international patent application publication numbers WO2000/14257, WO2013/126726, WO2012/129514, WO2014031687, WO2013/166321, WO2013/071154, WO2013/123061 U.S. patent application publication numbers US2002131960, US2013287748, US20130149337, U.S. Patent Nos.: 6,451,995, 7,446,190, 8,252,592, 8,339,645, 8,398,282, 7,446,179, 6,410,319, 7,070,995, 7,265,209, 7,354,762, 7,446,191, 8,324,353, and 8,479,118, and European patent application number EP2537416,and/or those described by Sadelain et al., Cancer Discov. 2013 April; 3(4): 388-398; Davila et al. (2013) PLoS ONE 8(4): e61338; Turtle et al., Curr. Opin. Immunol., 2012 October; 24(5): 633-39; Wu et al., Cancer, 2012 March 18(2): 160-75. In some aspects, the antigen receptors include a CAR as described in U.S. Patent No.: 7,446,190, and those described in International Patent Application Publication No.: WO/2014055668 A1. Examples of the CARs include CARs as disclosed in any of the aforementioned publications, such as WO2014031687, US 8,339,645, US 7,446,179, US 2013/0149337, U.S. Patent No.: 7,446,190, US Patent No.: 8,389,282, Kochenderfer et al., 2013, Nature Reviews Clinical Oncology, 10, 267-276 (2013); Wang et al. (2012) J. Immunother. 35(9): 689-701; and Brentjens et al., Sci Transl Med. 2013 5(177). See also WO2014031687, US 8,339,645, US 7,446,179, US 2013/0149337, U.S. Patent No.: 7,446,190, and US Patent No.: 8,389,282.

In some embodiments, the CAR is constructed with a specificity for a particular antigen (or marker or ligand), such as an antigen expressed in a particular cell type to be targeted by adoptive therapy, e.g., a cancer marker, and/or an antigen intended to induce a dampening response, such as an antigen expressed on a normal or non-diseased cell type. Thus, the CAR typically includes in its extracellular portion one or more antigen binding molecules, such as one or more antigen-binding fragment, domain, or portion, or one or more antibody variable domains, and/or antibody molecules. In some embodiments, the CAR includes an antigen-binding portion or portions of an antibody molecule, such as a single-chain antibody fragment (scFv) derived from the variable heavy (VH) and variable light (VL) chains of a monoclonal antibody (mAb).

In some embodiments, the antibody or antigen-binding portion thereof is expressed on cells as part of a recombinant receptor, such as an antigen receptor. Among the antigen receptors are functional non-TCR antigen receptors, such as chimeric antigen receptors (CARs). Generally, a CAR containing an antibody or antigen-binding fragment that exhibits TCR-like specificity directed against peptide-MHC complexes also may be referred to as a TCR-like CAR. In some embodiments, the extracellular antigen binding domain specific for an MHC-peptide complex of a TCR-like CAR is linked to one or more intracellular signaling components, in some aspects via linkers and/or transmembrane domain(s). In some embodiments, such molecules can typically mimic or approximate a signal through a natural antigen receptor, such as a TCR, and, optionally, a signal through such a receptor in combination with a costimulatory receptor.

In some embodiments, the recombinant receptor, such as a chimeric receptor (e.g. CAR), includes a ligand-binding domain that binds, such as specifically binds, to an antigen (or a ligand). Among the antigens targeted by the chimeric receptors are those expressed in the context of a disease, condition, or cell type to be targeted via the adoptive cell therapy. Among the diseases and conditions are proliferative, neoplastic, and malignant diseases and disorders, including cancers and tumors, including hematologic cancers, cancers of the immune system, such as lymphomas, leukemias, and/or myelomas, such as B, T, and myeloid leukemias, lymphomas, and multiple myelomas.

In some embodiments, the antigen (or a ligand) is a polypeptide. In some embodiments, it is a carbohydrate or other molecule. In some embodiments, the antigen (or a ligand) is selectively expressed or overexpressed on cells of the disease or condition, e.g., the tumor or pathogenic cells, as compared to normal or non-targeted cells or tissues. In other embodiments, the antigen is expressed on normal cells and/or is expressed on the engineered cells.

In some embodiments, the CAR contains an antibody or an antigen-binding fragment *(e.g.* scFv) that specifically recognizes an antigen, such as an intact antigen, expressed on the surface of a cell.

In certain embodiments, the antigen is αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), , type III epidermal growth factor receptor mutation (EGFR vIII), epithelial glycoprotein 2 (EPG-2), epithelial glycoprotein 40 (EPG-40), ephrinB2, ephrine receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPRC5D), Her2/neu (receptor tyrosine kinase erbB2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha (IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some embodiments include antigens associated with a B cell malignancy, such as any of a number of known B cell marker. In some embodiments, the antigen is or includes CD20, CD19, CD22, ROR1, CD45, CD21, CD5, CD33, Igkappa, Iglambda, CD79a, CD79b or CD30.

In some embodiments, the antigen is or includes a pathogen-specific or pathogen-expressed antigen. In some embodiments, the antigen is a viral antigen (such as a viral antigen from HIV, HCV, HBV, etc.), bacterial antigens, and/or parasitic antigens.

In some embodiments, the antigen or antigen binding domain is CD19. In some embodiments, the scFv contains a VH and a VL derived from an antibody or an antibody fragment specific to CD19. In some embodiments, the antibody or antibody fragment that binds CD19 is a mouse derived antibody such as FMC63 and SJ25C1. In some embodiments, the antibody or antibody fragment is a human antibody, e.g., as described in U.S. Patent Publication No. US 2016/0152723.

In some embodiments, the scFv is derived from FMC63. FMC63 generally refers to a mouse monoclonal IgG1 antibody raised against Nalm-1 and -16 cells expressing CD19 of human origin (Ling, N. R., *et al.* (1987). *Leucocyte typing III.* 302). The FMC63 antibody comprises CDRH1 and H2 set forth in SEQ ID NOS: 38, 39 respectively, and CDRH3 set forth in SEQ ID NOS: 40 or 54 and CDRL1 set forth in SEQ ID NOS: 35 and CDR L2 36 or 55 and CDR L3 sequences 37 or 34. The FMC63 antibody comprises the heavy chain variable region (V_{H}) comprising the amino acid sequence of SEQ ID NO: 41 and the light chain variable region (V_{L}) comprising the amino acid sequence of SEQ ID NO: 42. In some embodiments, the svFv comprises a variable light chain containing the CDRL1 sequence of SEQ ID NO:35, a CDRL2 sequence of SEQ ID NO:36, and a CDRL3 sequence of SEQ ID NO:37 and/or a variable heavy chain containing a CDRH1 sequence of SEQ ID NO:38, a CDRH2 sequence of SEQ ID NO:39, and a CDRH3 sequence of SEQ ID NO:40. In some embodiments, the scFv comprises a variable heavy chain region of FMC63 set forth in SEQ ID NO:41 and a variable light chain region of FMC63 set forth in SEQ ID NO:42. In some embodiments, the variable heavy and variable light chain are connected by a linker. In some embodiments, the linker is set forth in SEQ ID NO:56. In some embodiments, the scFv comprises, in order, a V_{H}, a linker, and a V_{L}. In some embodiments, the scFv comprises, in order, a V_{L}, a linker, and a V_{H}. In some embodiments, the svFc is encoded by a sequence of nucleotides set forth in SEQ ID NO:57 or a sequence that exhibits at least 85%, 86%, 87%, 88%, 89%, 90%, 91%, 92%, 93%, 94%, 95%, 96%, 97%, 98%, or 99% sequence identity to SEQ ID NO:57. In some embodiments, the scFv comprises the sequence of amino acids set forth in SEQ ID NO:43 or a sequence that exhibits at least 85%, 86%, 87%, 88%, 89%, 90%, 91%, 92%, 93%, 94%, 95%, 96%, 97%, 98%, or 99% sequence identity to SEQ ID NO:43.

In some embodiments the scFv is derived from SJ25C1. SJ25C1 is a mouse monoclonal IgG1 antibody raised against Nalm-1 and -16 cells expressing CD19 of human origin (Ling, N. R., *et al.* (1987). *Leucocyte typing III.* 302). The SJ25C1 antibody comprises CDRH1, H2 and H3 set forth in SEQ ID NOS: 47-49, respectively, and CDRL1, L2 and L3 sequences set forth in SEQ ID NOS: 44-46, respectively. The SJ25C1 antibody comprises the heavy chain variable region (V_{H}) comprising the amino acid sequence of SEQ ID NO: 50 and the light chain variable region (V_{L}) comprising the amino acid sequence of SEQ ID NO: 51. In some embodiments, the svFv comprises a variable light chain containing the CDRL1 sequence of SEQ ID NO:44, a CDRL2 sequence of SEQ ID NO: 45, and a CDRL3 sequence of SEQ ID NO:46 and/or a variable heavy chain containing a CDRH1 sequence of SEQ ID NO:47, a CDRH2 sequence of SEQ ID NO:48, and a CDRH3 sequence of SEQ ID NO:49. In some embodiments, the scFv comprises a variable heavy chain region of SJ25C1 set forth in SEQ ID NO:50 and a variable light chain region of SJ25C1 set forth in SEQ ID NO:51. In some embodiments, the variable heavy and variable light chain are connected by a linker. In some embodiments, the linker is set forth in SEQ ID NO:52. In some embodiments, the scFv comprises, in order, a V_{H}, a linker, and a V_{L}. In some embodiments, the scFv comprises, in order, a V_{L}, a linker, and a V_{H}. In some embodiments, the scFv comprises the sequence of amino acids set forth in SEQ ID NO:53 or a sequence that exhibits at least 85%, 86%, 87%, 88%, 89%, 90%, 91%, 92%, 93%, 94%, 95%, 96%, 97%, 98%, or 99% sequence identity to SEQ ID NO:53.

In some embodiments, the antigen or antigen binding domain is BCMA. In some embodiments, the scFv contains a VH and a VL derived from an antibody or an antibody fragment specific to BCMA. In some embodiments, the antibody or antibody fragment that binds BCMA is or contains a VH and a VL from an antibody or antibody fragment set forth in International Patent Applications, Publication Number WO 2016/090327 and WO 2016/090320.

In some embodiments, the antigen or antigen binding domain is GPRC5D. In some embodiments, the scFv contains a VH and a VL derived from an antibody or an antibody fragment specific to GPRC5D. In some embodiments, the antibody or antibody fragment that binds GPRC5D is or contains a VH and a VL from an antibody or antibody fragment set forth in International Patent Applications, Publication Number WO 2016/090329 and WO 2016/090312.

In some embodiments, the CAR contains a TCR-like antibody, such as an antibody or an antigen-binding fragment (e.g. scFv) that specifically recognizes an intracellular antigen, such as a tumor-associated antigen, presented on the cell surface as a MHC-peptide complex. In some embodiments, an antibody or antigen-binding portion thereof that recognizes an MHC-peptide complex can be expressed on cells as part of a recombinant receptor, such as an antigen receptor. Among the antigen receptors are functional non-TCR antigen receptors, such as chimeric antigen receptors (CARs). Generally, a CAR containing an antibody or antigen-binding fragment that exhibits TCR-like specificity directed against peptide-MHC complexes also may be referred to as a TCR-like CAR.

Reference to "Major histocompatibility complex" (MHC) refers to a protein, generally a glycoprotein, that contains a polymorphic peptide binding site or binding groove that can, in some cases, complex with peptide antigens of polypeptides, including peptide antigens processed by the cell machinery. In some cases, MHC molecules can be displayed or expressed on the cell surface, including as a complex with peptide, *i.e.* MHC-peptide complex, for presentation of an antigen in a conformation recognizable by an antigen receptor on T cells, such as a TCRs or TCR-like antibody. Generally, MHC class I molecules are heterodimers having a membrane spanning α chain, in some cases with three α domains, and a non-covalently associated β2 microglobulin. Generally, MHC class II molecules are composed of two transmembrane glycoproteins, α and β, both of which typically span the membrane. An MHC molecule can include an effective portion of an MHC that contains an antigen binding site or sites for binding a peptide and the sequences necessary for recognition by the appropriate antigen receptor. In some embodiments, MHC class I molecules deliver peptides originating in the cytosol to the cell surface, where a MHC-peptide complex is recognized by T cells, such as generally CD8⁺ T cells, but in some cases CD4+ T cells. In some embodiments, MHC class II molecules deliver peptides originating in the vesicular system to the cell surface, where they are typically recognized by CD4⁺ T cells. Generally, MHC molecules are encoded by a group of linked loci, which are collectively termed H-2 in the mouse and human leukocyte antigen (HLA) in humans. Hence, typically human MHC can also be referred to as human leukocyte antigen (HLA).

The term "MHC-peptide complex" or "peptide-MHC complex" or variations thereof, refers to a complex or association of a peptide antigen and an MHC molecule, such as, generally, by non-covalent interactions of the peptide in the binding groove or cleft of the MHC molecule. In some embodiments, the MHC-peptide complex is present or displayed on the surface of cells. In some embodiments, the MHC-peptide complex can be specifically recognized by an antigen receptor, such as a TCR, TCR-like CAR or antigen-binding portions thereof.

In some embodiments, a peptide, such as a peptide antigen or epitope, of a polypeptide can associate with an MHC molecule, such as for recognition by an antigen receptor. Generally, the peptide is derived from or based on a fragment of a longer biological molecule, such as a polypeptide or protein. In some embodiments, the peptide typically is about 8 to about 24 amino acids in length. In some embodiments, a peptide has a length of from or from about 9 to 22 amino acids for recognition in the MHC Class II complex. In some embodiments, a peptide has a length of from or from about 8 to 13 amino acids for recognition in the MHC Class I complex. In some embodiments, upon recognition of the peptide in the context of an MHC molecule, such as MHC-peptide complex, the antigen receptor, such as TCR or TCR-like CAR, produces or triggers an activation signal to the T cell that induces a T cell response, such as T cell proliferation, cytokine production, a cytotoxic T cell response or other response.

In some embodiments, a TCR-like antibody or antigen-binding portion can be produced (see e.g. US Published Application Nos. US 2002/0150914; US 2003/0223994; US 2004/0191260; US 2006/0034850; US 2007/00992530; US20090226474; US20090304679; and International PCT Publication No. WO 03/068201).

In some embodiments, an antibody or antigen-binding portion thereof that specifically binds to a MHC-peptide complex, can be produced by immunizing a host with an effective amount of an immunogen containing a specific MHC-peptide complex. In some cases, the peptide of the MHC-peptide complex is an epitope of antigen capable of binding to the MHC, such as a tumor antigen, for example a universal tumor antigen, myeloma antigen or other antigen as described below. In some embodiments, an effective amount of the immunogen is then administered to a host for eliciting an immune response, wherein the immunogen retains a three-dimensional form thereof for a period of time sufficient to elicit an immune response against the three-dimensional presentation of the peptide in the binding groove of the MHC molecule. Serum collected from the host is then assayed to determine if desired antibodies that recognize a three-dimensional presentation of the peptide in the binding groove of the MHC molecule is being produced. In some embodiments, the produced antibodies can be assessed to confirm that the antibody can differentiate the MHC-peptide complex from the MHC molecule alone, the peptide of interest alone, and a complex of MHC and irrelevant peptide. The desired antibodies can then be isolated.

In some embodiments, an antibody or antigen-binding portion thereof that specifically binds to an MHC-peptide complex can be produced by employing antibody library display methods, such as phage antibody libraries. In some embodiments, phage display libraries of mutant Fab, scFv or other antibody forms can be generated, for example, in which members of the library are mutated at one or more residues of a CDR or CDRs. See *e.g.* US published application No. US20020150914, US2014/0294841; and Cohen CJ. et al. (2003) J Mol. Recogn. 16:324-332.

The term "antibody" herein is used in the broadest sense and includes polyclonal and monoclonal antibodies, including intact antibodies and functional (antigen-binding) antibody fragments, including fragment antigen binding (Fab) fragments, F(ab')₂ fragments, Fab' fragments, Fv fragments, recombinant IgG (rIgG) fragments, variable heavy chain (V_{H}) regions capable of specifically binding the antigen, single chain antibody fragments, including single chain variable fragments (scFv), and single domain antibodies (e.g., sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, e.g., bispecific, antibodies, diabodies, triabodies, and tetrabodies, tandem di-scFv, tandem tri-scFv. Unless otherwise stated, the term "antibody" should be understood to encompass functional antibody fragments thereof. The term also encompasses intact or full-length antibodies, including antibodies of any class or sub-class, including IgG and sub-classes thereof, IgM, IgE, IgA, and IgD.

In some embodiments, the antigen-binding proteins, antibodies and antigen binding fragments thereof specifically recognize an antigen of a full-length antibody. In some embodiments, the heavy and light chains of an antibody can be full-length or can be an antigen-binding portion (a Fab, F(ab')2, Fv or a single chain Fv fragment (scFv)). In other embodiments, the antibody heavy chain constant region is chosen from, *e.g.,* IgG1, IgG2, IgG3, IgG4, IgM, IgA1, IgA2, IgD, and IgE, particularly chosen from, *e.g.,* IgG1, IgG2, IgG3, and IgG4, more particularly, IgG1 (*e.g.,* human IgG1). In another embodiment, the antibody light chain constant region is chosen from, *e.g.,* kappa or lambda, particularly kappa.

Among the provided antibodies are antibody fragments. An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; variable heavy chain (V_{H}) regions, single-chain antibody molecules such as scFvs and single-domain V_{H} single antibodies; and multispecific antibodies formed from antibody fragments. In particular embodiments, the antibodies are single-chain antibody fragments comprising a variable heavy chain region and/or a variable light chain region, such as scFvs.

The term "variable region" or "variable domain" refers to the domain of an antibody heavy or light chain that is involved in binding the antibody to antigen. The variable domains of the heavy chain and light chain (V_{H} and V_{L}, respectively) of a native antibody generally have similar structures, with each domain comprising four conserved framework regions (FRs) and three CDRs. (See, *e.g.,* Kindt et al. Kuby Immunology, 6th ed., W.H. Freeman and Co., page 91 (2007). A single V_{H} or V_{L} domain may be sufficient to confer antigen-binding specificity. Furthermore, antibodies that bind a particular antigen may be isolated using a V_{H} or V_{L} domain from an antibody that binds the antigen to screen a library of complementary V_{L} or V_{H} domains, respectively. See, *e.g.,* Portolano et al., J. Immunol. 150:880-887 (1993); Clarkson et al., Nature 352:624-628 (1991).

Single-domain antibodies are antibody fragments comprising all or a portion of the heavy chain variable domain or all or a portion of the light chain variable domain of an antibody. In certain embodiments, a single-domain antibody is a human single-domain antibody. In some embodiments, the CAR comprises an antibody heavy chain domain that specifically binds the antigen, such as a cancer marker or cell surface antigen of a cell or disease to be targeted, such as a tumor cell or a cancer cell, such as any of the target antigens described herein or known in the art.

Antibody fragments can be made by various techniques, including but not limited to proteolytic digestion of an intact antibody as well as production by recombinant host cells. In some embodiments, the antibodies are recombinantly-produced fragments, such as fragments comprising arrangements that do not occur naturally, such as those with two or more antibody regions or chains joined by synthetic linkers, e.g., peptide linkers, and/or that are may not be produced by enzyme digestion of a naturally-occurring intact antibody. In some embodiments, the antibody fragments are scFvs.

A "humanized" antibody is an antibody in which all or substantially all CDR amino acid residues are derived from non-human CDRs and all or substantially all FR amino acid residues are derived from human FRs. A humanized antibody optionally may include at least a portion of an antibody constant region derived from a human antibody. A "humanized form" of a non-human antibody, refers to a variant of the non-human antibody that has undergone humanization, typically to reduce immunogenicity to humans, while retaining the specificity and affinity of the parental non-human antibody. In some embodiments, some FR residues in a humanized antibody are substituted with corresponding residues from a non-human antibody *(e.g.,* the antibody from which the CDR residues are derived), *e.g.,* to restore or improve antibody specificity or affinity.

Thus, in some embodiments, the chimeric antigen receptor, including TCR-like CARs, includes an extracellular portion containing an antibody or antibody fragment. In some embodiments, the antibody or fragment includes an scFv. In some aspects, the chimeric antigen receptor includes an extracellular portion containing the antibody or fragment and an intracellular signaling region. In some embodiments, the intracellular signaling region comprises an intracellular signaling domain. In some embodiments, the intracellular signaling domain is or comprises a primary signaling domain, a signaling domain that is capable of inducing a primary activation signal in a T cell, a signaling domain of a T cell receptor (TCR) component, and/or a signaling domain comprising an immunoreceptor tyrosine-based activation motif (ITAM).

In some embodiments, the recombinant receptor such as the CAR, such as the antibody portion thereof, further includes a spacer, which may be or include at least a portion of an immunoglobulin constant region or variant or modified version thereof, such as a hinge region, e.g., an IgG4 hinge region, and/or a C_{H}1/C_{L} and/or Fc region. In some embodiments, the recombinant receptor further comprises a spacer and/or a hinge region. In some embodiments, the constant region or portion is of a human IgG, such as IgG4 or IgG1. In some aspects, the portion of the constant region serves as a spacer region between the antigen-recognition component, e.g., scFv, and transmembrane domain. The spacer can be of a length that provides for increased responsiveness of the cell following antigen binding, as compared to in the absence of the spacer. In some examples, the spacer is at or about 12 amino acids in length or is no more than 12 amino acids in length. Exemplary spacers include those having at least about 10 to 229 amino acids, about 10 to 200 amino acids, about 10 to 175 amino acids, about 10 to 150 amino acids, about 10 to 125 amino acids, about 10 to 100 amino acids, about 10 to 75 amino acids, about 10 to 50 amino acids, about 10 to 40 amino acids, about 10 to 30 amino acids, about 10 to 20 amino acids, or about 10 to 15 amino acids, and including any integer between the endpoints of any of the listed ranges. In some embodiments, a spacer region has about 12 amino acids or less, about 119 amino acids or less, or about 229 amino acids or less. Exemplary spacers include IgG4 hinge alone, IgG4 hinge linked to CH2 and CH3 domains, or IgG4 hinge linked to the CH3 domain. Exemplary spacers include, but are not limited to, those described in Hudecek et al. (2013) Clin. Cancer Res., 19:3153 or international patent application publication number WO2014031687. In some embodiments, the spacer has the sequence set forth in SEQ ID NO: 1, and is encoded by the sequence set forth in SEQ ID NO: 2. In some embodiments, the spacer has the sequence set forth in SEQ ID NO: 3. In some embodiments, the spacer has the sequence set forth in SEQ ID NO: 4.

In some embodiments, the constant region or portion is of IgD. In some embodiments, the spacer has the sequence set forth in SEQ ID NO: 5. In some embodiments, the spacer has a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 1, 3, 4 and 5. In some embodiments, the spacer has the sequence set forth in SEQ ID NOS: 23-31. In some embodiments, the spacer has a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 27-31, 58, 59.

The antigen recognition domain generally is linked to one or more intracellular signaling components, such as signaling components that mimic activation through an antigen receptor complex, such as a TCR complex, in the case of a CAR, and/or signal via another cell surface receptor. Thus, in some embodiments, the antigen binding component (e.g., antibody) is linked to one or more transmembrane and intracellular signaling regions. In some embodiments, the transmembrane domain is fused to the extracellular domain. In one embodiment, a transmembrane domain that naturally is associated with one of the domains in the receptor, e.g., CAR, is used. In some instances, the transmembrane domain is selected or modified by amino acid substitution to avoid binding of such domains to the transmembrane domains of the same or different surface membrane proteins to minimize interactions with other members of the receptor complex.

The transmembrane domain in some embodiments is derived either from a natural or from a synthetic source. Where the source is natural, the domain in some aspects is derived from any membrane-bound or transmembrane protein. Transmembrane regions include those derived from (i.e. comprise at least the transmembrane region(s) of) the alpha, beta or zeta chain of the T-cell receptor, CD28, CD3 epsilon, CD45, CD4, CD5, CD8, CD9, CD 16, CD22, CD33, CD37, CD64, CD80, CD86, CD 134, CD137, CD 154. Alternatively the transmembrane domain in some embodiments is synthetic. In some aspects, the synthetic transmembrane domain comprises predominantly hydrophobic residues such as leucine and valine. In some aspects, a triplet of phenylalanine, tryptophan and valine will be found at each end of a synthetic transmembrane domain. In some embodiments, the linkage is by linkers, spacers, and/or transmembrane domain(s).

Among the intracellular signaling region are those that mimic or approximate a signal through a natural antigen receptor, a signal through such a receptor in combination with a costimulatory receptor, and/or a signal through a costimulatory receptor alone. In some embodiments, a short oligo- or polypeptide linker, for example, a linker of between 2 and 10 amino acids in length, such as one containing glycines and serines, e.g., glycine-serine doublet, is present and forms a linkage between the transmembrane domain and the cytoplasmic signaling domain of the CAR.

The receptor, e.g., the CAR, generally includes at least one intracellular signaling component or components. In some embodiments, the receptor includes an intracellular component of a TCR complex, such as a TCR CD3 chain that mediates T-cell activation and cytotoxicity, e.g., CD3 zeta chain. Thus, in some aspects, the ROR1-binding antibody is linked to one or more cell signaling modules. In some embodiments, cell signaling modules include CD3 transmembrane domain, CD3 intracellular signaling domains, and/or other CD transmembrane domains. In some embodiments, the receptor, e.g., CAR, further includes a portion of one or more additional molecules such as Fc receptor γ, CD8, CD4, CD25, or CD16. For example, in some aspects, the CAR includes a chimeric molecule between CD3-zeta (CD3-ζ) or Fc receptor γ and CD8, CD4, CD25 or CD16.

In some embodiments, upon ligation of the CAR, the cytoplasmic domain or intracellular signaling region of the CAR activates at least one of the normal effector functions or responses of the immune cell, e.g., T cell engineered to express the CAR. For example, in some contexts, the CAR induces a function of a T cell such as cytolytic activity or T-helper activity, such as secretion of cytokines or other factors. In some embodiments, a truncated portion of an intracellular signaling region of an antigen receptor component or costimulatory molecule is used in place of an intact immunostimulatory chain, for example, if it transduces the effector function signal. In some embodiments, the intracellular signaling regions, e.g., comprising intracellular domain or domains, include the cytoplasmic sequences of the T cell receptor (TCR), and in some aspects also those of co-receptors that in the natural context act in concert with such receptor to initiate signal transduction following antigen receptor engagement, and/or any derivative or variant of such molecules, and/or any synthetic sequence that has the same functional capability.

In the context of a natural TCR, full activation generally requires not only signaling through the TCR, but also a costimulatory signal. Thus, in some embodiments, to promote full activation, a component for generating secondary or co-stimulatory signal is also included in the CAR. In other embodiments, the CAR does not include a component for generating a costimulatory signal. In some aspects, an additional CAR is expressed in the same cell and provides the component for generating the secondary or costimulatory signal.

T cell activation is in some aspects described as being mediated by two classes of cytoplasmic signaling sequences: those that initiate antigen-dependent primary activation through the TCR (primary cytoplasmic signaling sequences), and those that act in an antigen-independent manner to provide a secondary or co-stimulatory signal (secondary cytoplasmic signaling sequences). In some aspects, the CAR includes one or both of such signaling components.

In some aspects, the CAR includes a primary cytoplasmic signaling sequence that regulates primary activation of the TCR complex. Primary cytoplasmic signaling sequences that act in a stimulatory manner may contain signaling motifs which are known as immunoreceptor tyrosine-based activation motifs or ITAMs. Examples of ITAM containing primary cytoplasmic signaling sequences include those derived from TCR or CD3 zeta, FcR gamma or FcR beta. In some embodiments, cytoplasmic signaling molecule(s) in the CAR contain(s) a cytoplasmic signaling domain, portion thereof, or sequence derived from CD3 zeta.

In some embodiments, the CAR includes a signaling region and/or transmembrane portion of a costimulatory receptor, such as CD28, 4-1BB, OX40, DAP10, and ICOS. In some aspects, the same CAR includes both the signaling region and costimulatory components.

In some embodiments, the signaling region is included within one CAR, whereas the costimulatory component is provided by another CAR recognizing another antigen. In some embodiments, the CARs include activating or stimulatory CARs, and costimulatory CARs, both expressed on the same cell *(see* WO2014/055668).

In certain embodiments, the intracellular signaling region comprises a CD28 transmembrane and signaling domain linked to a CD3 (e.g., CD3-zeta) intracellular domain. In some embodiments, the intracellular signaling region comprises a chimeric CD28 and CD137 (4-1BB, TNFRSF9) co-stimulatory domains, linked to a CD3 zeta intracellular domain.

In some embodiments, the CAR encompasses one or more, e.g., two or more, costimulatory domains and an activation domain, e.g., primary activation domain, in the cytoplasmic portion. Exemplary CARs include intracellular components of CD3-zeta, CD28, and 4-1BB.

In some cases, CARs are referred to as first, second, and/or third generation CARs. In some aspects, a first generation CAR is one that solely provides a CD3-chain induced signal upon antigen binding; in some aspects, a second-generation CARs is one that provides such a signal and costimulatory signal, such as one including an intracellular signaling domain from a costimulatory receptor such as CD28 or CD137; in some aspects, a third generation CAR in some aspects is one that includes multiple costimulatory domains of different costimulatory receptors.

In some embodiments, the chimeric antigen receptor includes an extracellular portion containing the antibody or fragment described herein. In some aspects, the chimeric antigen receptor includes an extracellular portion containing the antibody or fragment described herein and an intracellular signaling domain. In some embodiments, the antibody or fragment includes an scFv or a single-domain V_{H} antibody and the intracellular domain contains an ITAM. In some aspects, the intracellular signaling domain includes a signaling domain of a zeta chain of a CD3-zeta (CD3ζ) chain. In some embodiments, the chimeric antigen receptor includes a transmembrane domain disposed between the extracellular domain and the intracellular signaling region.

In some aspects, the transmembrane domain contains a transmembrane portion of CD28. The extracellular domain and transmembrane can be linked directly or indirectly. In some embodiments, the extracellular domain and transmembrane are linked by a spacer, such as any described herein. In some embodiments, the chimeric antigen receptor contains an intracellular domain of a T cell costimulatory molecule, such as between the transmembrane domain and intracellular signaling domain. In some aspects, the T cell costimulatory molecule is CD28 or 4-1BB.

In some embodiments, the CAR contains an antibody, e.g., an antibody fragment, a transmembrane domain that is or contains a transmembrane portion of CD28 or a functional variant thereof, and an intracellular signaling domain containing a signaling portion of CD28 or functional variant thereof and a signaling portion of CD3 zeta or functional variant thereof. In some embodiments, the CAR contains an antibody, e.g., antibody fragment, a transmembrane domain that is or contains a transmembrane portion of CD28 or a functional variant thereof, and an intracellular signaling domain containing a signaling portion of a 4-1BB or functional variant thereof and a signaling portion of CD3 zeta or functional variant thereof. In some such embodiments, the receptor further includes a spacer containing a portion of an Ig molecule, such as a human Ig molecule, such as an Ig hinge, e.g. an IgG4 hinge, such as a hinge-only spacer.

In some embodiments, the transmembrane domain of the receptor, e.g., the CAR is a transmembrane domain of human CD28 or variant thereof, e.g., a 27-amino acid transmembrane domain of a human CD28 (Accession No.: P10747.1), or is a transmembrane domain that comprises the sequence of amino acids set forth in SEQ ID NO: 8 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO:8; in some embodiments, the transmembrane-domain containing portion of the recombinant receptor comprises the sequence of amino acids set forth in SEQ ID NO: 9 or a sequence of amino acids having at least at or about 85%, 86%, 87%, 88%, 89%, 90%, 91%, 92%, 93%, 94%, 95%, 96%, 97%, 98%, 99% or more sequence identity thereto.

In some embodiments, the chimeric antigen receptor contains an intracellular domain of a T cell costimulatory molecule. In some aspects, the T cell costimulatory molecule is CD28 or 4-1BB.

In some embodiments, the intracellular signaling region comprises an intracellular costimulatory signaling domain of human CD28 or functional variant or portion thereof, such as a 41 amino acid domain thereof and/or such a domain with an LL to GG substitution at positions 186-187 of a native CD28 protein. In some embodiments, the intracellular signaling domain can comprise the sequence of amino acids set forth in SEQ ID NO: 10 or 11 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 10 or 11. In some embodiments, the intracellular region comprises an intracellular costimulatory signaling domain of 4-1BB or functional variant or portion thereof, such as a 42-amino acid cytoplasmic domain of a human 4-1BB (Accession No. Q07011.1) or functional variant or portion thereof, such as the sequence of amino acids set forth in SEQ ID NO: 12 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 12.

In some embodiments, the intracellular signaling region comprises a human CD3 chain, optionally a CD3 zeta stimulatory signaling domain or functional variant thereof, such as an 112 AA cytoplasmic domain of isoform 3 of human CD3ζ (Accession No.: P20963.2) or a CD3 zeta signaling domain as described in U.S. Patent No.: 7,446,190 or U.S. Patent No. 8,911,993. In some embodiments, the intracellular signaling region comprises the sequence of amino acids set forth in SEQ ID NO: 13, 14 or 15 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 13, 14 or 15.

In some aspects, the spacer contains only a hinge region of an IgG, such as only a hinge of IgG4 or IgG1, such as the hinge only spacer set forth in SEQ ID NO:1. In other embodiments, the spacer is an Ig hinge, e.g., and IgG4 hinge, linked to a C_{H}2 and/or C_{H}3 domains. In some embodiments, the spacer is an Ig hinge, e.g., an IgG4 hinge, linked to C_{H}2 and C_{H}3 domains, such as set forth in SEQ ID NO:3. In some embodiments, the spacer is an Ig hinge, e.g., an IgG4 hinge, linked to a C_{H}3 domain only, such as set forth in SEQ ID NO:4. In some embodiments, the spacer is or comprises a glycine-serine rich sequence or other flexible linker such as known flexible linkers.

### 2. Chimeric Auto-Antibody Receptors (CAARs)

In some embodiments, among the recombinant receptor expressed by the engineered cells used in connection with the provided methods, uses, articles of manufacture and compositions is a chimeric autoantibody receptor (CAAR). In some embodiments, the CAAR is specific for an autoantibody. In some embodiments, a cell expressing the CAAR, such as a T cell engineered to express a CAAR, can be used to specifically bind to and kill autoantibody-expressing cells, but not normal antibody expressing cells. In some embodiments, CAAR-expressing cells can be used to treat an autoimmune disease associated with expression of self-antigens, such as autoimmune diseases. In some embodiments, CAAR-expressing cells can target B cells that ultimately produce the autoantibodies and display the autoantibodies on their cell surfaces, mark these B cells as disease-specific targets for therapeutic intervention. In some embodiments, CAAR-expressing cells can be used to efficiently targeting and killing the pathogenic B cells in autoimmune diseases by targeting the disease-causing B cells using an antigen-specific chimeric autoantibody receptor. In some embodiments, the recombinant receptor is a CAAR, such as any described in U.S. Patent Application Pub. No. US 2017/0051035.

In some embodiments, the CAAR comprises an autoantibody binding domain, a transmembrane domain, and an intracellular signaling region. In some embodiments, the intracellular signaling region comprises an intracellular signaling domain. In some embodiments, the intracellular signaling domain is or comprises a primary signaling domain, a signaling domain that is capable of inducing a primary activation signal in a T cell, a signaling domain of a T cell receptor (TCR) component, and/or a signaling domain comprising an immunoreceptor tyrosine-based activation motif (ITAM). In some embodiments, the intracellular signaling region comprises a secondary or costimulatory signaling region (secondary intracellular signaling regions).

In some embodiments, the autoantibody binding domain comprises an autoantigen or a fragment thereof. The choice of autoantigen can depend upon the type of autoantibody being targeted. For example, the autoantigen may be chosen because it recognizes an autoantibody on a target cell, such as a B cell, associated with a particular disease state, e.g. an autoimmune disease, such as an autoantibody-mediated autoimmune disease. In some embodiments, the autoimmune disease includes pemphigus vulgaris (PV). Exemplary autoantigens include desmoglein 1 (Dsg1) and Dsg3.

### 3. Multi-targeting

In some embodiments, the cells used in connection with the provided methods, uses, articles of manufacture and compositions include cells employing multi-targeting strategies. In some embodiments, the cells express multi-chain chimeric antigen receptors (CAR) or express two or more genetically engineered receptors on the cell, each recognizing the same of a different antigen and typically each including a different intracellular signaling component. Such multi-targeting strategies are described, for example, in International Patent Application, Publication No.: WO 2014055668 A1 (describing combinations of activating and costimulatory CARs, e.g., targeting two different antigens present individually on off-target, e.g., normal cells, but present together only on cells of the disease or condition to be treated) and Fedorov et al., Sci. Transl. Medicine, 5(215) (2013) (describing cells expressing an activating and an inhibitory CAR, such as those in which the activating CAR binds to one antigen expressed on both normal or non-diseased cells and cells of the disease or condition to be treated, and the inhibitory CAR binds to another antigen expressed only on the normal cells or cells which it is not desired to treat).

For example, in some embodiments, the cells include a receptor expressing a first genetically engineered antigen receptor (e.g., CAR or TCR) which is capable of inducing an activating or stimulatory signal to the cell, generally upon specific binding to the antigen recognized by the first receptor, e.g., the first antigen. In some embodiments, the cell further includes a second genetically engineered antigen receptor (e.g., CAR or TCR), e.g., a chimeric costimulatory receptor, which is capable of inducing a costimulatory signal to the immune cell, generally upon specific binding to a second antigen recognized by the second receptor. In some embodiments, the first antigen and second antigen are the same. In some embodiments, the first antigen and second antigen are different.

In some embodiments, the first and/or second genetically engineered antigen receptor (e.g. CAR or TCR) is capable of inducing an activating signal to the cell. In some embodiments, the receptor includes an intracellular signaling component containing ITAM or ITAM-like motifs. In some embodiments, the activation induced by the first receptor involves a signal transduction or change in protein expression in the cell resulting in initiation of an immune response, such as ITAM phosphorylation and/or initiation of ITAM-mediated signal transduction cascade, formation of an immunological synapse and/or clustering of molecules near the bound receptor (e.g. CD4 or CD8, etc.), activation of one or more transcription factors, such as NF-κB and/or AP-1, and/or induction of gene expression of factors such as cytokines, proliferation, and/or survival.

In some embodiments, the first and/or second receptor includes intracellular signaling domains or regions of costimulatory receptors such as CD28, CD137 (4-1BB), OX40, and/or ICOS. In some embodiments, the first and second receptor include an intracellular signaling domain of a costimulatory receptor that are different. In one embodiment, the first receptor contains a CD28 costimulatory signaling region and the second receptor contain a 4-1BB co-stimulatory signaling region or vice versa.

In some embodiments, the first and/or second receptor includes both an intracellular signaling domain containing ITAM or ITAM-like motifs and an intracellular signaling domain of a costimulatory receptor.

In some embodiments, the first receptor contains an intracellular signaling domain containing ITAM or ITAM-like motifs and the second receptor contains an intracellular signaling domain of a costimulatory receptor. The costimulatory signal in combination with the activating signal induced in the same cell is one that results in an immune response, such as a robust and sustained immune response, such as increased gene expression, secretion of cytokines and other factors, and T cell mediated effector functions such as cell killing.

In some embodiments, neither ligation of the first receptor alone nor ligation of the second receptor alone induces a robust immune response. In some aspects, if only one receptor is ligated, the cell becomes tolerized or unresponsive to antigen, or inhibited, and/or is not induced to proliferate or secrete factors or carry out effector functions. In some such embodiments, however, when the plurality of receptors are ligated, such as upon encounter of a cell expressing the first and second antigens, a desired response is achieved, such as full immune activation or stimulation, e.g., as indicated by secretion of one or more cytokine, proliferation, persistence, and/or carrying out an immune effector function such as cytotoxic killing of a target cell.

In some embodiments, the two receptors induce, respectively, an activating and an inhibitory signal to the cell, such that binding by one of the receptor to its antigen activates the cell or induces a response, but binding by the second inhibitory receptor to its antigen induces a signal that suppresses or dampens that response. Examples are combinations of activating CARs and inhibitory CARs or iCARs. Such a strategy may be used, for example, in which the activating CAR binds an antigen expressed in a disease or condition but which is also expressed on normal cells, and the inhibitory receptor binds to a separate antigen which is expressed on the normal cells but not cells of the disease or condition.

In some embodiments, the multi-targeting strategy is employed in a case where an antigen associated with a particular disease or condition is expressed on a non-diseased cell and/or is expressed on the engineered cell itself, either transiently (e.g., upon stimulation in association with genetic engineering) or permanently. In such cases, by requiring ligation of two separate and individually specific antigen receptors, specificity, selectivity, and/or efficacy may be improved.

In some embodiments, the plurality of antigens, e.g., the first and second antigens, are expressed on the cell, tissue, or disease or condition being targeted, such as on the cancer cell. In some aspects, the cell, tissue, disease or condition is multiple myeloma or a multiple myeloma cell. In some embodiments, one or more of the plurality of antigens generally also is expressed on a cell which it is not desired to target with the cell therapy, such as a normal or non-diseased cell or tissue, and/or the engineered cells themselves. In such embodiments, by requiring ligation of multiple receptors to achieve a response of the cell, specificity and/or efficacy is achieved.

### 4. T cell Receptor

In some embodiments, engineered cells, such as T cells, are provided that express a T cell receptor (TCR) or antigen-binding portion thereof that recognizes an peptide epitope or T cell epitope of a target polypeptide, such as an antigen of a tumor, viral or autoimmune protein.

In some embodiments, a "T cell receptor" or "TCR" is a molecule that contains a variable α and β chains (also known as TCRα and TCRβ, respectively) or a variable γ and δ chains (also known as TCRα and TCRβ, respectively), or antigen-binding portions thereof, and which is capable of specifically binding to a peptide bound to an MHC molecule. In some embodiments, the TCR is in the αβ form. Typically, TCRs that exist in αβ and γδ forms are generally structurally similar, but T cells expressing them may have distinct anatomical locations or functions. A TCR can be found on the surface of a cell or in soluble form. Generally, a TCR is found on the surface of T cells (or T lymphocytes) where it is generally responsible for recognizing antigens bound to major histocompatibility complex (MHC) molecules.

Unless otherwise stated, the term "TCR" should be understood to encompass full TCRs as well as antigen-binding portions or antigen-binding fragments thereof. In some embodiments, the TCR is an intact or full-length TCR, including TCRs in the αβ form or γδ form. In some embodiments, the TCR is an antigen-binding portion that is less than a full-length TCR but that binds to a specific peptide bound in an MHC molecule, such as binds to an MHC-peptide complex. In some cases, an antigen-binding portion or fragment of a TCR can contain only a portion of the structural domains of a full-length or intact TCR, but yet is able to bind the peptide epitope, such as MHC-peptide complex, to which the full TCR binds. In some cases, an antigen-binding portion contains the variable domains of a TCR, such as variable α chain and variable β chain of a TCR, sufficient to form a binding site for binding to a specific MHC-peptide complex. Generally, the variable chains of a TCR contain complementarity determining regions involved in recognition of the peptide, MHC and/or MHC-peptide complex.

In some embodiments, the variable domains of the TCR contain hypervariable loops, or complementarity determining regions (CDRs), which generally are the primary contributors to antigen recognition and binding capabilities and specificity. In some embodiments, a CDR of a TCR or combination thereof forms all or substantially all of the antigen-binding site of a given TCR molecule. The various CDRs within a variable region of a TCR chain generally are separated by framework regions (FRs), which generally display less variability among TCR molecules as compared to the CDRs (see, e.g., Jores et al., Proc. Nat'l Acad. Sci. U.S.A. 87:9138, 1990; Chothia et al., EMBO J. 7:3745, 1988; see also Lefranc et al., Dev. Comp. Immunol. 27:55, 2003). In some embodiments, CDR3 is the main CDR responsible for antigen binding or specificity, or is the most important among the three CDRs on a given TCR variable region for antigen recognition, and/or for interaction with the processed peptide portion of the peptide-MHC complex. In some contexts, the CDR1 of the alpha chain can interact with the N-terminal part of certain antigenic peptides. In some contexts, CDR1 of the beta chain can interact with the C-terminal part of the peptide. In some contexts, CDR2 contributes most strongly to or is the primary CDR responsible for the interaction with or recognition of the MHC portion of the MHC-peptide complex. In some embodiments, the variable region of the β-chain can contain a further hypervariable region (CDR4 or HVR4), which generally is involved in superantigen binding and not antigen recognition (Kotb (1995) Clinical Microbiology Reviews, 8:411-426).

In some embodiments, a TCR also can contain a constant domain, a transmembrane domain and/or a short cytoplasmic tail (see, e.g., Janeway et al., Immunobiology: The Immune System in Health and Disease, 3rd Ed., Current Biology Publications, p. 4:33, 1997). In some aspects, each chain of the TCR can possess one N-terminal immunoglobulin variable domain, one immunoglobulin constant domain, a transmembrane region, and a short cytoplasmic tail at the C-terminal end. In some embodiments, a TCR is associated with invariant proteins of the CD3 complex involved in mediating signal transduction.

In some embodiments, a TCR chain contains one or more constant domain. For example, the extracellular portion of a given TCR chain (e.g., α-chain or β-chain) can contain two immunoglobulin-like domains, such as a variable domain (e.g., Vα or Vβ; typically amino acids 1 to 116 based on Kabat numbering Kabat et al., "Sequences of Proteins of Immunological Interest, US Dept. Health and Human Services, Public Health Service National Institutes of Health, 1991, 5th ed.) and a constant domain (e.g., α-chain constant domain or Cα, typically positions 117 to 259 of the chain based on Kabat numbering or β chain constant domain or C_{β}, typically positions 117 to 295 of the chain based on Kabat) adjacent to the cell membrane. For example, in some cases, the extracellular portion of the TCR formed by the two chains contains two membrane-proximal constant domains, and two membrane-distal variable domains, which variable domains each contain CDRs. The constant domain of the TCR may contain short connecting sequences in which a cysteine residue forms a disulfide bond, thereby linking the two chains of the TCR. In some embodiments, a TCR may have an additional cysteine residue in each of the α and β chains, such that the TCR contains two disulfide bonds in the constant domains.

In some embodiments, the TCR chains contain a transmembrane domain. In some embodiments, the transmembrane domain is positively charged. In some cases, the TCR chain contains a cytoplasmic tail. In some cases, the structure allows the TCR to associate with other molecules like CD3 and subunits thereof. For example, a TCR containing constant domains with a transmembrane region may anchor the protein in the cell membrane and associate with invariant subunits of the CD3 signaling apparatus or complex. The intracellular tails of CD3 signaling subunits (e.g. CD3y, CD3δ, CD3ε and CD3ζ chains) contain one or more immunoreceptor tyrosine-based activation motif or ITAM that are involved in the signaling capacity of the TCR complex.

In some embodiments, the TCR may be a heterodimer of two chains α and β (or optionally γ and δ) or it may be a single chain TCR construct. In some embodiments, the TCR is a heterodimer containing two separate chains (α and β chains or γ and δ chains) that are linked, such as by a disulfide bond or disulfide bonds.

In some embodiments, the TCR can be generated from a known TCR sequence(s), such as sequences of Vα,β chains, for which a substantially full-length coding sequence is readily available. Methods for obtaining full-length TCR sequences, including V chain sequences, from cell sources can be used. In some embodiments, nucleic acids encoding the TCR can be obtained from a variety of sources, such as by polymerase chain reaction (PCR) amplification of TCR-encoding nucleic acids within or isolated from a given cell or cells, or synthesis of publicly available TCR DNA sequences.

In some embodiments, the TCR is obtained from a biological source, such as from cells such as from a T cell (e.g. cytotoxic T cell), T-cell hybridomas or other publicly available source. In some embodiments, the T-cells can be obtained from *in vivo* isolated cells. In some embodiments, the TCR is a thymically selected TCR. In some embodiments, the TCR is a neoepitope-restricted TCR. In some embodiments, the T- cells can be a cultured T-cell hybridoma or clone. In some embodiments, the TCR or antigen-binding portion thereof or antigen-binding fragment thereof can be synthetically generated from knowledge of the sequence of the TCR.

In some embodiments, the TCR is generated from a TCR identified or selected from screening a library of candidate TCRs against a target polypeptide antigen, or target T cell epitope thereof. TCR libraries can be generated by amplification of the repertoire of Vα and Vβ from T cells isolated from a subject, including cells present in PBMCs, spleen or other lymphoid organ. In some cases, T cells can be amplified from tumor-infiltrating lymphocytes (TILs). In some embodiments, TCR libraries can be generated from CD4+ or CD8+ cells. In some embodiments, the TCRs can be amplified from a T cell source of a normal of healthy subject, i.e. normal TCR libraries. In some embodiments, the TCRs can be amplified from a T cell source of a diseased subject, i.e. diseased TCR libraries. In some embodiments, degenerate primers are used to amplify the gene repertoire of Vα and Vβ, such as by RT-PCR in samples, such as T cells, obtained from humans. In some embodiments, scTv libraries can be assembled from naive Vα and Vβ libraries in which the amplified products are cloned or assembled to be separated by a linker. Depending on the source of the subject and cells, the libraries can be HLA allele-specific. Alternatively, in some embodiments, TCR libraries can be generated by mutagenesis or diversification of a parent or scaffold TCR molecule. In some aspects, the TCRs are subjected to directed evolution, such as by mutagenesis, e.g., of the α or β chain. In some aspects, particular residues within CDRs of the TCR are altered. In some embodiments, selected TCRs can be modified by affinity maturation. In some embodiments, antigen-specific T cells may be selected, such as by screening to assess CTL activity against the peptide. In some aspects, TCRs, e.g. present on the antigen-specific T cells, may be selected, such as by binding activity, e.g., particular affinity or avidity for the antigen.

In some embodiments, the TCR or antigen-binding portion thereof is one that has been modified or engineered. In some embodiments, directed evolution methods are used to generate TCRs with altered properties, such as with higher affinity for a specific MHC-peptide complex. In some embodiments, directed evolution is achieved by display methods including, but not limited to, yeast display (Holler et al. (2003) Nat Immunol, 4, 55-62; Holler et al. (2000) Proc Natl Acad Sci USA, 97, 5387-92), phage display (Li et al. (2005) Nat Biotechnol, 23, 349-54), or T cell display (Chervin et al. (2008) J Immunol Methods, 339, 175-84). In some embodiments, display approaches involve engineering, or modifying, a known, parent or reference TCR. For example, in some cases, a wild-type TCR can be used as a template for producing mutagenized TCRs in which in one or more residues of the CDRs are mutated, and mutants with an desired altered property, such as higher affinity for a desired target antigen, are selected.

In some embodiments, peptides of a target polypeptide for use in producing or generating a TCR of interest are known or can be readily identified. In some embodiments, peptides suitable for use in generating TCRs or antigen-binding portions can be determined based on the presence of an HLA-restricted motif in a target polypeptide of interest, such as a target polypeptide described below. In some embodiments, peptides are identified using available computer prediction models. In some embodiments, for predicting MHC class I binding sites, such models include, but are not limited to, ProPred1 (Singh and Raghava (2001) Bioinformatics 17(12): 1236-1237, and SYFPEITHI (see Schuler et al. (2007) Immunoinformatics Methods in Molecular Biology, 409(1): 75-93 2007). In some embodiments, the MHC-restricted epitope is HLA-A0201, which is expressed in approximately 39-46% of all Caucasians and therefore, represents a suitable choice of MHC antigen for use preparing a TCR or other MHC-peptide binding molecule.

HLA-A0201-binding motifs and the cleavage sites for proteasomes and immune-proteasomes using computer prediction models can be used. For predicting MHC class I binding sites, such models include, but are not limited to, ProPred1 (described in more detail in Singh and Raghava, ProPred: prediction of HLA-DR binding sites. BIOINFORMATICS 17(12):1236-1237 2001), and SYFPEITHI (see Schuler et al. SYFPEITHI, Database for Searching and T-Cell Epitope Prediction. in Immunoinformatics Methods in Molecular Biology, vol 409(1): 75-93 2007)

In some embodiments, the TCR or antigen binding portion thereof may be a recombinantly produced natural protein or mutated form thereof in which one or more property, such as binding characteristic, has been altered. In some embodiments, a TCR may be derived from one of various animal species, such as human, mouse, rat, or other mammal. A TCR may be cell-bound or in soluble form. In some embodiments, for purposes of the provided methods, the TCR is in cell-bound form expressed on the surface of a cell.

In some embodiments, the TCR is a full-length TCR. In some embodiments, the TCR is an antigen-binding portion. In some embodiments, the TCR is a dimeric TCR (dTCR). In some embodiments, the TCR is a single-chain TCR (sc-TCR). In some embodiments, a dTCR or scTCR have the structures as described in WO 03/020763, WO 04/033685, WO 2011/044186.

In some embodiments, the TCR contains a sequence corresponding to the transmembrane sequence. In some embodiments, the TCR does contain a sequence corresponding to cytoplasmic sequences. In some embodiments, the TCR is capable of forming a TCR complex with CD3. In some embodiments, any of the TCRs, including a dTCR or scTCR, can be linked to signaling domains that yield an active TCR on the surface of a T cell. In some embodiments, the TCR is expressed on the surface of cells.

In some embodiments a dTCR contains a first polypeptide wherein a sequence corresponding to a TCR α chain variable region sequence is fused to the N terminus of a sequence corresponding to a TCR α chain constant region extracellular sequence, and a second polypeptide wherein a sequence corresponding to a TCR β chain variable region sequence is fused to the N terminus a sequence corresponding to a TCR β chain constant region extracellular sequence, the first and second polypeptides being linked by a disulfide bond. In some embodiments, the bond can correspond to the native inter-chain disulfide bond present in native dimeric αβ TCRs. In some embodiments, the interchain disulfide bonds are not present in a native TCR. For example, in some embodiments, one or more cysteines can be incorporated into the constant region extracellular sequences of dTCR polypeptide pair. In some cases, both a native and a non-native disulfide bond may be desirable. In some embodiments, the TCR contains a transmembrane sequence to anchor to the membrane.

In some embodiments, a dTCR contains a TCR α chain containing a variable α domain, a constant α domain and a first dimerization motif attached to the C-terminus of the constant α domain, and a TCR β chain comprising a variable β domain, a constant β domain and a first dimerization motif attached to the C-terminus of the constant β domain, wherein the first and second dimerization motifs easily interact to form a covalent bond between an amino acid in the first dimerization motif and an amino acid in the second dimerization motif linking the TCR α chain and TCR β chain together.

In some embodiments, the TCR is a scTCR. Typically, a scTCR can be generated using methods known to those of skill in the art, See e.g., Soo Hoo, W. F. et al. PNAS (USA) 89, 4759 (1992); Wülfing, C. and Plückthun, A., J. Mol. Biol. 242, 655 (1994); Kurucz, I. et al. PNAS (USA) 90 3830 (1993); International published PCT Nos. WO 96/13593, WO 96/18105, WO99/60120, WO99/18129, WO 03/020763, WO2011/044186; and Schlueter, C. J. et al. J. Mol. Biol. 256, 859 (1996). In some embodiments, a scTCR contains an introduced non-native disulfide interchain bond to facilitate the association of the TCR chains (see e.g. International published PCT No. WO 03/020763). In some embodiments, a scTCR is a non-disulfide linked truncated TCR in which heterologous leucine zippers fused to the C-termini thereof facilitate chain association (see e.g. International published PCT No. WO99/60120). In some embodiments, a scTCR contain a TCRα variable domain covalently linked to a TCRβ variable domain via a peptide linker (see e.g., International published PCT No. WO99/18129).

In some embodiments, a scTCR contains a first segment constituted by an amino acid sequence corresponding to a TCR α chain variable region, a second segment constituted by an amino acid sequence corresponding to a TCR β chain variable region sequence fused to the N terminus of an amino acid sequence corresponding to a TCR β chain constant domain extracellular sequence, and a linker sequence linking the C terminus of the first segment to the N terminus of the second segment.

In some embodiments, a scTCR contains a first segment constituted by an α chain variable region sequence fused to the N terminus of an α chain extracellular constant domain sequence, and a second segment constituted by a β chain variable region sequence fused to the N terminus of a sequence β chain extracellular constant and transmembrane sequence, and, optionally, a linker sequence linking the C terminus of the first segment to the N terminus of the second segment.

In some embodiments, a scTCR contains a first segment constituted by a TCR β chain variable region sequence fused to the N terminus of a β chain extracellular constant domain sequence, and a second segment constituted by an α chain variable region sequence fused to the N terminus of a sequence α chain extracellular constant and transmembrane sequence, and, optionally, a linker sequence linking the C terminus of the first segment to the N terminus of the second segment.

In some embodiments, the linker of a scTCRs that links the first and second TCR segments can be any linker capable of forming a single polypeptide strand, while retaining TCR binding specificity. In some embodiments, the linker sequence may, for example, have the formula -P-AA-P- wherein P is proline and AA represents an amino acid sequence wherein the amino acids are glycine and serine. In some embodiments, the first and second segments are paired so that the variable region sequences thereof are orientated for such binding. Hence, in some cases, the linker has a sufficient length to span the distance between the C terminus of the first segment and the N terminus of the second segment, or vice versa, but is not too long to block or reduces bonding of the scTCR to the target ligand. In some embodiments, the linker can contain from or from about 10 to 45 amino acids, such as 10 to 30 amino acids or 26 to 41 amino acids residues, for example 29, 30, 31 or 32 amino acids. In some embodiments, the linker has the formula -PGGG-(SGGGG)5-P- wherein P is proline, G is glycine and S is serine (SEQ ID NO: 23). In some embodiments, the linker has the sequence GSADDAKKDAAKKDGKS (SEQ ID NO: 24).

In some embodiments, the scTCR contains a covalent disulfide bond linking a residue of the immunoglobulin region of the constant domain of the α chain to a residue of the immunoglobulin region of the constant domain of the β chain. In some embodiments, the interchain disulfide bond in a native TCR is not present. For example, in some embodiments, one or more cysteines can be incorporated into the constant region extracellular sequences of the first and second segments of the scTCR polypeptide. In some cases, both a native and a non-native disulfide bond may be desirable.

In some embodiments of a dTCR or scTCR containing introduced interchain disulfide bonds, the native disulfide bonds are not present. In some embodiments, the one or more of the native cysteines forming a native interchain disulfide bonds are substituted to another residue, such as to a serine or alanine. In some embodiments, an introduced disulfide bond can be formed by mutating non-cysteine residues on the first and second segments to cysteine. Exemplary non-native disulfide bonds of a TCR are described in published International PCT No. WO2006/000830.

In some embodiments, the TCR or antigen-binding fragment thereof exhibits an affinity with an equilibrium binding constant for a target antigen of between or between about 10-5 and 10-12 M and all individual values and ranges therein. In some embodiments, the target antigen is an MHC-peptide complex or ligand.

In some embodiments, nucleic acid or nucleic acids encoding a TCR, such as α and β chains, can be amplified by PCR, cloning or other suitable means and cloned into a suitable expression vector or vectors. The expression vector can be any suitable recombinant expression vector, and can be used to transform or transfect any suitable host. Suitable vectors include those designed for propagation and expansion or for expression or both, such as plasmids and viruses.

In some embodiments, the vector can a vector of the pUC series (Fermentas Life Sciences), the pBluescript series (Stratagene, LaJolla, Calif.), the pET series (Novagen, Madison, Wis.), the pGEX series (Pharmacia Biotech, Uppsala, Sweden), or the pEX series (Clontech, Palo Alto, Calif.). In some cases, bacteriophage vectors, such as λG10, λGT11, λZapII (Stratagene), λEMBL4, and λNM1149, also can be used. In some embodiments, plant expression vectors can be used and include pBI01, pBI101.2, pBI101.3, pBI121 and pBIN19 (Clontech). In some embodiments, animal expression vectors include pEUK-Cl, pMAM and pMAMneo (Clontech). In some embodiments, a viral vector is used, such as a retroviral vector.

In some embodiments, the recombinant expression vectors can be prepared using standard recombinant DNA techniques. In some embodiments, vectors can contain regulatory sequences, such as transcription and translation initiation and termination codons, which are specific to the type of host (e.g., bacterium, fungus, plant, or animal) into which the vector is to be introduced, as appropriate and taking into consideration whether the vector is DNA- or RNA-based. In some embodiments, the vector can contain a nonnative promoter operably linked to the nucleotide sequence encoding the TCR or antigen-binding portion (or other MHC-peptide binding molecule). In some embodiments, the promoter can be a non-viral promoter or a viral promoter, such as a cytomegalovirus (CMV) promoter, an SV40 promoter, an RSV promoter, and a promoter found in the long-terminal repeat of the murine stem cell virus. Other known promoters also are contemplated.

In some embodiments, to generate a vector encoding a TCR, the α and β chains are PCR amplified from total cDNA isolated from a T cell clone expressing the TCR of interest and cloned into an expression vector. In some embodiments, the α and β chains are cloned into the same vector. In some embodiments, the α and β chains are cloned into different vectors. In some embodiments, the generated α and β chains are incorporated into a retroviral, e.g. lentiviral, vector.

### B. Nucleic Acids and Vectors

Also provided are one or more polynucleotides (e.g., nucleic acid molecules) encoding recombinant receptors, vectors for genetically engineering cells to express the receptors and methods for producing the engineered cells. In some aspects, the recombinant receptor is or contains a chimeric antigen receptor (CAR). In some aspects, the recombinant receptor is or contains a T cell receptor (TCR), e.g., a transgenic TCR.

In some cases, the nucleic acid sequence encoding the recombinant receptor, e.g., chimeric antigen receptor (CAR) contains a signal sequence that encodes a signal peptide. Nonlimiting exemplary examples of signal peptides include, for example, the GMCSFR alpha chain signal peptide set forth in SEQ ID NO: 26 and encoded by the nucleotide sequence set forth in SEQ ID NO: 25, or the CD8 alpha signal peptide set forth in SEQ ID NO: 18.

In certain cases where nucleic acid molecules encode two or more different polypeptide chains, each of the polypeptide chains can be encoded by a separate nucleic acid molecule. For example, two separate nucleic acids are provided, and each can be individually transferred or introduced into the cell for expression in the cell.

In some embodiments, such as those where the polynucleotide contains a first and second nucleic acid sequence, the coding sequences encoding each of the different polypeptide chains can be operatively linked to a promoter, which can be the same or different. In some embodiments, the nucleic acid molecule can contain a promoter that drives the expression of two or more different polypeptide chains. In some embodiments, such nucleic acid molecules can be multicistronic (bicistronic or tricistronic, see *e.g.,* U.S. Patent No. 6,060,273). In some embodiments, transcription units can be engineered as a bicistronic unit containing an IRES (internal ribosome entry site), which allows coexpression of gene products by a message from a single promoter. Alternatively, in some cases, a single promoter may direct expression of an RNA that contains, in a single open reading frame (ORF), two or three genes separated from one another by sequences encoding a self-cleavage peptide *(e.g.,* 2A sequences) or a protease recognition site *(e.g.,* furin). The ORF thus encodes a single polypeptide, which, either during (in the case of 2A) or after translation, is processed into the individual proteins. In some cases, the peptide, such as a T2A, can cause the ribosome to skip (ribosome skipping) synthesis of a peptide bond at the C-terminus of a 2A element, leading to separation between the end of the 2A sequence and the next peptide downstream (*see,* for example, de Felipe. Genetic Vaccines and Ther. 2:13 (2004) and de Felipe et al. Traffic 5:616-626 (2004)). Various 2A elements are known. Examples of 2A sequences that can be used in the methods and system disclosed herein, without limitation, 2A sequences from the foot-and-mouth disease virus (F2A, *e.g.,* SEQ ID NO: 22), equine rhinitis A virus (E2A, *e.g.,* SEQ ID NO: 21), Thosea asigna virus (T2A, *e.g.,* SEQ ID NO: 6 or 17), and porcine teschovirus-1 (P2A, *e.g.,* SEQ ID NO: 19 or 20) as described in U.S. Patent Publication No. 20070116690.

In some embodiments, extrinsic marker genes may in some cases be utilized in connection with engineered cell therapies to permit detection or selection of cells and, in some cases, also to promote cell suicide. Exemplary surrogate markers can include truncated forms of cell surface polypeptides, such as truncated forms that are non-functional and to not transduce or are not capable of transducing a signal or a signal ordinarily transduced by the full-length form of the cell surface polypeptide, and/or do not or are not capable of internalizing. Exemplary truncated cell surface polypeptides including truncated forms of growth factors or other receptors such as a truncated human epidermal growth factor receptor 2 (tHER2), a truncated epidermal growth factor receptor (tEGFR, exemplary tEGFR sequence set forth in SEQ ID NO: 7 or 16) or a prostate-specific membrane antigen (PSMA) or modified form thereof. tEGFR may contain an epitope recognized by the antibody cetuximab (Erbitux^{®}) or other therapeutic anti-EGFR antibody or binding molecule, which can be used to identify or select cells that have been engineered with the tEGFR construct and an encoded exogenous protein, and/or to eliminate or separate cells expressing the encoded exogenous protein. See U.S. Patent No. 8,802,374 and Liu et al., Nature Biotech. 2016 April; 34(4): 430-434). In some aspects, the marker, e.g. surrogate marker, includes all or part (e.g., truncated form) of CD34, a NGFR, a CD19 or a truncated CD19, e.g., a truncated non-human CD19, or epidermal growth factor receptor *(e.g.,* tEGFR).

In some embodiments, the marker is or comprises a fluorescent protein, such as green fluorescent protein (GFP), enhanced green fluorescent protein (EGFP), such as super-fold GFP (sfGFP), red fluorescent protein (RFP), such as tdTomato, mCherry, mStrawberry, AsRed2, DsRed or DsRed2, cyan fluorescent protein (CFP), blue green fluorescent protein (BFP), enhanced blue fluorescent protein (EBFP), and yellow fluorescent protein (YFP), and variants thereof, including species variants, monomeric variants, and codon-optimized and/or enhanced variants of the fluorescent proteins. In some embodiments, the marker is or comprises an enzyme, such as a luciferase, the lacZ gene from *E. coli,* alkaline phosphatase, secreted embryonic alkaline phosphatase (SEAP), chloramphenicol acetyl transferase (CAT). Exemplary light-emitting reporter genes include luciferase (luc), β-galactosidase, chloramphenicol acetyltransferase (CAT), β-glucuronidase (GUS) or variants thereof.

In some embodiments, the marker is a selection marker. In some embodiments, the selection marker is or comprises a polypeptide that confers resistance to exogenous agents or drugs. In some embodiments, the selection marker is an antibiotic resistance gene. In some embodiments, the selection marker is an antibiotic resistance gene confers antibiotic resistance to a mammalian cell. In some embodiments, the selection marker is or comprises a Puromycin resistance gene, a Hygromycin resistance gene, a Blasticidin resistance gene, a Neomycin resistance gene, a Geneticin resistance gene or a Zeocin resistance gene or a modified form thereof.

In some embodiments, the nucleic acid encoding the marker is operably linked to a polynucleotide encoding for a linker sequence, such as a cleavable linker sequence, *e.g.,* a T2A. For example, a marker, and optionally a linker sequence, can be any as disclosed in PCT Pub. No. WO2014031687. For example, the marker can be a truncated EGFR (tEGFR) that is, optionally, linked to a linker sequence, such as a T2A cleavable linker sequence. An exemplary polypeptide for a truncated EGFR (*e.g*. tEGFR) comprises the sequence of amino acids set forth in SEQ ID NO: 7 or 16 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 7 or 16. In some examples, a truncated epidermal growth factor receptor (EGFRt), such as set forth in SEQ ID NOs: 7 or 16, in some cases can be co-expressed with a transgene of interest (a CAR or TCR) in transduced cells (see e.g. U.S. Patent No. 8,802,374). EGFRt may contain an epitope recognized by the antibody cetuximab (Erbitux^{®}) or other therapeutic anti-EGFR antibody or binding molecule, which can be used to identify or select cells that have been engineered with the EGFRt construct and another recombinant receptor, such as a chimeric antigen receptor (CAR), and/or to eliminate or separate cells expressing the receptor. See U.S. Patent No. 8,802,374 and Liu et al., Nature Biotech. 2016 April; 34(4): 430-434).

Also provided are vectors or constructs containing such nucleic acids and/or polynucleotides. In some embodiments, the vectors or constructs contain one or more promoters operatively linked to the nucleic acid encoding the recombinant receptor to drive expression thereof. In some embodiments, the promoter is operatively linked to one or more than one nucleic acid molecules or polynucleotides. Thus, also provided are vectors, such as those that contain any of the polynucleotides provided herein.

In some cases, the vector is a viral vector, such as a retroviral vector, e.g., a lentiviral vector or a gammaretroviral vector. Also provided are compositions containing such vectors or combination of vectors. In some embodiments, the set or combination of vectors, are used together for engineering of cells. In some embodiments, the first and the second vectors in the set are introduced simultaneously or sequentially, in any order into a cell for engineering.

In some embodiments, the vectors include viral vectors, e.g., retroviral or lentiviral, non-viral vectors or transposons, e.g. *Sleeping Beauty* transposon system, vectors derived from simian virus 40 (SV40), adenoviruses, adeno-associated virus (AAV), lentiviral vectors or retroviral vectors, such as gamma-retroviral vectors, retroviral vector derived from the Moloney murine leukemia virus (MoMI,V), myeloproliferative sarcoma virus (MPSV), murine embryonic stem cell virus (MESV), murine stem cell virus (MSCV), spleen focus forming virus (SFFV) or adeno-associated virus (AAV).

### C. Vectors and methods for genetically engineering

Various methods for the introduction of genetically engineered components, e.g., recombinant receptors, e.g., CARs or TCRs, are well known. Exemplary methods include those for transfer of nucleic acids encoding the receptors, including via viral, e.g., retroviral or lentiviral, transduction, transposons, and electroporation. In some embodiments, surface glycan expression is assessed in a composition of cells that are collected prior to, during, or immediately after the genetic engineering process. In some embodiments, the surface glycan expression is assessed and compared among compositions of cells at corresponding stages of the process to introduce genetically engineered components. In some embodiments, the compositions are or have been engineered to express the same recombinant receptor, but by different methods of introducing the genetic material.

In some embodiments, gene transfer is accomplished by first stimulating the cell, such as by combining it with a stimulus that induces a response such as proliferation, survival, and/or activation, e.g., as measured by expression of a cytokine or activation marker, followed by transduction of the activated cells, and expansion in culture to numbers sufficient for clinical applications.

In some embodiments, recombinant nucleic acids are transferred into cells using recombinant infectious virus particles, such as, e.g., vectors derived from simian virus 40 (SV40), adenoviruses, adeno-associated virus (AAV). In some embodiments, recombinant nucleic acids are transferred into T cells using recombinant lentiviral vectors or retroviral vectors, such as gamma-retroviral vectors (see, e.g., Koste et al. Gene Therapy doi: 10.1038/gt.2014.25 (2014); Carlens et al. Exp Hematol., 28(10): 1137-46 (2000); Alonso-Camino et al. Mol Ther Nucl Acids, 2, e93 (2013); Park et al., Trends Biotechnol., November 29(11): 550-557 (2011). In some embodiments, the virus is adeno-associated virus (AAV).

In some embodiments, the retroviral vector has a long terminal repeat sequence (LTR), e.g., a retroviral vector derived from the Moloney murine leukemia virus (MoMI,V), myeloproliferative sarcoma virus (MPSV), murine embryonic stem cell virus (MESV), murine stem cell virus (MSCV), spleen focus forming virus (SFFV). Most retroviral vectors are derived from murine retroviruses. In some embodiments, the retroviruses include those derived from any avian or mammalian cell source. The retroviruses typically are amphotropic, meaning that they are capable of infecting host cells of several species, including humans. In one embodiment, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman, BioTechniques, 7:980-990 (1989); Miller, A. D. Human Gene Therapy, 1:5-14 (1990); Scarpa et al. Virology, 180:849-852 (1991); Burns et al. Proc. Natl. Acad. Sci. USA, 90:8033-8037 (1993); and Boris-Lawrie and Temin, Cur. Opin. Genet. Develop., 3:102-109 (1993).

Methods of lentiviral transduction are known. Exemplary methods are described in, e.g., Wang et al., J. Immunother., 35(9): 689-701 (2012); Cooper et al. Blood. 101:1637-1644 (2003); Verhoeyen et al., Methods Mol Biol., 506: 97-114 (2009); and Cavalieri et al., Blood., 102(2): 497-505 (2003).

In some embodiments, recombinant nucleic acids are transferred into T cells via electroporation (see, e.g., Chicaybam et al, PLoS ONE 8(3): e60298 (2013) and Van Tedeloo et al. Gene Therapy 7(16): 1431-1437 (2000)). In some embodiments, recombinant nucleic acids are transferred into T cells via transposition (see, e.g., Manuri et al. Hum Gene Ther 21(4): 427-437 (2010); Sharma et al. Molec Ther Nucl Acids 2, e74 (2013); and Huang et al. Methods Mol Biol 506: 115-126 (2009)). Other methods of introducing and expressing genetic material in immune cells include calcium phosphate transfection (e.g., as described in Current Protocols in Molecular Biology, John Wiley & Sons, New York. N.Y.), protoplast fusion, cationic liposome-mediated transfection; tungsten particle-facilitated microparticle bombardment (Johnston, Nature, 346: 776-777 (1990)); and strontium phosphate DNA co-precipitation (Brash et al., Mol. Cell Biol., 7: 2031-2034 (1987)).

Other approaches and vectors for transfer of the nucleic acids encoding the recombinant products are those described, e.g., in international patent application, Publication No.: WO2014055668, and U.S. Patent No. 7,446,190.

In some embodiments, the cells, e.g., T cells, may be transfected either during or after expansion e.g., with a T cell receptor (TCR) or a chimeric antigen receptor (CAR). This transfection for the introduction of the gene of the desired receptor can be carried out with any suitable retroviral vector, for example. The genetically modified cell population can then be liberated from the initial stimulus (the CD3/CD28 stimulus, for example) and subsequently be stimulated with a second type of stimulus e.g., via a de novo introduced receptor). This second type of stimulus may include an antigenic stimulus in form of a peptide/MHC molecule, the cognate (cross-linking) ligand of the genetically introduced receptor (e.g., natural ligand of a CAR) or any ligand (such as an antibody) that directly binds within the framework of the new receptor (e.g., by recognizing constant regions within the receptor). See, for example, Cheadle et al, Methods Mol Biol. 907:645-66 (2012);or Barrett et al., Chimeric Antigen Receptor Therapy for Cancer Annual Review of Medicine, Vol. 65: 333-347 (2014).

In some cases, a vector may be used that does not require that the cells, e.g., T cells, are activated. In some such instances, the cells may be selected and/or transduced prior to activation. Thus, the cells may be engineered prior to, or subsequent to culturing of the cells, and in some cases at the same time as or during at least a portion of the culturing.

In some aspects, the cells further are engineered to promote expression of cytokines or other factors. Among additional nucleic acids, e.g., genes for introduction are those to improve the efficacy of therapy, such as by promoting viability and/or function of transferred cells; genes to provide a genetic marker for selection and/or evaluation of the cells, such as to assess in vivo survival or localization; genes to improve safety, for example, by making the cell susceptible to negative selection in vivo as described by Lupton S. D. et al., Mol. and Cell Biol., 11:6 (1991); and Riddell et al., Human Gene Therapy 3:319-338 (1992); see also the publications of PCT/US91/08442 and PCT/US94/05601 by Lupton et al. describing the use of bifunctional selectable fusion genes derived from fusing a dominant positive selectable marker with a negative selectable marker. See, e.g., Riddell et al., US Patent No. 6,040,177, at columns 14-17.

### D. Features of the Output Composition

In particular embodiments, the methods provided herein produce or generate a composition of cells that contain genetically engineered cells, e.g., an output composition. In certain embodiments, an output composition is a cell composition that results from some or all of the steps for genetically engineering cells. In certain embodiments, the output composition results from a process of genetically engineering cells of an input cell composition. In certain embodiments, process contains one or more steps for activating, transducing or transfecting, expanding, and/or harvesting cells, such as cells that were obtained from an input cell composition. In certain embodiments, the output cell composition contains cells that have been genetically engineered. In particular embodiments, the cells of the output composition have undergone all of the steps for a process of genetic engineering.

In some embodiments, the output composition contains cells that include one or more nucleic acids introduced via genetic engineering, and thereby express recombinant or genetically engineered products of such nucleic acids. In some embodiments, the nucleic acids are heterologous, i.e., normally not present in a cell or sample obtained from the cell, such as one obtained from another organism or cell, which for example, is not ordinarily found in the cell being engineered and/or an organism from which such cell is derived. In some embodiments, the nucleic acids are not naturally occurring, such as a nucleic acid not found in nature, including one comprising chimeric combinations of nucleic acids encoding various domains from multiple different cell types.

In some embodiments, the output composition contains cells that have been genetically engineered. In particular embodiments, the output cell composition contains engineered T cells. In some embodiments, the engineered T cells include engineered CD4+ T cells and engineered CD8+ T cells. In particular embodiments, the output composition contains or includes at least 25%, at least 30%, at least 40%, at least 50%, at least 60%, at least 70% , at least 75%, at least 80%, at least 85%, at least 90%, at least 95%, at least 96%, at least 97%, at least 98%, at least 99%, at least 99.5%, at least 99.9%, or 100% or about 100% engineered T cells. In certain embodiments, the engineered cells express a recombinant receptor. In particular embodiments, the output composition contains or includes at least 25%, at least 30%, at least 40%, at least 50%, at least 60%, at least 70% , at least 75%, at least 80%, at least 85%, at least 90%, at least 95%, at least 96%, at least 97%, at least 98%, at least 99%, at least 99.5%, at least 99.9%, or 100% or about 100% T cells that express a recombinant receptor. In some embodiments, the recombinant receptor is a TCR or a CAR. In particular embodiments, the recombinant receptor is a CAR.

### III. COMPOSITIONS AND FORMULATIONS

Provided herein are compositions or formulations containing cells prepared according to the methods of incubating (e.g., stimulating) described herein. In some embodiments, the compositions and methods described herein can be used to eliminate at least a portion of populations of cells, such as non-naïve-like T cells from a population of cells. Further provided are compositions comprising populations of cells that no longer contain undesired cells, or have a significantly reduced number of undesired cells in the stimulated composition, such as cells derived from non-naive like T cells and uses thereof. The compositions and methods provided herein are also used to selectively expand a population of cells that have been deleted for undesired subpopulations for use in the treatment. Also provided here is an output or enriched stimulated composition produced by any of the methods of stimulating T cells described herein.

In some embodiments, the cells produced using any of the methods of incubating (e.g., stimulating) described herein, such as cells genetically engineered with a recombinant receptor (e.g., CAR-T cells) are provided as compositions, including pharmaceutical compositions and formulations, such as unit dose form compositions including the number of cells for administration in a given dose or fraction thereof. The pharmaceutical compositions and formulations generally include one or more optional pharmaceutically acceptable carrier or excipient. In some embodiments, the composition includes at least one additional therapeutic agent.

In some embodiments, a composition of cells is generated or manufactured for the purposes of a cell therapy. In some embodiments, the cell composition is a pharmaceutical composition or formulation. Such compositions can be used in accord with the provided methods, for example, to assess their release for use in the prevention or treatment of diseases, conditions, and disorders, or in detection, diagnostic, and prognostic methods.

The term "pharmaceutical formulation" refers to a preparation which is in such form as to permit the biological activity of an active ingredient contained therein to be effective, and which contains no additional components which are unacceptably toxic to a subject to which the formulation would be administered. In some embodiments, methods provided herein may be used to compare surface glycan expression of cell compositions composed of the same engineered cells, but with different pharmaceutical formulations.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative. In particular embodiments, methods provided herein may be used to compare surface glycan expression of cell compositions composed of the same engineered cells, but with different pharmaceutically acceptable carriers.

In some embodiments, the T cell therapy, such as engineered T cells (e.g., CAR T cells), are formulated with a pharmaceutically acceptable carrier. In some aspects, the choice of carrier is determined in part by the particular cell and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some aspects, a mixture of two or more preservatives is used. The preservative or mixtures thereof are typically present in an amount of about 0.0001% to about 2% by weight of the total composition. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as polyethylene glycol (PEG).

Buffering agents in some aspects are included in the compositions. Suitable buffering agents include, for example, citric acid, sodium citrate, phosphoric acid, potassium phosphate, and various other acids and salts. In some aspects, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

The formulations can include aqueous solutions. The formulation or composition may also contain more than one active ingredient useful for the particular indication, disease, or condition being prevented or treated with the cells, including one or more active ingredients where the activities are complementary to the cells and/or the respective activities do not adversely affect one another. Such active ingredients are suitably present in combination in amounts that are effective for the purpose intended. Thus, in some embodiments, the pharmaceutical composition further includes other pharmaceutically active agents or drugs, such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc.

The pharmaceutical composition in some embodiments contains cells in amounts effective to treat or prevent the disease or condition, such as a therapeutically effective or prophylactically effective amount. Therapeutic or prophylactic efficacy in some embodiments is monitored by periodic assessment of treated subjects. For repeated administrations over several days or longer, depending on the condition, the treatment is repeated until a desired suppression of disease symptoms occurs. However, other dosage regimens may be useful and can be determined. The desired dosage can be delivered by a single bolus administration of the composition, by multiple bolus administrations of the composition, or by continuous infusion administration of the composition.

The cells may be formulated for administration using standard administration techniques, formulations, and/or devices. Provided are formulations and devices, such as syringes and vials, for storage and administration of the compositions. With respect to cells, administration can be autologous or heterologous. For example, immunoresponsive cells or progenitors can be obtained from one subject, and administered to the same subject or a different, compatible subject. Peripheral blood derived immunoresponsive cells or their progeny (e.g., in vivo, ex vivo or in vitro derived) can be administered via localized injection, including catheter administration, systemic injection, localized injection, intravenous injection, or parenteral administration. When administering a therapeutic composition (e.g., a pharmaceutical composition containing a genetically modified immunoresponsive cell), it will generally be formulated in a unit dosage injectable form (solution, suspension, emulsion).

Formulations include those for oral, intravenous, intraperitoneal, subcutaneous, pulmonary, transdermal, intramuscular, intranasal, buccal, sublingual, or suppository administration. In some embodiments, the agent or cell populations are administered parenterally. The term "parenteral," as used herein, includes intravenous, intramuscular, subcutaneous, rectal, vaginal, and intraperitoneal administration. In some embodiments, the agent or cell populations are administered to a subject using peripheral systemic delivery by intravenous, intraperitoneal, or subcutaneous injection.

Compositions in some embodiments are provided as sterile liquid preparations, e.g., isotonic aqueous solutions, suspensions, emulsions, dispersions, or viscous compositions, which may in some aspects be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol) and suitable mixtures thereof.

Sterile injectable solutions can be prepared by incorporating the cells in a solvent, such as in admixture with a suitable carrier, diluent, or excipient such as sterile water, physiological saline, glucose, dextrose, or the like. The compositions can also be lyophilized. The compositions can contain auxiliary substances such as wetting, dispersing, or emulsifying agents (e.g., methylcellulose), pH buffering agents, gelling or viscosity enhancing additives, preservatives, flavoring agents, colors, and the like, depending upon the route of administration and the preparation desired. Standard texts may in some aspects be consulted to prepare suitable preparations.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. Prolonged absorption of the injectable pharmaceutical form can be brought about by the use of agents delaying absorption, for example, aluminum monostearate and gelatin.

The formulations to be used for *in vivo* administration are generally sterile. Sterility may be readily accomplished, e.g., by filtration through sterile filtration membranes.

For the prevention or treatment of disease, the appropriate dosage may depend on the type of disease to be treated, the type of agent or agents, the type of cells or recombinant receptors, the severity and course of the disease, whether the agent or cells are administered for preventive or therapeutic purposes, previous therapy, the subject's clinical history and response to the agent or the cells, and the discretion of the attending physician. The compositions are in some embodiments suitably administered to the subject at one time or over a series of treatments.

Also provided are methods of using and uses of the cells and compositions, such as those present in an output composition described herein, in the treatment of diseases, conditions, and disorders in which the antigen recognized by the recombinant receptor (e.g. CAR) is expressed. Also provided herein are methods of treatment including administering to a subject an output or enriched output composition produced by any of the method of incubating (e.g., stimulating) described. In some embodiments, the method includes generating genetically engineered T cells using any of the methods described herein and administering the genetically engineered T cells from produced by the methods described herein.

Provided are methods of administering the engineered cells and compositions, and uses of such engineered cells and compositions to treat or prevent diseases, conditions, and disorders, including cancers. In some embodiments, the cell-based therapy is or comprises administration of cells, such as immune cells, for example T cell, that target a molecule expressed on the surface of a lesion, such as a tumor or a cancer. The provided methods and uses include methods and uses for adoptive cell therapy. In some embodiments, the methods include administration of the engineered cells or a composition containing the cells, such as cells from an output composition as described, to a subject, tissue, or cell, such as one having, at risk for, or suspected of having the disease, condition or disorder. In some embodiments, the cells, populations, and compositions are administered to a subject having the particular disease or condition to be treated, e.g., via adoptive cell therapy, such as adoptive T cell therapy. In some embodiments, the cells or compositions are administered to the subject, such as a subject having or at risk for the disease or condition, ameliorate one or more symptom of the disease or condition, such as by lessening tumor burden in a cancer expressing an antigen recognized by an engineered T cell.

The disease or condition that is treated in some aspects can be any in which expression of an antigen is associated with, specific to, and/or expressed on a cell or tissue of a disease, disorder or condition and/or involved in the etiology of a disease, condition or disorder, e.g. causes, exacerbates or otherwise is involved in such disease, condition, or disorder. Exemplary diseases and conditions can include diseases or conditions associated with malignancy or transformation of cells (e.g. cancer), autoimmune or inflammatory disease, or an infectious disease, e.g. caused by a bacterial, viral or other pathogen. Exemplary antigens, which include antigens associated with various diseases and conditions that can be treated, are described above. In particular embodiments, the immunomodulatory polypeptide and/or recombinant receptor, e.g., the chimeric antigen receptor or TCR, specifically binds to an antigen associated with the disease or condition. In some embodiments, the subject has a disease, disorder or condition, optionally a cancer, a tumor, an autoimmune disease, disorder or condition, or an infectious disease.

In some embodiments, the disease, disorder or condition includes tumors associated with various cancers. The cancer can in some embodiments be any cancer located in the body of a subject, such as, but not limited to, cancers located at the head and neck, breast, liver, colon, ovary, prostate, pancreas, brain, cervix, bone, skin, eye, bladder, stomach, esophagus, peritoneum, or lung. For example, the anti-cancer agent can be used for the treatment of colon cancer, cervical cancer, cancer of the central nervous system, breast cancer, bladder cancer, anal carcinoma, head and neck cancer, ovarian cancer, endometrial cancer, small cell lung cancer, non-small cell lung carcinoma, neuroendocrine cancer, soft tissue carcinoma, penile cancer, prostate cancer, pancreatic cancer, gastric cancer, gall bladder cancer or espohageal cancer. In some cases, the cancer can be a cancer of the blood. In some embodiments, the disease, disorder or condition is a tumor, such as a solid tumor, lymphoma, leukemia, blood tumor, metastatic tumor, or other cancer or tumor type. In some embodiments, the disease, disorder or condition is selected from among cancers of the colon, lung, liver, breast, prostate, ovarian, skin, melanoma, bone, brain cancer, ovarian cancer, epithelial cancers, renal cell carcinoma, pancreatic adenocarcinoma, cervical carcinoma, colorectal cancer, glioblastoma, neuroblastoma, Ewing sarcoma, medulloblastoma, osteosarcoma, synovial sarcoma, and/or mesothelioma.

Among the diseases, conditions, and disorders are tumors, including solid tumors, hematologic malignancies, and melanomas, and including localized and metastatic tumors, infectious diseases, such as infection with a virus or other pathogen, e.g., HIV, HCV, HBV, CMV, HPV, and parasitic disease, and autoimmune and inflammatory diseases. In some embodiments, the disease, disorder or condition is a tumor, cancer, malignancy, neoplasm, or other proliferative disease or disorder. Such diseases include but are not limited to leukemia, lymphoma, e.g., acute myeloid (or myelogenous) leukemia (AML), chronic myeloid (or myelogenous) leukemia (CML), acute lymphocytic (or lymphoblastic) leukemia (ALL), chronic lymphocytic leukemia (CLL), hairy cell leukemia (HCL), small lymphocytic lymphoma (SLL), Mantle cell lymphoma (MCL), Marginal zone lymphoma, Burkitt lymphoma, Hodgkin lymphoma (HL), non-Hodgkin lymphoma (NHL), Anaplastic large cell lymphoma (ALCL), follicular lymphoma, refractory follicular lymphoma, diffuse large B-cell lymphoma (DLBCL) and multiple myeloma (MM), a B cell malignancy is selected from among acute lymphoblastic leukemia (ALL), adult ALL, chronic lymphoblastic leukemia (CLL), non-Hodgkin lymphoma (NHL), and Diffuse Large B-Cell Lymphoma (DLBCL).

In some embodiments, the disease or condition is an infectious disease or condition, such as, but not limited to, viral, retroviral, bacterial, and protozoal infections, immunodeficiency, Cytomegalovirus (CMV), Epstein-Barr virus (EBV), adenovirus, BK polyomavirus. In some embodiments, the disease or condition is an autoimmune or inflammatory disease or condition, such as arthritis, e.g., rheumatoid arthritis (RA), Type I diabetes, systemic lupus erythematosus (SLE), inflammatory bowel disease, psoriasis, scleroderma, autoimmune thyroid disease, Grave's disease, Crohn's disease, multiple sclerosis, asthma, and/or a disease or condition associated with transplant.

In some embodiments, the antigen associated with the disease or disorder is selected from the group consisting of orphan tyrosine kinase receptor ROR1, B cell maturation antigen (BCMA), carbonic anhydrase 9 (CAIX), tEGFR, Her2/neu (receptor tyrosine kinase erbB2), L1-CAM, CD19, CD20, CD22, mesothelin, CEA, hepatitis B surface antigen, anti-folate receptor, CD23, CD24, CD30, CD33, CD38, CD44, EGFR, epithelial glycoprotein 2 (EPG-2), epithelial glycoprotein 40 (EPG-40), ephrine receptor A2 (EPHa2), Her2/neu (receptor tyrosine kinase erb-B2), Her3 (erb-B3), Her4 (erb-B4), erbB dimers, type III epidermal growth factor receptor mutation (EGFR vIII), folate binding protein (FBP), FCRL5, Fc receptor like 5 (FCRL5; also known as Fc receptor homolog 5 or FCRH5), fetal acetylcholine receptor, ganglioside GD2, ganglioside GD3, G Protein Coupled Receptor 5D (GPCR5D), HMW-MAA, IL-22R-alpha, IL-13R-alpha2, kinase insert domain receptor (kdr), kappa light chain, Leucine Rich Repeat Containing 8 Family Member A (LRRC8A), Lewis Y, L1-cell adhesion molecule, (L1-CAM), Melanoma-associated antigen (MAGE)-A1, MAGE-A3, MAGE-A6, Preferentially expressed antigen of melanoma (PRAME), survivin, TAG72, B7-H6, IL-13 receptor alpha 2 (IL-13Ra2), CA9, GD3, HMW-MAA, CD171, G250/CAIX, Human leukocyte antigen A1 (HLA-A1), MAGE A1, HLA-A2, NY-ESO-1, PSCA, folate receptor-a, CD44v6, CD44v7/8, αvβ6 integrin (avb6 integrin), 8H9, NCAM, VEGF receptors, 5T4, Foetal AchR, natural killer group 2 member D (NKG2D) ligands, CD44v6, dual antigen, a cancer-testes antigen, mesothelin, murine CMV, mucin 1 (MUC1), MUC16, prostate stem cell antigen (PSCA), NKG2D, a cancer-testis antigen cancer/testis antigen 1B (CTAG, also known as NY-ESO-1 and LAGE-2), MART-1, glycoprotein 100 (gp100), oncofetal antigen, ROR1, Trophoblast glycoprotein (TPBG also known as 5T4), TAG72, VEGF-R2, carcinoembryonic antigen (CEA), Her2/neu, estrogen receptor, progesterone receptor, ephrinB2, CD123, c-Met, GD-2, O-acetylated GD2 (OGD2), CE7, Wilms Tumor 1 (WT-1), a cyclin, cyclin A2, C-C Motif Chemokine Ligand 1 (CCL-1), CD138, a pathogen-specific antigen and an antigen associated with a universal tag, and/or biotinylated molecules, and/or molecules expressed by HIV, HCV, HBV or other pathogens.

In some embodiments, the antigen or ligand is a tumor antigen or cancer marker. In some embodiments, the antigen or ligand the antigen is or includes αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), , type III epidermal growth factor receptor mutation (EGFR vIII), epithelial glycoprotein 2 (EPG-2), epithelial glycoprotein 40 (EPG-40), ephrinB2, ephrine receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPCR5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens.

In some embodiments, the disease or condition is a B cell malignancy. In some embodiments, the B cell malignancy is a leukemia or a lymphoma. In some aspects, the disease or condition is acute lymphoblastic leukemia (ALL), adult ALL, chronic lymphoblastic leukemia (CLL), non-Hodgkin lymphoma (NHL), or Diffuse Large B-Cell Lymphoma (DLBCL). In some cases, the disease or condition is an NHL, such as or including an NHL that is an aggressive NHL, diffuse large B cell lymphoma (DLBCL), NOS (de novo and transformed from indolent), primary mediastinal large B cell lymphoma (PMBCL), T cell/histocyte-rich large B cell lymphoma (TCHRBCL), Burkitt's lymphoma, mantle cell lymphoma (MCL), and/or follicular lymphoma (FL), optionally, follicular lymphoma Grade 3B (FL3B). In some aspects, the recombinant receptor, such as a CAR, specifically binds to an antigen associated with the disease or condition or expressed in cells of the environment of a lesion associated with the B cell malignancy. Antigens targeted by the receptors in some embodiments include antigens associated with a B cell malignancy, such as any of a number of known B cell marker. In some embodiments, the antigen targeted by the receptor is CD20, CD19, CD22, ROR1, CD45, CD21, CD5, CD33, Igkappa, Iglambda, CD79a, CD79b or CD30.

In some embodiments, the disease or condition is a myeloma, such as a multiple myeloma. In some aspects, the recombinant receptor, such as a CAR, specifically binds to an antigen associated with the disease or condition or expressed in cells of the environment of a lesion associated with the multiple myeloma. Antigens targeted by the receptors in some embodiments include antigens associated with multiple myeloma, such as GPRC5d or BCMA.

In some embodiments, the antigen is a pathogen-specific or pathogen-expressed antigen. In some embodiments, the antigen is a viral antigen (such as a viral antigen from HIV, HCV, HBV, etc.), bacterial antigens, and/or parasitic antigens.

In some embodiments, the immune cells express a T cell receptor (TCR) or other antigen-binding receptor. In some embodiments, the immune cells express a recombinant receptor, such as a transgenic TCR or a chimeric antigen receptor (CAR). In some embodiments, the cells are autologous to the subject. In some embodiments, the cells are allogeneic to the subject.

Methods for administration of engineered cells for adoptive cell therapy are known and may be used in connection with the provided methods and compositions. For example, adoptive T cell therapy methods are described, e.g., in US Patent Application Publication No. 2003/0170238 to Gruenberg et al; US Patent No. 4,690,915 to Rosenberg; Rosenberg (2011) Nat Rev Clin Oncol. 8(10):577-85). See, *e.g.,* Themeli et al., (2013) Nat Biotechnol. 31(10): 928-933; Tsukahara et al., (2013) Biochem Biophys Res Commun 438(1): 84-9; Davila et al., (2013) PLoS ONE 8(4): e61338.

In some embodiments, the cell therapy, e.g., adoptive T cell therapy, is carried out by autologous transfer, in which the cells are isolated and/or otherwise prepared from the subject who is to receive the cell therapy, or from a sample derived from such a subject. Thus, in some aspects, the cells are derived from a subject, e.g., patient, in need of a treatment and the cells, following isolation and processing are administered to the same subject.

In some embodiments, the cell therapy, e.g., adoptive T cell therapy, is carried out by allogeneic transfer, in which the cells are isolated and/or otherwise prepared from a subject other than a subject who is to receive or who ultimately receives the cell therapy, *e.g.,* a first subject. In such embodiments, the cells then are administered to a different subject, *e.g.,* a second subject, of the same species. In some embodiments, the first and second subjects are genetically identical. In some embodiments, the first and second subjects are genetically similar. In some embodiments, the second subject expresses the same HLA class or supertype as the first subject.

In certain embodiments, the cells, or individual populations of sub-types of cells, are administered to the subject at a range of about one million to about 100 billion cells and/or that amount of cells per kilogram of body weight, such as, e.g., 1 million to about 50 billion cells (e.g., about 5 million cells, about 25 million cells, about 500 million cells, about 1 billion cells, about 5 billion cells, about 20 billion cells, about 30 billion cells, about 40 billion cells, or a range defined by any two of the foregoing values), such as about 10 million to about 100 billion cells (e.g., about 20 million cells, about 30 million cells, about 40 million cells, about 60 million cells, about 70 million cells, about 80 million cells, about 90 million cells, about 10 billion cells, about 25 billion cells, about 50 billion cells, about 75 billion cells, about 90 billion cells, or a range defined by any two of the foregoing values), and in some cases about 100 million cells to about 50 billion cells (e.g., about 120 million cells, about 250 million cells, about 350 million cells, about 450 million cells, about 650 million cells, about 800 million cells, about 900 million cells, about 3 billion cells, about 30 billion cells, about 45 billion cells) or any value in between these ranges and/or per kilogram of body weight. Dosages may vary depending on attributes particular to the disease or disorder and/or patient and/or other treatments.

In some embodiments, for example, where the subject is a human, the dose includes fewer than about 5 × 10⁸ total recombinant receptor (e.g., CAR)-expressing cells, T cells, or peripheral blood mononuclear cells (PBMCs). In some embodiments, for example, where the subject is a human, the dose includes fewer than about 1 × 10⁸ total recombinant receptor (e.g., CAR)-expressing cells, T cells, or peripheral blood mononuclear cells (PBMCs), e.g., in the range of about 1 × 10⁶ to 1 × 10⁸ such cells, such as 2 × 10⁶, 5 × 10⁶, 1 × 10⁷, 5 × 10⁷, or 1 × 10⁸ or total such cells, or the range between any two of the foregoing values.

In some embodiments, the dose of genetically engineered cells comprises from or from about 1 × 10⁵ to 5 × 10⁸ total CAR-expressing T cells, 1 × 10⁵ to 2.5 × 10⁸ total CAR-expressing T cells, 1 × 10⁵ to 1 × 10⁸ total CAR-expressing T cells, 1 × 10⁵ to 5 × 10⁷ total CAR-expressing T cells, 1 × 10⁵ to 2.5 × 10⁷ total CAR-expressing T cells, 1 × 10⁵ to 1 × 10⁷ total CAR-expressing T cells, 1 × 10⁵ to 5 × 10⁶ total CAR-expressing T cells, 1 × 10⁵ to 2.5 × 10⁶ total CAR-expressing T cells, 1 × 10⁵ to 1 × 10⁶ total CAR-expressing T cells, 1 × 10⁶ to 5 × 10⁸ total CAR-expressing T cells, 1 × 10⁶ to 2.5 × 10⁸ total CAR-expressing T cells, 1 × 10⁶ to 1 × 10⁸ total CAR-expressing T cells, 1 × 10⁶ to 5 × 10⁷ total CAR-expressing T cells, 1 × 10⁶ to 2.5 × 10⁷ total CAR-expressing T cells, 1 × 10⁶ to 1 × 10⁷ total CAR-expressing T cells, 1 × 10⁶ to 5 × 10⁶ total CAR-expressing T cells, 1 × 10⁶ to 2.5 × 10⁶ total CAR-expressing T cells, 2.5 × 10⁶ to 5 × 10⁸ total CAR-expressing T cells, 2.5 × 10⁶ to 2.5 × 10⁸ total CAR-expressing T cells, 2.5 × 10⁶ to 1 × 10⁸ total CAR-expressing T cells, 2.5 × 10⁶ to 5 × 10⁷ total CAR-expressing T cells, 2.5 × 10⁶ to 2.5 × 10⁷ total CAR-expressing T cells, 2.5 × 10⁶ to 1 × 10⁷ total CAR-expressing T cells, 2.5 × 10⁶ to 5 × 10⁶ total CAR-expressing T cells, 5 × 10⁶ to 5 × 10⁸ total CAR-expressing T cells, 5 × 10⁶ to 2.5 × 10⁸ total CAR-expressing T cells, 5 × 10⁶ to 1 × 10⁸ total CAR-expressing T cells, 5 × 10⁶ to 5 × 10⁷ total CAR-expressing T cells, 5 × 10⁶ to 2.5 × 10⁷ total CAR-expressing T cells, 5 × 10⁶ to 1 × 10⁷ total CAR-expressing T cells, 1 × 10⁷ to 5 × 10⁸ total CAR-expressing T cells, 1 × 10⁷ to 2.5 × 10⁸ total CAR-expressing T cells, 1 × 10⁷ to 1 × 10⁸ total CAR-expressing T cells, 1 × 10' to 5 × 10⁷ total CAR-expressing T cells, 1 × 10⁷ to 2.5 × 10⁷ total CAR-expressing T cells, 2.5 × 10⁷ to 5 × 10⁸ total CAR-expressing T cells, 2.5 × 10⁷ to 2.5 × 10⁸ total CAR-expressing T cells, 2.5 × 10⁷ to 1 × 10⁸ total CAR-expressing T cells, 2.5 × 10⁷ to 5 × 10⁷ total CAR-expressing T cells, 5 × 10⁷ to 5 × 10⁸ total CAR-expressing T cells, 5 × 10⁷ to 2.5 × 10⁸ total CAR-expressing T cells, 5 × 10⁷ to 1 × 10⁸ total CAR-expressing T cells, 1 × 10⁸ to 5 × 10⁸ total CAR-expressing T cells, 1 × 10⁸ to 2.5 × 10⁸ total CAR-expressing T cells, or 2.5 × 10⁸ to 5 × 10⁸ total CAR-expressing T cells.

In some embodiments, the dose of genetically engineered cells comprises at least or at least about 1 × 10⁵ CAR-expressing cells, at least or at least about 2.5 × 10⁵ CAR-expressing cells, at least or at least about 5 × 10⁵ CAR-expressing cells, at least or at least about 1 × 10⁶ CAR-expressing cells, at least or at least about 2.5 × 10⁶ CAR-expressing cells, at least or at least about 5 × 10⁶ CAR-expressing cells, at least or at least about 1 × 10⁷ CAR-expressing cells, at least or at least about 2.5 × 10⁷ CAR-expressing cells, at least or at least about 5 × 10⁷ CAR-expressing cells, at least or at least about 1 × 10⁸ CAR-expressing cells, at least or at least about 2.5 × 10⁸ CAR-expressing cells, or at least or at least about 5 × 10⁸ CAR-expressing cells.

In some embodiments, the cell therapy comprises administration of a dose comprising a number of cell from or from about 1 × 10⁵ to 5 × 10⁸ total recombinant receptor-expressing cells, total T cells, or total peripheral blood mononuclear cells (PBMCs), from or from about 5 × 10⁵ to 1 × 10⁷ total recombinant receptor-expressing cells, total T cells, or total peripheral blood mononuclear cells (PBMCs) or from or from about 1 × 10⁶ to 1 × 10⁷ total recombinant receptor-expressing cells, total T cells, or total peripheral blood mononuclear cells

(PBMCs), each inclusive. In some embodiments, the cell therapy comprises administration of a dose of cells comprising a number of cells at least or at least about 1 × 10⁵ total recombinant receptor-expressing cells, total T cells, or total peripheral blood mononuclear cells (PBMCs), such at least or at least 1 × 10⁶, at least or at least about 1 × 10⁷, at least or at least about 1 × 10⁸ of such cells. In some embodiments, the number is with reference to the total number of CD3+ or CD8+, in some cases also recombinant receptor-expressing (e.g. CAR+) cells. In some embodiments, the cell therapy comprises administration of a dose comprising a number of cell from or from about 1 × 10⁵ to 5 × 10⁸ CD3+ or CD8+ total T cells or CD3+ or CD8+ recombinant receptor-expressing cells, from or from about 5 × 10⁵ to 1 × 10⁷ CD3+ or CD8+ total T cells or CD3+ or CD8+ recombinant receptor-expressing cells, or from or from about 1 × 10⁶ to 1 × 10⁷ CD3+ or CD8+ total T cells or CD3+ or CD8+recombinant receptor-expressing cells, each inclusive. In some embodiments, the cell therapy comprises administration of a dose comprising a number of cell from or from about 1 × 10⁵ to 5 × 10⁸ total CD3+/CAR+ or CD8+/CAR+ cells, from or from about 5 × 10⁵ to 1 × 10⁷ total CD3+/CAR+ or CD8+/CAR+ cells, or from or from about 1 × 10⁶ to 1 × 10⁷ total CD3+/CAR+ or CD8+/CAR+ cells, each inclusive.

In some embodiments, the T cells of the dose include CD4+ T cells, CD8+ T cells or CD4+ and CD8+ T cells.

In some embodiments, for example, where the subject is human, the CD8+ T cells of the dose, including in a dose including CD4+ and CD8+ T cells, includes between about 1 × 10⁶ and 5 × 10⁸ total recombinant receptor (e.g., CAR)-expressing CD8+cells, e.g., in the range of about 5 × 10⁶ to 1 × 10⁸ such cells, such cells 1 × 10⁷, 2.5 × 10⁷, 5 × 10⁷, 7.5 × 10⁷, 1 × 10⁸, or 5 × 10⁸ total such cells, or the range between any two of the foregoing values. In some embodiments, the patient is administered multiple doses, and each of the doses or the total dose can be within any of the foregoing values. In some embodiments, the dose of cells comprises the administration of from or from about 1 × 10' to 0.75 × 10⁸ total recombinant receptor-expressing CD8+ T cells, 1 × 10⁷ to 2.5 × 10⁷ total recombinant receptor-expressing CD8+ T cells, from or from about 1 × 10' to 0.75 × 10⁸ total recombinant receptor-expressing CD8+ T cells, each inclusive. In some embodiments, the dose of cells comprises the administration of or about 1 × 10', 2.5 × 10⁷, 5 × 10⁷ 7.5 × 10⁷, 1 × 10⁸, or 5 × 10⁸ total recombinant receptor-expressing CD8+ T cells.

In some embodiments, the dose of cells, e.g., recombinant receptor-expressing T cells, is administered to the subject as a single dose or is administered only one time within a period of two weeks, one month, three months, six months, 1 year or more.

In some aspects, the pharmaceutical compositions and formulations are provided as unit dose form compositions including the number of cells for administration in a given dose or fraction thereof. In some embodiments, the provided methods produce cells in a predictable timeline to dosing as compared to other methods of incubating (e.g., stimulating) cells. In some cases, the dose of cells for administration is determined based on the number of naïve-like cells in the input composition. In some embodiments, a dose comprising cells manufactured using a process comprising a shorter period of stimulation (for example, in some embodiments, an incubation time of or of about 2 days, 3 days, 4 days, 5 days, or 6 days of cells with a stimulatory agent) may be lower than a dose comprising cells manufactured in a process comprising a longer period of stimulation. In some embodiments, a dose comprising cells manufactured using a process comprising a shorter period of stimulation may comprise a greater percentage or proportion of naïve-like cells, and may comprise fewer total cells compared with a dose comprising cells manufactured using a process comprising a longer period of stimulation.

In some embodiments, cells of the dose may be administered by administration of a plurality of compositions or solutions, such as a first and a second, optionally more, each containing some cells of the dose. In some aspects, the plurality of compositions, each containing a different population and/or sub-types of cells, are administered separately or independently, optionally within a certain period of time. For example, the populations or sub-types of cells can include CD8⁺ and CD4⁺ T cells, respectively, and/or CD8+- and CD4+-enriched populations, respectively, e.g., CD4+ and/or CD8+ T cells each individually including cells genetically engineered to express the recombinant receptor. In some embodiments, the administration of the dose comprises administration of a first composition comprising a dose of CD8+ T cells or a dose of CD4+ T cells and administration of a second composition comprising the other of the dose of CD4+ T cells and the CD8+ T cells.

In some embodiments, the administration of the composition or dose, e.g., administration of the plurality of cell compositions, involves administration of the cell compositions separately. In some aspects, the separate administrations are carried out simultaneously, or sequentially, in any order. In some embodiments, the dose comprises a first composition and a second composition, and the first composition and second composition are administered 0 to 12 hours apart, 0 to 6 hours apart or 0 to 2 hours apart. In some embodiments, the initiation of administration of the first composition and the initiation of administration of the second composition are carried out no more than 2 hours, no more than 1 hour, or no more than 30 minutes apart, no more than 15 minutes, no more than 10 minutes or no more than 5 minutes apart. In some embodiments, the initiation and/or completion of administration of the first composition and the completion and/or initiation of administration of the second composition are carried out no more than 2 hours, no more than 1 hour, or no more than 30 minutes apart, no more than 15 minutes, no more than 10 minutes or no more than 5 minutes apart.

In some composition, the first composition, e.g., first composition of the dose, comprises CD4+ T cells. In some composition, the first composition, e.g., first composition of the dose, comprises CD8+ T cells. In some embodiments, the first composition is administered prior to the second composition.

In some embodiments, the dose or composition of cells includes a defined or target ratio of CD4+ cells expressing a recombinant receptor to CD8+ cells expressing a recombinant receptor and/or of CD4+ cells to CD8+ cells, which ratio optionally is approximately 1:1 or is between approximately 1:3 and approximately 3:1, such as approximately 1:1. In some aspects, the administration of a composition or dose with the target or desired ratio of different cell populations (such as CD4+:CD8+ ratio or CAR+CD4+:CAR+CD8+ ratio, e.g., 1:1) involves the administration of a cell composition containing one of the populations and then administration of a separate cell composition comprising the other of the populations, where the administration is at or approximately at the target or desired ratio. In some aspects, administration of a dose or composition of cells at a defined ratio leads to improved expansion, persistence and/or antitumor activity of the T cell therapy.

In some embodiments, the subject receives multiple doses, e.g., two or more doses or multiple consecutive doses, of the cells. In some embodiments, two doses are administered to a subject. In some embodiments, the subject receives the consecutive dose, e.g., second dose, is administered approximately 4, 5, 6, 7, 8, 9, 10, 11, 12, 13, 14, 15, 16, 17, 18, 19, 20 or 21 days after the first dose. In some embodiments, multiple consecutive doses are administered following the first dose, such that an additional dose or doses are administered following administration of the consecutive dose. In some aspects, the number of cells administered to the subject in the additional dose is the same as or similar to the first dose and/or consecutive dose. In some embodiments, the additional dose or doses are larger than prior doses.

In some aspects, the size of the first and/or consecutive dose is determined based on one or more criteria such as response of the subject to prior treatment, e.g. chemotherapy, disease burden in the subject, such as tumor load, bulk, size, or degree, extent, or type of metastasis, stage, and/or likelihood or incidence of the subject developing toxic outcomes, e.g., CRS, macrophage activation syndrome, tumor lysis syndrome, neurotoxicity, and/or a host immune response against the cells and/or recombinant receptors being administered.

In some aspects, the time between the administration of the first dose and the administration of the consecutive dose is about 9 to about 35 days, about 14 to about 28 days, or 15 to 27 days. In some embodiments, the administration of the consecutive dose is at a time point more than about 14 days after and less than about 28 days after the administration of the first dose. In some aspects, the time between the first and consecutive dose is about 21 days. In some embodiments, an additional dose or doses, e.g. consecutive doses, are administered following administration of the consecutive dose. In some aspects, the additional consecutive dose or doses are administered at least about 14 and less than about 28 days following administration of a prior dose. In some embodiments, the additional dose is administered less than about 14 days following the prior dose, for example, 4, 5, 6, 7, 8, 9, 10, 11, 12, or 13 days after the prior dose. In some embodiments, no dose is administered less than about 14 days following the prior dose and/or no dose is administered more than about 28 days after the prior dose.

In some embodiments, the dose of cells, e.g., recombinant receptor-expressing cells, comprises two doses (e.g., a double dose), comprising a first dose of the T cells and a consecutive dose of the T cells, wherein one or both of the first dose and the second dose comprises administration of the split dose of T cells.

In some embodiments, the cells are administered as part of a further combination treatment, such as simultaneously with or sequentially with, in any order, another therapeutic intervention, such as an antibody or engineered cell or receptor or agent, such as a cytotoxic or therapeutic agent. For example, in some embodiments, an anti-cancer agent or immunomodulatory agent can be used in combination therapy with adoptive cell therapy with engineered cell expressing a recombinant receptor, e.g. a CAR. In some contexts, the cells are co-administered with another therapy sufficiently close in time such that the cell populations enhance the effect of the P one or more additional therapeutic agents, or vice versa. In some embodiments, the cells are administered prior to the one or more additional therapeutic agents. In some embodiments, the cells are administered after the one or more additional therapeutic agents.

In some embodiments, the one or more additional therapeutic agents include a cytokine, such as IL-2, for example, to enhance persistence. In some embodiments, the methods comprise administration of a chemotherapeutic agent. In some embodiments, the one or more additional therapeutic agents include one or more lymphodepleting therapies, such as prior to or simultaneous with initiation of administration of the engineered cells. In some embodiments, the lymphodepleting therapy comprises administration of a phosphamide, such as cyclophosphamide. In some embodiments, the lymphodepleting therapy can include administration of fludarabine. In some embodiments, fludarabine is excluded in the lymphodepleting therapy. In some embodiments, a lymphodepleting therapy is not administered.

In some embodiments, the methods include administering a preconditioning agent, such as a lymphodepleting or chemotherapeutic agent, such as cyclophosphamide, fludarabine, or combinations thereof, to a subject prior to the initiation of the cell therapy. For example, the subject may be administered a preconditioning agent at least 2 days prior, such as at least 3, 4, 5, 6, or 7 days prior, to the initiation of the cell therapy. In some embodiments, the subject is administered a preconditioning agent no more than 7 days prior, such as no more than 6, 5, 4, 3, or 2 days prior, to the initiation of the cell therapy.

In some embodiments, the subject is preconditioned with cyclophosphamide at a dose between or between about 20 mg/kg and 100 mg/kg, such as between or between about 40 mg/kg and 80 mg/kg. In some aspects, the subject is preconditioned with or with about 60 mg/kg of cyclophosphamide. In some embodiments, the cyclophosphamide can be administered in a single dose or can be administered in a plurality of doses, such as given daily, every other day or every three days. In some embodiments, the cyclophosphamide is administered once daily for one or two days. In some embodiments, where the lymphodepleting agent comprises cyclophosphamide, the subject is administered cyclophosphamide at a dose between or between about 100 mg/m² and 500 mg/m², such as between or between about 200 mg/m² and 400 mg/m², or 250 mg/m² and 350 mg/m², inclusive. In some instances, the subject is administered about 300 mg/m² of cyclophosphamide. In some embodiments, the cyclophosphamide can be administered in a single dose or can be administered in a plurality of doses, such as given daily, every other day or every three days. In some embodiments, cyclophosphamide is administered daily, such as for 1-5 days, for example, for 3 to 5 days. In some instances, the subject is administered about 300 mg/m² of cyclophosphamide, daily for 3 days, prior to initiation of the cell therapy.

In some embodiments, where the lymphodepleting agent comprises fludarabine, the subject is administered fludarabine at a dose between or between about 1 mg/m² and 100 mg/m², such as between or between about 10 mg/m² and 75 mg/m², 15 mg/m² and 50 mg/m², 20 mg/m² and 40 mg/m², or 24 mg/m² and 35 mg/m², inclusive. In some instances, the subject is administered about 30 mg/m² of fludarabine. In some embodiments, the fludarabine can be administered in a single dose or can be administered in a plurality of doses, such as given daily, every other day or every three days. In some embodiments, fludarabine is administered daily, such as for 1-5 days, for example, for 3 to 5 days. In some instances, the subject is administered about 30 mg/m² of fludarabine, daily for 3 days, prior to initiation of the cell therapy.

In some embodiments, the lymphodepleting agent comprises a combination of agents, such as a combination of cyclophosphamide and fludarabine. Thus, the combination of agents may include cyclophosphamide at any dose or administration schedule, such as those described above, and fludarabine at any dose or administration schedule, such as those described above. For example, in some aspects, the subject is administered 60 mg/kg (~2 g/m²) of cyclophosphamide and 3 to 5 doses of 25 mg/m² fludarabine prior to the first or subsequent dose.

The cells can be administered by any suitable means. The cells are administered in a dosing regimen to achieve a therapeutic effect, such as a reduction in tumor burden. Dosing and administration may depend in part on the schedule of administration of the immunomodulatory compound, which can be administered prior to, subsequent to and/or simultaneously with initiation of administration of the T cell therapy. Various dosing schedules of the T cell therapy include but are not limited to single or multiple administrations over various time-points, bolus administration, and pulse infusion.

### IV. HITS AND ARTICLES OF MANUFACTURE

Also provided are articles of manufacture, such as kits and devices, for the administration of the cells to subjects in according to the provided methods for adoptive cell therapy, and for storage and administration of the cells and compositions, such as the input compositions or output compositions as described.

In some embodiments, a kit containing the compositions described above and instructions for use are provided. In some embodiments, provided do kits that include a composition comprising a therapeutically effective amount of any of the engineered cells stimulated according to any of the methods described herein. In some embodiments, provided are kits that include a composition comprising a therapeutically effective amount of any of the engineered cells described herein, and instructions for administering, to a subject for treating a disease or condition. In some aspects, the instructions are for selecting or enriching from a population of cells, cells expressing an antigen receptor containing an antigen binding domain specifically recognized by a stimulatory reagent. In some cases, the instructions are for expanding cells expressing an antigen receptor containing an antigen-binding domain specifically recognized by the stimulatory reagent from a population of cells.

In some embodiments, the kit further contains instructions for administering, to a subject for treating a disease or condition, the engineered cell stimulated using the methods described here, in a combined therapy for treating the disease or condition. In some examples, the kit further contains a therapeutic agent. In some embodiments, the therapeutic agent is an immunomodulatory agent, a cytotoxic agent, an anti-cancer agent or a radiotherapeutic.

Also provided herein are articles of manufacture. In some embodiments, the articles of manufacture includes any of the engineered cells, compositions, polynucleotides, set of polynucleotides, composition containing set of polynucleotides, vectors, set of vectors, composition containing set of vectors or kits provided herein.

The articles of manufacture or kits include one or more containers, typically a plurality of containers, packaging material, and a label or package insert on or associated with the container or containers and/or packaging, generally including instructions for administration of the cells to a subject. The kits and articles of manufacture may include a container and a label or package insert on or associated with the container.

In some embodiments, the containers contain the cells to be administered, e.g., one or more unit doses thereof. The article of manufacture typically includes a plurality of containers, each containing a single unit dose of the cells. The unit dose may be an amount or number of the cells to be administered to the subject in the first dose or twice the number (or more) the cells to be administered in the first or any one or more consecutive dose(s). It may be the lowest dose or lowest possible dose of the cells that would be administered to the subject in connection with the administration method. In some embodiments, the unit dose is the minimum number of cells or number of cells or the minimum number of reference units or the target reference units or reference units within a target range that would be administered in a single dose to any subject having a particular disease or condition or any subject, according to the methods herein.

Suitable containers include, for example, bottles, vials, syringes, IV solution bags, etc. The containers may be formed from a variety of materials such as glass or plastic. The container in some embodiments holds a composition which is by itself or combined with another composition effective for treating, preventing and/or diagnosing the condition. In some embodiments, the container has a sterile access port. Exemplary containers include an intravenous solution bags, vials, including those with stoppers pierceable by a needle for injection, or bottles or vials for orally administered agents. The label or package insert may indicate that the composition is used for treating a disease or condition. The article of manufacture may further include a package insert indicating that the compositions can be used to treat a particular condition. Alternatively, or additionally, the article of manufacture may further include another or the same container comprising a pharmaceutically-acceptable buffer. It may further include other materials such as other buffers, diluents, filters, needles, and/or syringes.

In particular embodiments, the containers are bags, e.g., flexible bags, such as those suitable for infusion of cells to subjects, e.g., flexible plastic or PVC bags, and/or IV solution bags. The bags in some embodiments are sealable and/or able to be sterilized, so as to provide sterile solution and delivery of the cells and compositions. In some embodiments, the containers, e.g., bags, have a capacity of at or about or at least at or about 10, 20, 30, 40, 50, 60, 70, 80, 90, 100, 200, 300, 400, 500, or 1000 mL capacity, such as between at or about 10 and at or about 100 or between at or about 10 and at or about 500 mL capacity, each inclusive. In some embodiments, the containers, e.g., bags, are and/or are made from material which is stable and/or provide stable storage and/or maintenance of cells at one or more of various temperatures, such as in cold temperatures, e.g. below at or about or at or about -20°C, -80°C, - 120°C, 135°C and/or temperatures suitable for cryopreservation, and/or other temperatures, such as temperatures suitable for thawing the cells and body temperature such as at or about 37 °C, for example, to permit thawing, e.g., at the subject's location or location of treatment, e.g., at bedside, immediately prior to treatment.

The containers may be formed from a variety of materials such as glass or plastic. In some embodiments, the container has one or more port, e.g., sterile access ports, for example, for connection of tubing or cannulation to one or more tubes, e.g., for intravenous or other infusion and/or for connection for purposes of transfer to and from other containers, such as cell culture and/or storage bags or other containers. Exemplary containers include infusion bags, intravenous solution bags, vials, including those with stoppers pierceable by a needle for injection.

The article of manufacture may further include a package insert or label with one or more pieces of identifying information and/or instructions for use. In some embodiments, the information or instructions indicates that the contents can or should be used to treat a particular condition or disease, and/or providing instructions therefor. The label or package insert may indicate that the contents of the article of manufacture are to be used for treating the disease or condition. In some embodiments, the label or package insert provides instructions to treat a subject, e.g., the subject from which the cells have been derived, via a method involving the administration of a first and one or more consecutive doses of the cells, e.g., according to any of the embodiments of the provided methods. In some embodiments, the instructions specify administration, in a first dose, of one unit dose, e.g., the contents of a single individual container in the article of manufacture, followed by one or more consecutive doses at a specified time point or within a specified time window and/or after the detection of the presence or absence or amount or degree of one or more factors or outcomes in the subject.

In some embodiments, the instructions specify administering one or more of the unit doses to the subject.

In some embodiments, the label or package insert or packaging comprises an identifier to indicate the specific identity of the subject from which the cells are derived and/or are to be administered. In the case of autologous transfer, the identity of the subject from which the cells are derived is the same as the identity of the subject to which the cells are to be administered. Thus, the identifying information may specify that the cells are to be administered to a particular patient, such as the one from which the cells were originally derived. Such information may be present in the packaging material and/or label in the form of a bar code or other coded identifier, or may indication the name and/or other identifying characteristics of the subj ect.

The article of manufacture in some embodiments includes one or more, typically a plurality, of containers containing compositions comprising the cells, e.g., individual unit dose forms thereof, and further include one or more additional containers with a composition contained therein which includes a further agent, such as a cytotoxic or otherwise therapeutic agent, for example, which is to be administered in combination, e.g., simultaneously or sequentially in any order, with the cells. Alternatively, or additionally, the article of manufacture may further include another or the same container comprising a pharmaceutically-acceptable buffer. It may further include other materials such as other buffers, diluents, filters, tubing, needles, and/or syringes.

The term "package insert" is used to refer to instructions customarily included in commercial packages of therapeutic products, that contain information about the indications, usage, dosage, administration, combination therapy, contraindications and/or warnings concerning the use of such therapeutic products.

### V. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

As used herein, a "subject" is a mammal, such as a human or other animal, and typically is human. In some embodiments, the subject, e.g., patient, to whom the immunomodulatory polypeptides, engineered cells, or compositions are administered, is a mammal, typically a primate, such as a human. In some embodiments, the primate is a monkey or an ape. The subject can be male or female and can be any suitable age, including infant, juvenile, adolescent, adult, and geriatric subjects. In some embodiments, the subject is a non-primate mammal, such as a rodent.

As used herein, "treatment" (and grammatical variations thereof such as "treat" or "treating") refers to complete or partial amelioration or reduction of a disease or condition or disorder, or a symptom, adverse effect or outcome, or phenotype associated therewith. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. The terms do not imply complete curing of a disease or complete elimination of any symptom or effect(s) on all symptoms or outcomes.

As used herein, "delaying development of a disease" means to defer, hinder, slow, retard, stabilize, suppress and/or postpone development of the disease (such as cancer). This delay can be of varying lengths of time, depending on the history of the disease and/or individual being treated. As is evident, a sufficient or significant delay can, in effect, encompass prevention, in that the individual does not develop the disease. For example, a late stage cancer, such as development of metastasis, may be delayed.

"Preventing," as used herein, includes providing prophylaxis with respect to the occurrence or recurrence of a disease in a subject that may be predisposed to the disease but has not yet been diagnosed with the disease. In some embodiments, the provided cells and compositions are used to delay development of a disease or to slow the progression of a disease.

As used herein, to "suppress" a function or activity is to reduce the function or activity when compared to otherwise same conditions except for a condition or parameter of interest, or alternatively, as compared to another condition. For example, cells that suppress tumor growth reduce the rate of growth of the tumor compared to the rate of growth of the tumor in the absence of the cells.

An "effective amount" of an agent, e.g., a pharmaceutical formulation, cells, or composition, in the context of administration, refers to an amount effective, at dosages/amounts and for periods of time necessary, to achieve a desired result, such as a therapeutic or prophylactic result.

A "therapeutically effective amount" of an agent, e.g., a pharmaceutical formulation or engineered cells, refers to an amount effective, at dosages and for periods of time necessary, to achieve a desired therapeutic result, such as for treatment of a disease, condition, or disorder, and/or pharmacokinetic or pharmacodynamic effect of the treatment. The therapeutically effective amount may vary according to factors such as the disease state, age, sex, and weight of the subject, and the immunomodulatory polypeptides or engineered cells administered. In some embodiments, the provided methods involve administering the immunomodulatory polypeptides, engineered cells, or compositions at effective amounts, e.g., therapeutically effective amounts.

A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

As used herein, recitation that nucleotides or amino acid positions "correspond to" nucleotides or amino acid positions in a disclosed sequence, such as set forth in the Sequence listing, refers to nucleotides or amino acid positions identified upon alignment with the disclosed sequence to maximize identity using a standard alignment algorithm, such as the GAP algorithm. By aligning the sequences, one can identify corresponding residues, for example, using conserved and identical amino acid residues as guides. In general, to identify corresponding positions, the sequences of amino acids are aligned so that the highest order match is obtained (see, e.g. : Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New. Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; Carrillo et al. (1988) SIAM J Applied Math 48: 1073).

As used herein, the singular forms "a," "an," and "the" include plural referents unless the context clearly dictates otherwise. For example, "a" or "an" means "at least one" or "one or more." It is understood that aspects and variations described herein include "consisting" and/or "consisting essentially of" aspects and variations.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

The term "about" as used herein refers to the usual error range for the respective value readily known to the skilled person in this technical field. Reference to "about" a value or parameter herein includes (and describes) embodiments that are directed to that value or parameter per se. For example, description referring to "about X" includes description of "X". In certain embodiments, "about" a stated value refers to a value within ±25%, ±20%, ±10%, ±5%, ±1%, ±0.1%, or ±0.01% of the stated value.

As used herein, a composition refers to any mixture of two or more products, substances, or compounds, including cells. It may be a solution, a suspension, liquid, powder, a paste, aqueous, non-aqueous or any combination thereof.

As used herein, a statement that a cell or population of cells is "positive" for a particular marker refers to the detectable presence on or in the cell of a particular marker, typically a surface marker. When referring to a surface marker, the term refers to the presence of surface expression as detected by flow cytometry, for example, by staining with an antibody that specifically binds to the marker and detecting said antibody, wherein the staining is detectable by flow cytometry at a level substantially above the staining detected carrying out the same procedure with an isotype-matched control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected by flow cytometry, for example, by staining with an antibody that specifically binds to the marker and detecting said antibody, wherein the staining is not detected by flow cytometry at a level substantially above the staining detected carrying out the same procedure with an isotype-matched control under otherwise identical conditions, and/or at a level substantially lower than that for cell known to be positive for the marker, and/or at a level substantially similar as compared to that for a cell known to be negative for the marker.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

### VI. EXEMPLARY EMBODIMENTS

Among the provided embodiments are:
1. A method for genetically engineering T cells, the method comprising:
   (a) incubating an input composition, under stimulating conditions, for between 2 and 6 days, said input composition comprising a population of T cells comprising naïve-like T cells and non-naïve-like T cells, wherein the stimulating conditions comprises a stimulatory reagent capable of activating one or more intracellular signaling domains of one or more components of a TCR complex and/or one or more intracellular signaling domains of one or more costimulatory molecules, thereby generating a stimulated composition; and
   (b) introducing a nucleic acid encoding a genetically engineered recombinant receptor into the stimulated composition of T cells, wherein the introducing is carried out during at least a portion of the incubating.
2. A method for genetically engineering T cells, the method comprising:
   (a) incubating an input composition, under stimulating conditions, for between 2 and 6 days, said input composition comprising a population of T cells comprising naïve-like T cells and non-naive-like T cells, , thereby generating a stimulated composition; and
   (b) introducing a nucleic acid encoding a genetically engineered recombinant receptor into the stimulated composition of T cells, wherein the introducing is carried out during at least a portion of the incubating.
3. The method of embodiment 2, wherein the stimulating conditions comprise incubating the input composition with a stimulatory reagent capable of activating one or more intracellular signaling domains of one or more components of a TCR complex and/or one or more intracellular signaling domains of one or more costimulatory molecules.
4. A method for genetically engineering T cells, the method comprising incubating an input composition, under stimulating conditions, for between 2 and 6 days, said input composition comprising a population of T cells comprising naïve-like T cells and non-naïve-like T cells, wherein:
   the stimulating conditions comprises a stimulatory reagent capable of activating one or more intracellular signaling domains of one or more components of a TCR complex and/or one or more intracellular signaling domains of one or more costimulatory molecules, thereby generating a stimulated composition; and
   the incubating the input composition under stimulating conditions is performed prior to, during and/or subsequent to introducing a nucleic acid encoding a genetically engineered recombinant receptor.
5. The method of any of embodiments 1-4, wherein the incubating is carried out for at least 3 days.
6. The method of any of embodiments 1-4, wherein the incubating is carried out for at least 4 days.
7. The method of any of embodiments 1-4, wherein the incubating is carried out for at least 5 days.
8. The method of any of embodiments 1-4, wherein the incubating is carried out for at least 6 days.
9. A method for stimulating T cells, the method comprising:
   (a) incubating, under stimulating conditions, an input composition comprising T cells comprising a culture-initiating amount of naïve-like T cells or a CD8+ T cell subset thereof, thereby producing a stimulated composition ; and
   (b) introducing into the stimulated cell composition a nucleic acid encoding a genetically engineered recombinant receptor, wherein the method thereby generates an output composition comprising T cells expressing the genetically engineered recombinant receptor.
10. The method of embodiment 9, wherein the T cells comprise naïve-like T cells and non-naive-like T cells, wherein the stimulating conditions preferentially induces expansion or proliferation of the naive-like T cells compared to the non-naive like T cells in the stimulated composition.
11. The method of embodiment 9 or embodiment 10, wherein the introducing is carried out during at least a portion of the incubating or is carried out subsequent to the incubating.
12. The method of any of embodiments 9-11, wherein the culture-initiating amount of naïve-like T cells or a CD8+ T cell subset thereof is from or from about 0.1 × 10⁸ to 5 × 10⁸, from or from about 0.1 × 10⁸ to 4 × 10⁸, from or from about 0.1 × 10⁸ to 2 × 10⁸, from or from about 0.1 × 10⁸ to 1 × 10⁸, from or from about 1 × 10⁸ to 5 × 10⁸ from or from about 1 × 10⁸ to 4 × 10⁸, from or from about 1 × 10⁸ to 2 × 10⁸, from or from about 2 × 10⁸ to 5 × 10⁸, from or from about 2 × 10⁸ to 4 × 10⁸ of the naïve-like T cells or a CD8+ T cell subset thereof.
13. The method of any of embodiments 9-12, wherein the culture-initiating amount of naïve-like T cells or a CD8+ T cell subset thereof is at least or at least about or is or is about 0.5 × 10⁸, 0.75 × 10⁸, 1 × 10⁸, 1.5 × 10⁸, 2 × 10⁸, or 4 × 10⁸ of the naïve-like T cells or a CD8+ T cell subset thereof.
14. The method of any of embodiments 9-13, wherein the culture-initiating amount of naïve-like T cells or a CD8+ T cell subset thereof is at least or at least about or is or is about 2 × 10⁸ of the naïve-like T cells or a CD8+ T cell subset thereof.
15. A method for stimulating T cells, the method comprising incubating, under stimulating conditions, an input composition comprising T cells comprising a culture-initiating amount of naive-like T cells or a CD8+ T cell subset thereof of from or from about 1 × 10⁸ to 4 × 10⁸ naïve-like T cells or a CD8+ T cell subset thereof, thereby producing a stimulated composition.
16. The method of embodiment 15, wherein the T cells comprise naïve-like T cells and non-naive-like T cells, wherein the stimulating conditions preferentially induces expansion or proliferation of the naive-like T cells compared to the non-naive like T cells in the stimulated composition.
17. The method of embodiment 15 or embodiment 16, wherein the culture-initiating amount of naive-like T cells or a CD8+ T cell subset thereof is at least or at least about or is or is about 2 × 10⁸ of the naive-like T cells or a CD8+ T cell subset thereof.
18. The method of any of embodiments 17-17, wherein the culture initiating amount is an amount of naïve-like CD8+ T cells.
19. The method of any of embodiments 1-18, wherein the naive-like T cells or naïve-like CD8+ T cells:
   are surface positive for a T cell activation marker selected from the group consisting of CD45RA, CD27, CD28, and CCR7; and/or
   are surface negative for a marker selected from the group consisting of CD25, CD45RO, CD56, CD62L, KLRG1; and/or
   have low expression of CD95; and/or
   are negative for intracellular expression of a cytokine selected from the group consisting of IL-2, IFN-γ, IL-4, IL-10.
20. The method of any of embodiments 1-19, wherein the naïve-like cells or the naïve-like CD8+ cells:
   are surface positive for a T cell activation marker selected from the group consisting of CD45RA, CD27, CD28, and CCR7; and/or
   are surface negative for a marker selected from the group consisting of CD45RO, CD56, KLRG1; and/or
   have low expression of CD95.
21. The method of any of embodiments 1-20, wherein the naive-like T cells or the naïve-like CD8+ cells are CD45RA+, CD27+, CCR7+, and/or CD45RO-.
22. The method of any of embodiments 1-17 and 16-21, wherein the non-naïve-like T cells:
   are surface negative for a T cell activation marker selected from the group consisting of CD45RA, CD27, CD28, and CCR7; and/or
   are surface positive for a marker selected from the group consisting of CD25, CD45RO, CD56, CD62L, KLRG1, and perforin; and/or
   are positive intracellular expression of a cytokine selected from the group consisting of IL-2, IFN-γ, IL-4, IL-10; and/or
   have high expression of CD95.
23. The method of any of embodiments 1-14 and 16-22, wherein the non-naïve-like T cells are CD45RA-, CD27-, CCR7-, and/or CD45RO+.
24. The method of any of embodiments 1-23, wherein the cells of the input composition have not been and are not, prior to the incubation, subjected to a selection step based on an endogenous T cell surface marker that differentiates between naïve-like and non-naïve-like T cells.
25. The method of any of embodiments15-24 further comprising introducing a genetically engineered recombinant receptor into the stimulated cells,
   wherein the method thereby generates an output composition comprising T cells expressing the genetically engineered recombinant receptor.
26. The method of embodiment 26, wherein incubating the composition under stimulating conditions is performed prior to, during and/or subsequent to introducing a nucleic acid encoding a genetically engineered recombinant receptor.
27. The method of any of embodiments 1-14 and 25-26, wherein the recombinant receptor is capable of binding to a target antigen that is associated with, specific to, and/or expressed on a cell or tissue of a disease, disorder or condition.
28. The method of embodiment 27, wherein the disease, disorder or condition is an infectious disease or disorder, an autoimmune disease, an inflammatory disease, or a tumor or a cancer.
29. The method of embodiment 27 or embodiment 28, wherein the target antigen is a tumor antigen.
30. The method of any of embodiments 27-29, wherein the target antigen is selected from among αvβ6 integrin (avb6 integrin),, B cell maturation antigen (BCMA), carbonic anhydrase 9 (CAIX), Her2/neu (receptor tyrosine kinase erbB2), L1-CAM, B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), and hepatitis B surface antigen, anti-folate receptor, a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), epithelial glycoprotein 2 (EPG-2), epithelial glycoprotein 40 (EPG-40), ephrinB2, ephrin receptor A2 (EPHa2), erb-B2, erb-B3, erb-B4, erbB dimers, type III epidermal growth factor receptor mutation (EGFR vIII), folate binding protein (FBP), Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glypican-3 (GPC3), G Protein Coupled Receptor 5D (GPRC5D), Her2/neu (receptor tyrosine kinase erbB2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha (IL-22R-alpha), IL-13R-alpha2 (IL-13Rα2), kinase insert domain receptor (kdr), kappa light chain, Lewis Y, L1-cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), folate receptor-a, 8H9, dual antigen, glycoprotein 100 (gp100), cular endothelial growth factor receptor (VEGFR), vascular endothelial growth factor receptor 2 (VEGF-R2), estrogen receptor, progesterone receptor, Wilms Tumor 1 (WT-1), a pathogen-specific or pathogen-expressed antigen and an antigen associated with a universal tag.
31. The method of any of embodiments 1-14 and 25-30, wherein the recombinant receptor is or comprises a functional non-TCR antigen receptor or a TCR or antigen-binding fragment thereof.
32. The method of any of embodiments 1-14 and 25-30, wherein the recombinant receptor is a chimeric antigen receptor (CAR).
33. The method of any of embodiments 1-14 and 25-32, wherein the recombinant receptor comprises an extracellular domain comprising an antigen-binding domain, optionally wherein the antigen-binding domain specifically binds the target antigen.
34. The method of embodiment 33, wherein the antigen-binding domain is or comprises an antibody or an antibody fragment thereof, which optionally is a single chain fragment.
35. The method of embodiment 33, wherein the fragment comprises antibody variable regions joined by a flexible linker.
36. The method of embodiment 34 or embodiment 35, wherein the fragment comprises an scFv.
37. The method of any of embodiments 1-14 and 25-36, wherein the recombinant receptor further comprises a spacer and/or a hinge region.
38. The method of any of embodiments 1-14 and 25-37, wherein the recombinant receptor comprises an intracellular signaling region.
39. The method of embodiment 38, wherein the intracellular signaling region comprises an intracellular signaling domain.
40. The method of embodiment 39, wherein the intracellular signaling domain is or comprises a primary signaling domain, a signaling domain that is capable of inducing a primary activation signal in a T cell, a signaling domain of a T cell receptor (TCR) component, and/or a signaling domain comprising an immunoreceptor tyrosine-based activation motif (ITAM).
41. The method of embodiment 40, wherein the intracellular signaling domain is or comprises an intracellular signaling domain of a CD3 chain, optionally a CD3-zeta (CD3ζ) chain, or a signaling portion thereof.
42. The method of any of embodiments 38-41, wherein the recombinant receptor further comprises a transmembrane domain disposed between the extracellular domain and the intracellular signaling region.
43. The method of any of embodiments 38-42, wherein the intracellular signaling region further comprises a costimulatory signaling region.
44. The method of embodiment 43, wherein the costimulatory signaling region comprises an intracellular signaling domain of a T cell costimulatory molecule or a signaling portion thereof.
45. The method of embodiment 43 or embodiment 44, wherein the costimulatory signaling region comprises an intracellular signaling domain of a CD28, a 4-1BB or an ICOS or a signaling portion thereof.
46. The method of any of embodiments 43-45, wherein the costimulatory signaling region is between the transmembrane domain and the intracellular signaling region.
47. The method of any of embodiments 1-46, wherein the stimulating condition comprises incubation with a stimulatory reagent capable of activating T cells, CD4+ T cells and/or CD8+ T cells; is capable of inducing a signal through a TCR complex; and/or is capable of inducing proliferation of T cells, CD4+ T cells and/or CD8+ T cells.
48. The method of any of embodiments 1-47, wherein the stimulating condition comprises incubation with a stimulatory reagent capable of activating one or more intracellular signaling domains of one or more components of a TCR complex and/or one or more intracellular signaling domains of one or more costimulatory molecules.
49. The method of embodiment 47 or embodiment 48, wherein the stimulatory reagent comprises a primary agent that specifically binds to a member of a TCR complex, optionally that specifically binds to CD3.
50. The method of embodiment 49, wherein the stimulatory agent further comprises a secondary agent that specifically binds to a T cell costimulatory molecule, optionally wherein the costimulatory molecule is selected from the group consisting of CD28, CD137 (4-1-BB), OX40, or ICOS.
51. The method of any of embodiments 47-50, wherein the primary and secondary agents comprise antibodies, optionally wherein the one or more stimulating agent comprises incubation with an anti-CD3 antibody and an anti-CD28 antibody.
52. The method of embodiment 50 or embodiment 51, wherein the primary and/or secondary are present on the surface of a solid support.
53. The method of embodiment 52, wherein the solid support is or comprises a bead.
54. The method of embodiment 53, wherein the bead comprises a diameter of greater than or greater than about 3.5 µm but no more than about 9 µm or no more than about 8 µm or no more than about 7 µm or no more than about 6 µm or no more than about 5 µm.
55. The method of embodiment 53 or embodiment 54, wherein the bead comprises a diameter of or about 4.5 µm.
56. The method of any of embodiments 53-55, wherein the bead comprises a diameter that is or is about the same size as a lymphocyte or an antigen presenting cell.
57. The method of any of embodiments 53-56, wherein the bead is inert.
58. The method of any of embodiments 53-57, wherein the bead is or comprises a polystyrene surface, and optionally comprises a magnetic or superparamagnetic core.
59. The method of any of embodiments 53-58, wherein the stimulating condition comprises incubating the cells with a ratio of beads to cells that is from or from about 1:1 to 10:1, from or from about 1:1 to 8:1, from or from about 1:1 to 6:1, from or from about 1:1 to 4:1, from or from about 1:1 to 3:1, from or from about 2:1 to 4:1, from or from about 2:1 to 3:1, from or from about 1:1 to 2:1, from or from about 4:1 to 10:1, from or from about 4:1 to 8:1, from or from about 4:1 to 6:1, from or from about 6:1 to 10:1, from or from about 6:1 to 8:1, from or from about 8:1 to 10:1, from or from about 1:1 to 1:10, from or from about 1:1 to 1:8, from or from about 1:1 to 1:6, from or from about 1:1 to 1:4, from or from about 1:2 to 1:3.
60. A method for genetically engineering T cells, the method comprising:
   (a) incubating an input composition, under stimulating conditions, for between 2 and 6 days, said input composition comprising a population of T cells comprising naïve-like T cells and non-naïve-like T cells, wherein the stimulating conditions comprises a stimulatory reagent comprising an anti-CD3 antibody and a secondary agent that is an anti-CD28 antibody that is attached to a bead, wherein the ratio of beads to cells during the incubating is from or from about 1:1 to 4:1; and
   (b) introducing a nucleic acid encoding a genetically engineered recombinant receptor into the stimulated composition of T cells, wherein the introducing is carried out during at least a portion of the incubating.
61. A method for genetically engineering T cells, the method comprising incubating an input composition, under stimulating conditions, for between 2 and 6 days, said input composition comprising a population of T cells comprising naïve-like T cells and non-naïve-like T cells, wherein:
   wherein the stimulating conditions comprises a stimulatory reagent comprising an anti-CD3 antibody and a secondary agent that is an anti-CD28 antibody that is attached to a bead, wherein the ratio of beads to cells during the incubating is from or from about 1:1 to 4:1; and
   the incubating the input composition under stimulating conditions is performed prior to, during and/or subsequent to introducing a nucleic acid encoding a genetically engineered recombinant receptor.
62. The method of embodiment 59, wherein the ratio of beads to cells is from or from about 3:1.
63. The method of any of claims 59, wherein the ratio of beads to cells is from or from about 1:1.
64. The method of any of embodiments 1-63, wherein the T cells are from a biological sample, optionally wherein the biological sample is from a human subject.
65. The method of embodiment 64, wherein the biological sample is or comprises a whole blood sample, a buffy coat sample, a peripheral blood mononuclear cells (PBMC) sample, an unfractionated T cell sample, a lymphocyte sample, a white blood cell sample, an apheresis product, or a leukapheresis product
66. The method of any of embodiments 1-65, wherein the T cells comprise CD4+ and/or CD8+ cells.
67. The method of any of claims 1-66, wherein the T cells comprise CD4+ and CD8+ T cells and the ratio of CD4+ to CD8+ T cells is between at or about 2:1 and at or about 1:5
68. The method of embodiment 55, wherein a ratio of the CD4+ cells to the CD8+ cells is or is about 1:1, 1:2, 2:1, 1:3, or 3:1.
69. The method of any of embodiments 1-68, wherein the naive-like T cells comprise naïve-like CD4+ T cells and/or naïve-like CD8+ T cells.
70. The method of any of embodiments 1-69, wherein the naïve-like T cells are polyclonal.
71. The method of embodiment 70, wherein the clonality of the naïve-like T cells is determined by clonal sequencing, optionally next-generation sequencing, or spectratype analysis.
72. The method of any of embodiments 1-71, wherein the presence, amount, number or percentage of naive-like T cells is detected by flow cytometry.
73. The method of any of embodiments 1-72, wherein the stimulating condition does not comprise N-acetylcysteine (NAC).
74. The method of any of embodiments 1-72, wherein the stimulating condition does not comprise IL-15 and/or IL-7.
75. The method of any of embodiments 1-72, wherein the stimulating condition results or induces death or the non-naive like T cells or a subpopulation thereof.
76. The method of any of embodiments 1-75, wherein the stimulation condition results in activation-induced cell death (AICD) of non-naive like T cells or a subpopulation thereof.
77. The method of any of embodiments 1-76, further comprising adding DNAase during the incubation and/or to the stimulated composition.
78. The method of any of embodiments 1-77, wherein the incubation is carried out for greater than or about 1, 2, 3, 4, 5, 6, 7, 8, 9, 10, 11, 12, 13, 14, 15, or 16 days.
79. The method of any of embodiments 1-78, wherein the percent of cells, in the stimulated composition, derived from the naïve-like T cells is increased greater than or greater than about 1.5-fold, 2-fold, 3-fold, 4-fold, 5-fold, 6-fold , 7-fold, 8-fold, 9-fold, 10-fold, 50-fold, 100-fold compared to the percent of naive-like cells in the input composition.
80. The method of any of embodiments 1-79, wherein the ratio, in the stimulated composition, of cells derived from the naïve-like T cells compared to cells derived from the non-naïve-like T cells is increased greater than or greater than about 1.5-fold, 2-fold, 3-fold, 4-fold, 5-fold, 6-fold , 7-fold, 8-fold, 9-fold, 10-fold, 50-fold, 100-fold compared to the ratio of the naïve-like T cells compared to non-naïve-like T cells in the input composition.
81. The method of any of embodiments 1-80, wherein the stimulated composition comprises greater than 75%, 85%, 90%, 91%, 92%, 93%, 94%, 95%, 96%, 97%, 98%, 99% of cells that are derived from naïve-like T cells of the input composition.
82. The method of any of embodiments 1-81, wherein the stimulated composition comprises less than 10% of cells derived from the non-naive like T cells.
83. The method of any of embodiments 1-82, wherein the stimulated composition comprises less than 9%, 8%, 7%, 6%, 5%, 4%, 3%, 2%, 1%, 0.5% or 0.1% cells derived from the non-naive T cells.
84. The method of any of embodiments 1-83, wherein, of the cells in the input composition, a greater percentage of the naïve-like T cells, as compared to the non-naive-like T cells, are induced to proliferate and/or become activated.
85. The method of any of embodiments 1-84, wherein a greater percentage of the T cells that were naive-like in the input composition, as compared to the percentage of the T cells that were non-naïve-like in the input composition, are dividing at day 1, 2, 3, 4, 5, 6, 7, 8, 9, or 10 following initiation of said incubation.
86. The method of any of embodiments 1-85, wherein the stimulating conditions are capable of inducing proliferation of a greater percentage of cells of a human naïve-like T cell population, as compared to human non-naïve-like T cells, at day 1, 2, 3, 4, 5, 6, 7, 8, 9, or 10 following initiation of incubation under the conditions.
87. The method of any of embodiments 1-86, wherein: (i) the non-naïve-like T cells are selected from the group consisting of effector T (T_{EFF}) cells, memory T cells, central memory T cells (T_{CM}), effector memory T (T_{EM}) cells, and combinations thereof or (ii) the non-naïve-like T cells are a plurality of T cells comprising or consisting of effector T (T_{EFF}) cells and/or memory T cells, the memory T cells optionally comprising central memory T cells (T_{CM}) and/or effector memory T (T_{EM}) cells.
88. The method of any of embodiments 1-87, wherein:
   the percentage of naïve-like T cells in the input composition is less than the percentage of engineered cells in the stimulated composition derived from naïve-like T cells in the input composition.
89. The method of any of embodiments 1-14 and 25-88, wherein a greater percentage of the cells introduced with the nucleic acid are, or are derived from the proliferation of, naïve-like T cells in the input composition, compared to non-naïve-like T cells in the input composition.
90. The method of any of embodiments 1-14 and 25-89, wherein the introduction is by transduction.
91. The method of any of embodiments 1-14 and 25-90, wherein the nucleic acid comprises a viral vector.
92. The method of embodiment 91, wherein the viral vector is a retroviral vector.
93. The method of embodiment 91 or embodiment 92, wherein the viral vector is a lentiviral vector or a gammaretroviral vector.
94. The method of any of embodiments 1-14 and 25-89, wherein the introduction is by transposition of a transposon comprising the nucleic acid molecule.
95. The method of any of embodiments 1-94 that is performed in vitro or ex vivo.
96. An output composition produced by the method of any of embodiments 1-83.
97. A pharmaceutical composition comprising the output composition of embodiment 98. The pharmaceutical composition of embodiment 97, further comprising a pharmaceutical carrier.
99. A method of treatment, comprising administering to a mammalian subject an output composition produced by the method of any of embodiments 1-95 or a pharmaceutical composition of embodiment 97 or embodiment 98.
100. The method of embodiment 99, wherein the cells are derived from the subject to which the cells are administered.

### VII. EXAMPLES

The following examples are included for illustrative purposes only and are not intended to limit the scope of the invention.

### Example 1 :Assessment of Naive-Like Markers in Engineered T cell compositions containing CAR+ T cells.

Engineered compositions of primary T cells containing T cells expressing chimeric antigen receptors (CARs) were produced by two parallel processes that utilized a stimulatory reagent composed of paramagnetic polystyrene-coated beads with attached anti-CD3 and anti-CD28 antibodies to activate T cells prior to transduction with a viral vector. In both processes, cells were engineered by lentiviral transduction to express the same anti-BCMA CAR. The CAR contained an scFv antigen-binding domain specific for BCMA, a CD28 transmembrane region, a 4-1BB costimulatory signaling region, and a CD3-zeta derived intracellular signaling domain. The processes differed in their duration and in the conditions for expansion of cells. The produced T cell compositions were assessed for cell surface markers.

In both processes, separate compositions of CD4+ and CD8+ cells were selected from isolated PBMCs from a leukapheresis sample from a human donor, and the selected cell compositions were cryofrozen. The separate compositions of CD4+ and CD8+ T cells were subsequently thawed and mixed at a ratio of 1:1 of viable CD4+ T cells to viable CD8+ T cells. Approximately 300 × 10⁶ T cells (150 × 10⁶ CD4+ and 150 × 10⁶ CD8+ T cells) of the mixed input cell composition were stimulated by incubating the cells for 18-30 hours in the presence of anti-CD3/anti-CD28 antibody conjugated beads at a 1:1 bead to cell ratio in serum free media. The media also contained recombinant IL-2, IL-7, and IL-15. Following the stimulation, the cells were washed and resuspended in the serum free media containing additives as well as recombinant IL-2, IL-7 and IL-15.

In one process (hereinafter "non-expanded"), the cells from the stimulated cell composition were then transduced with a lentiviral vector encoding the anti-BCMA CAR by spinoculation. After the spinoculation, the cells were washed and resuspended in the serum free media containing recombinant IL-2, IL-7 and IL-15. The cells of the resuspended compositions were incubated at about 37.0 °C in an incubator. At about 96 hours after initiation of the stimulation, cells were rinsed twice in the presence of a magnetic field to remove the anti CD3/anti-CD28 antibody conjugated paramagnetic beads, and formulated in a solution containing 10% DMSO. The formulated cell composition was transferred to a bag or vial and were stored at approximately -80° C.

In another process (hereinafter "expanded"), following the incubation, approximately 100 ×10⁶ viable cells from the stimulated cell composition were concentrated in the serum free media containing recombinant IL-2, IL-7 and IL-15. The cells were transduced with a lentiviral vector encoding the same anti-BCMA CAR as described above by spinoculation at approximately 1600 g for 60 minutes. After spinoculation, the cells were resuspended in the serum free media containing recombinant IL-2, IL-7, and IL-15, and incubated for about 18 to 30 hours at about 37 °C. The cells were then cultivated for expansion by transfer to a bioreactor (e.g. a rocking motion bioreactor) in about 500 mL of the exemplary serum free media containing twice the concentration of IL-2, IL-7, and IL-15 as used during the incubation and transduction steps. When a set viable cell density was achieved, perfusion was initiated, where media was replaced by semi-continuous perfusion with continual mixing. The cells were cultivated the next day in the bioreactor until a threshold cell density of about 3 ×10⁶ cells/ml was achieved, which typically occurred in a process involving 6-7 days of expansion. The anti-CD3 and anti-CD28 antibody conjugated paramagnetic beads were removed from the cell composition by exposure to a magnetic field. The cells were then collected, and formulated and cryoprotected as described above.

T cell compositions produced from expanded and non-expanded engineering processes were stained with antibodies recognizing surface markers including CD4, CD8, CCR7 and CD27 and quantified by flow cytometry. The percentage of CD4+CAR+ and CD8+CAR+ T cells positive for both CCR7 and CD27 staining are shown in **FIG. 1A****.,** **FIG. 1B** and **FIG. 1C** depict the percentage of CCR7+CD27+ cells for CD4+CAR+ T cells or CD8+CAR+, respectively, in the produced T cell compositions, compared to the percentage of CCR7+CD27+ cells in input composition prior to the incubation with the anti-CD3/anti-CD28 antibody conjugated bead stimulatory reagent (CMAT). As shown, a greater percentage of CCR7+CD27+ were observed in the T cell compositions produced from the non-expanded engineering process as compared to the expanded engineering process.

Further analysis of cells generated by the expanded process from a representative donor at various days during the process of manufacture, including at activation (AMAT), transduction (XMAT) or at various times after initiation of cultivation (inoc +2, inoc +4 or inoc +5), demonstrate expansion of cells is associated with a more differentiated phenotype as determined by a decreased percentage of CCR7+ CD27+ cells **(****FIG. 1D****).**

### Example 2 :Relationship of Naive-like Phenotype on Donor Response to CAR-Expressing T cells

Exemplary therapeutic T cell compositions containing autologous T cells expressing a chimeric antigen-receptor (CAR) specific for CD19 were generated. The anti-CD19 CAR contained an anti-CD19 scFv derived from a murine antibody (variable region derived from FMC63), an immunoglobulin-derived spacer, a transmembrane domain derived from CD28, a costimulatory region derived from 4-1BB, and a CD3-zeta intracellular signaling domain.

For generation of cell compositions for administration, autologous cells were isolated from the subjects via leukapheresis. Leukapheresis samples were subjected to a process for generation of CAR-expressing cells. The process involved washing of cells using an automated wash and immunoaffinity based selection for purification of CD4⁺ and CD8⁺ T cells, resulting in two compositions, enriched for CD8⁺ (in which a median of 99%, Inter Quartile Range (IQR) 98-100%, of cells were CD8⁺) and CD4⁺ (in which a median of 99%, IQR 99-100%, cells were CD4⁺) cells, respectively.

Cells of the enriched CD4⁺ and CD8⁺ compositions were activated with anti-CD3/anti-CD28 paramagnetic beads and then were separately subjected to lentiviral transduction with a vector encoding an anti-CD19 CAR with a 4-1BB costimulatory domain. Transduced populations then were separately incubated in the presence of stimulating reagents for cell expansion. Expanded CD8⁺ and CD4⁺ cells were formulated and cryopreserved separately and stored prior to administration. To minimize variations, between lots and/or cell compositions derived from different patients, such as those having different patient attributes, in parameters indicative of cell health, cells were held at constant volumes across lots. Cell products exhibited a tight range of viable cell concentrations (based on an assessment of cell compositions for one group of subjects, CD8⁺: median 31 × 10⁶ cells/mL, IQR 28-40 × 10⁶ cells/mL, N=38; CD4⁺: median 35 × 10⁶ cells/mL, IQR 31-40 × 10⁶, N=36).

The therapeutic CAR⁺ T cell composition described above were administered to subjects with relapsed or refractory (R/R) aggressive non-Hodgkin's lymphoma (NHL) in a clinical study. Specifically, a cohort of adult human subjects with R/R NHL, including diffuse large B-cell lymphoma (DLBCL) de novo or transformed from indolent lymphoma (NOS), high-grade B-cell lymphoma (including double/triple hit), DLBCL transformed from chronic lymphocytic leukemia (CLL) or marginal zone lymphomas (MZL), primary mediastinal large b-cell lymphoma (PMBCL), and follicular lymphoma grade 3b (FLG3B), were administered with anti-CD19 CAR-expressing T cell compositions. Outcomes were separately assessed for a core subset of subjects within the full cohort (excluding those subjects with a poor performance status (ECOG 2), DLBCL transformed from marginal zone lymphomas (MZL) and/or chronic lymphocytic leukemia (CLL, Richter's), and excluding those subjects with primary mediastinal large b-cell lymphoma (PMBCL), and follicular lymphoma grade 3b (FLG3B) (core cohort)). The core cohort included subjects with DLBCL, NOS and transformed follicular lymphoma (tFL) or high grade B-cell lymphoma (double/triple hit) or high-grade B-cell lymphoma, with MYC and BCL2 and/or BCL6 rearrangements with DLBCL histology (double/triple hit) and with Eastern Cooperative Oncology Group performance status (ECOG PS) of 0 or 1 The analysis at this time point presented in this example is based on assessment of a total of 91 subjects in the full cohort (88 (65 from the CORE cohort) assessed for response and 91 (67 from the CORE cohort) assessed for safety) that had been administered the anti-CD19 CAR-expressing cells.

The cryopreserved cell compositions containing anti-CD19 CAR-expressing cells were thawed prior to intravenous administration. The therapeutic T cell dose was administered as a defined cell composition by administering the formulated CD4⁺ CAR⁺ cell population and the formulated CD8⁺ CAR⁺ population separately administered at a target ratio of approximately 1:1. Subjects were administered a single or double dose of CAR-expressing T cells (each single dose via separate infusions of CD4⁺ CAR-expressing T cells and CD8⁺ CAR-expressing T cells, respectively) as follows: a single dose of dose level 1 (DL1) containing 5 × 10⁷ total CAR-expressing T cells, or a single dose of dose level 2 (DL2) containing 1 × 10⁸ total CAR-expressing T cells. In some cases, the subjects were administered a double dose of DL1 in which each dose was administered approximately fourteen (14) days apart, administered on day 1 and day 14, including one subject that inadvertently received two DL2 doses via the two-dose schedule, due to a dosing error. The dose level and the target numbers of T cell subsets for the administered composition at DL1 and DL2 are set forth in **Table E1.** In the core cohort, 34 subjects were administered DL1, and 27 subjects were administered DL2.

| **Table E1. Target dose level and number of T cell subsets for cell compositions containing anti-CD19 CAR T cells** | | | |
|---|---|---|---|
| **Dose level** | **Helper T cell (T_{H}) Dose (CD4⁺CAR⁺)** | **Cytotoxic T Cell (T_{C}) Dose (CD8⁺CAR⁺)** | **Total T Cell Dose (CD3⁺ CAR⁺)** |
| 1 | 25 × 10⁶ | 25 × 10⁶ | 50 × 10⁶ |
| 2 | 50 × 10⁶ | 50 × 10⁶ | 100 × 10⁶ |

**Table E2** shows the overall response and safety outcomes for the full cohort and the core cohort at the two dose levels. The objective response rate (ORR) was 74%, including 52% subjects who showed a complete response (CR). The incidence of any grade of cytokine release syndrome (CRS) was 35%, with 1% severe CRS; and the incidence of any grade of neurotoxicity (NT) was 19%, with 1% severe NT.

| **Table E2. Response and Safety After CAR⁺ Cell Administration** | | | | |
|---|---|---|---|---|
| | **FULL** | **CORE** | | |
| | **All Dose Levels** | **All Dose Levels^{a}** | **DL1** | **DL2** |
| **Best Overall Response (BOR), n^{b}** | 88 | 65 | 34 | 27 |
| ORR, % (95% CI) | 74 (63,83) | 80(68, 89) | 77 (59,89) | 82 (62, 94) |
| CR, % (95% CI) | 52 (41,63) | 55(43, 68) | 47 (30, 65) | 63 (42, 81) |
| **Safety, n^{c}** | 91 | 67 | 34 | 29 |
| Any CRS, % (95% CI) | 35 (25, 46) | 36 (24, 48) | 41 (25, 59) | 24 (10, 44) |
| sCRS(grade 3-4), % (95% CI) | 1 (0, 6) | 1 (0, 8) | 38 (0, 15) | 0 |
| Any NT, % (95% CI) | 19 (11,28) | 21 (12, 33) | 24 (11, 41) | 17 (6, 36) |
| sNT(grade 3-4), % (95% CI) | 12 (6, 21) | 15 (7, 26) | 21 (9, 38) | 7 (1, 23) |

| | | | | |
|---|---|---|---|---|
| ^{a}Four patients treated on DL1D (dose level 1, two-dose schedule) with similar outcomes. ^{b} Includes patients with event of PD, death, or 28-day restaging scans. One patient did not have restaging scans available. ^{c} Includes all subjects who have received at least one dose of conforming CAR-expressing cell product 28 days prior to data snapshot date or died. | | | | |

### A. Association between Cell Attributes of Anti-CD19 CAR-Expressing T Cells and Response

The relationship between certain phenotypic attributes of the CAR⁺ T cells in the therapeutic compositions and parameters associated with clinical response outcomes were assessed. The correlations between memory phenotype in the composition and function translated to a positive correlation between central memory subset composition and peak *in vivo* expansion of CAR⁺ cells (ρ=0.42, P=0.002), and progression-free survival (PFS) (Kaplan-Meier survival estimate, *P*=0.0164) that were observed. **FIGS. 2A-2D** show the Kaplan-Meier survival curves for subjects who were administered CAR⁺ T cell compositions, divided into groups that were administered compositions containing a frequency of CCR7⁺CD27⁺ CAR⁺ T cells among CD4⁺ CAR⁺ T cells **(****FIG. 2A** for progression free survival, **FIG. 2C** for duration of response) and among CD8⁺ CAR⁺ T cells **(****FIG. 2B** for progression free survival, **FIG. 2D** for duration of response) that is above or below a certain threshold level. Higher CCR7⁺CD27⁺ memory cells in the composition were observed to be correlated with longer progression free survival.

### Example 3: Assessment of T cell Clonality Using Sequencing and Analysis Methods

Compositions of T cells were assessed for T cell clonal abundance, using single cell sequencing of T cell receptor (TCR) pairs, before and after genetic engineering to express a chimeric antigen receptor (CAR).

Autologous T cells were isolated from the subjects via leukapheresis by immunoaffinity based selection for purification of CD4⁺ and CD8⁺ T cells, resulting in two compositions, enriched for CD8⁺ and CD4+ T cells, respectively. Cells of the enriched CD4⁺ and CD8⁺ compositions were activated with anti-CD3/anti-CD28 paramagnetic beads and then were separately subjected to lentiviral transduction with a vector encoding an anti-CD19 CAR. The anti-CD19 CAR contained an anti-CD19 scFv derived from a murine antibody (variable region derived from FMC63), an immunoglobulin-derived spacer, a transmembrane domain derived from CD28, a costimulatory region derived from 4-1BB, and a CD3-zeta intracellular signaling domain. Transduced populations then were separately incubated in the presence of stimulating reagents for cell expansion. Expanded CD8⁺ and CD4⁺ compositions containing CAR-expressing T cells were formulated and cryopreserved separately and stored.

T cell clonality of the isolated CD4+ and CD8+ T cell compositions before engineering (CMAT) and of the CD4+ and CD8+ therapeutic CAR+T cell compositions after engineering by the process described above was assessed. To assess T cell clonality, the cells were subject to single-cell αβ-paired TCR sequencing, generally as described in WO2016044227, WO2016176322 and WO2012048340. Based on barcoded single-cell sequencing of TCR genes, T cell clonality and diversity of the identified clones in a cell population were determined. In some cases, the Shannon index was used as a threshold to filter clones ("Shannon- adjusted clonality"), which preserves inter-sample relationships while eliminating sample noise. See, Chaara et al. (2018) Front Immunol 9:1038).

**FIG. 3** shows the clonality of the each sample after applying the Shannon index. As shown in **FIG. 3****,** the clonality of CD4 and CD8 cells differed among T cell compositions before and after the genetic engineering, with the engineered CAR+ T cell compositions exhibiting a reduced clonality compared to the T cells in the compositions prior to initiation of the process for engineering the cells.

CD4+ and CD8+ therapeutic CAR+T cell compositions after genetic engineering were sorted by flow cytometry expression of a factor indicative of apoptosis, such as surface staining with Annexin V (Annexin V-) or caspase 3 cleavage (indicating non-apoptotic cells), and for expression of various surface markers including CD45RA, CCR7, CD27, CD4 and CD8. The phenotype of the cells was correlated to the degree of clonality of the cells. It was observed that, in both the CD4+ and CD8+ therapeutic CAR+T cell compositions, the presence of apoptotic marker-negative cells that were CCR7⁻/CD27⁻ positively correlated highly with the clonality of the cells in the composition. Likewise, the presence of apoptotic marker-negative cells that were CCR7+ or CCR7+/CD27+ negatively correlated with the clonality of cells in each of the CD4+ and CD8+ therapeutic CAR+T cell compositions. These results are consistent with an observation that clonality of cells may be inversely correlated with less differentiated, naïve-like cells, such as determined by CCR7 and/or CD27 positivity.

The present invention is not intended to be limited in scope to the particular disclosed embodiments, which are provided, for example, to illustrate various aspects of the invention. Various modifications to the compositions and methods described will become apparent from the description and teachings herein. Such variations may be practiced without departing from the true scope and spirit of the disclosure and are intended to fall within the scope of the present disclosure.

### SEQUENCES

| **#** | **SEQUENCE** | **ANNOTATION** |
|---|---|---|
| **1** | ESKYGPPCPPCP | spacer (IgG4hinge) (aa) Homo sapiens |
| **2** | GAATCTAAGTACGGACCGCCCTGCCCCCCTTGCCCT | spacer (IgG4hinge) (nt) Homo sapiens |
| **3** | ESKYGPPCPPCPGQPREPQVYTLPPSQEEMTKNQVSLTCLVKGFYPSDIAVEWESN GQPENNYKTTPPVLDSDGSFFLYSRLTVDKSRWQEGNVFSCSVMHEALHNHYTQKS LSLSLGK | Hinge-CH3 spacer Homo sapiens |
| **4** | ESKYGPPCPPCPAPEFLGGPSVFLFPPKPKDTLMISRTPEVTCVVVDVSQEDPEVQ FNWYVDGVEVHNAKTKPREEQFNSTYRVVSVLTVLHQDWLNGKEYKCKVSNKGLPS SIEKTISKAKGQPREPQVYTLPPSQEEMTKNQVSLTCLVKGFYPSDIAVEWESNGQ PENNYKTTPPVLDSDGSFFLYSRLTVDKSRWQEGNVFSCSVMHEALHNHYTQKSLS LSLGK | Hinge-CH2-CH3 spacer Homo sapiens |
| **5** | RWPESPKAQASSVPTAQPQAEGSLAKATTAPATTRNTGRGGEEKKKEKEKEEQEER ETKTPECPSHTQPLGVYLLTPAVQDLWLRDKATFTCFVVGSDLKDAHLTWEVAGKV PTGGVEEGLLERHSNGSQSQHSRLTLPRSLWNAGTSVTCTLNHPSLPPQRLMALRE PAAQAPVKLSLNLLASSDPPEAASWLLCEVSGFSPPNILLMWLEDQREVNTSGFAP ARPPPQPGSTTFWAWSVLRVPAPPSPQPATYTCVVSHEDSRTLLNASRSLEVSYVT DH | IgD-hinge-Fc Homo sapiens |
| **6** | LEGGGEGRGSLLTCGDVEENPGPR | T2A artificial |
| **7** | MLLLVTSLLLCELPHPAFLLIPRKVCNGIGIGEFKDSLSINATNIKHFKNCTSISG DLHILPVAFRGDSFTHTPPLDPQELDILKTVKEITGFLLIQAWPENRTDLHAFENL EIIRGRTKQHGQFSLAVVSLNITSLGLRSLKEISDGDVIISGNKNLCYANTINWKK LFGTSGQKTKIISNRGENSCKATGQVCHALCSPEGCWGPEPRDCVSCRNVSRGREC VDKCNLLEGEPREFVENSECIQCHPECLPQAMNITCTGRGPDNCIQCAHYIDGPHC VKTCPAGVMGENNTLVWKYADAGHVCHLCHPNCTYGCTGPGLEGCPTNGPKIPSIA TGMVGALLLLLVVALGIGLFM | tEGFR artificial |
| **8** | FWVLVVVGGVLACYSLLVTVAFIIFWV | CD28 (amino acids 153-179 of Accession No. P10747) Homo sapiens |
| **9** | IEVMYPPPYLDNEKSNGTIIHVKGKHLCPSPLFPGPSKP FWVLVVVGGVLACYSLLVTVAFIIFWV | CD28 (amino acids 114-179 of Accession No. P10747) Homo sapiens |
| **10** | RSKRSRLLHSDYMNMTPRRPGPTRKHYQPYAPPRDFAAYRS | CD28 (amino acids 180-220 of P10747) Homo sapiens |
| **11** | RSKRSRGGHSDYMNMTPRRPGPTRKHYQPYAPPRDFAAYRS | CD28 (LL to GG) Homo sapiens |
| **12** | KRGRKKLLYIFKQPFMRPVQTTQEEDGCSCRFPEEEEGGCEL | 4-1BB (amino acids 214-255 of Q07011.1) Homo sapiens |
| **13** | RVKFSRSADAPAYQQGQNQLYNELNLGRREEYDVLDKRRGRDPEMGGKPRRKNPQE GLYNELQKDKMAEAYSEIGMKGERRRGKGHDGLYQGLSTATKDTYDALHMQALPPR | CD3 zeta Homo sapiens |
| **14** | RVKFSRSAEPPAYQQGQNQLYNELNLGRREEYDVLDKRRGRDPEMGGKPRRKNPQE GLYNELQKDKMAEAYSEIGMKGERRRGKGHDGLYQGLSTATKDTYDALHMQALPPR | CD3 zeta Homo sapiens |
| **15** | RVKFSRSADAPAYKQGQNQLYNELNLGRREEYDVLDKRRGRDPEMGGKPRRKNPQE GLYNELQKDKMAEAYSEIGMKGERRRGKGHDGLYQGLSTATKDTYDALHMQALPPR | CD3 zeta Homo sapiens |
| **16** | RKVCNGIGIGEFKDSLSINATNIKHFKNCTSISGDLHILPVAFRGDSFTHTPPLDP QELDILKTVKEITGFLLIQAWPENRTDLHAFENLEIIRGRTKQHGQFSLAVVSLNI | tEGFR artificial |
| | TSLGLRSLKEISDGDVIISGNKNLCYANTINWKKLFGTSGQKTKIISNRGENSCKA TGQVCHALCSPEGCWGPEPRDCVSCRNVSRGRECVDKCNLLEGEPREFVENSECIQ CHPECLPQAMNITCTGRGPDNCIQCAHYIDGPHCVKTCPAGVMGENNTLVWKYADA GHVCHLCHPNCTYGCTGPGLEGCPTNGPKIPSIATGMVGALLLLLVVALGIGLFM | |
| **17** | EGRGSLLTCGDVEENPGP | T2A artificial |
| **18** | MALPVTALLLPLALLLHA | CD8 alpha signal peptide |
| **19** | GSGATNFSLLKQAGDVEENPGP | P2A |
| **20** | ATNFSLLKQAGDVEENPGP | P2A |
| **21** | QCTNYALLKLAGDVESNPGP | E2A |
| **22** | VKQTLNFDLLKLAGDVESNPGP | F2A |
| **23** | PGGG-(SGGGG)5-P- wherein P is proline, G is glycine and S is serine | linker |
| **24** | GSADDAKKDAAKKDGKS | linker |
| **25** | atgcttctcctggtgacaagccttctgctctgtgagttaccacacccagcattcct cctgatccca | GMCSFR alpha chain signal sequence |
| **26** | MLLLVTSLLLCELPHPAFLLIP | GMCSFR alpha chain signal sequence |
| **27** | Glu Arg Lys Cys Cys Val Glu Cys Pro Pro Cys Pro | Hinge |
| **28** | ELKTPLGDTHTCPRCPEPKSCDTPPPCPRCPEPKSCDTPPPCPRCPEPKSC DTPPPCPRCP | Hinge |
| **29** | Glu Ser Lys Tyr Gly Pro Pro Cys Pro Ser Cys Pro | Hinge |
| **30** | Glu Ser Lys Tyr Gly Pro Pro Cys Pro Pro Cys Pro | Hinge |
| **31** | Tyr Gly Pro Pro Cys Pro Pro Cys Pro | Hinge |
| **32** | Lys Tyr Gly Pro Pro Cys Pro Pro Cys Pro | Hinge |
| **33** | Glu Val Val Val Lys Tyr Gly Pro Pro Cys Pro Pro Cys Pro | Hinge |
| **34** | QQGNTLPYT | FMC63 LC-CDR3 |
| **35** | RASQDISKYLN | FMC63 CDR L1 |
| **36** | SRLHSGV | FMC63 CDR L2 |
| **37** | GNTLPYTFG | FMC63 CDR L3 |
| **38** | DYGVS | FMC63 CDR H1 |
| **39** | VIWGSETTYYNSALKS | FMC63 CDR H2 |
| **40** | YAMDYWG | FMC63 CDRH3 |
| **41** | EVKLQESGPGLVAPSQSLSVTCTVSGVSLPDYGVSWIRQPPRKGLEWLGVIWGSET TYYNSALKSRLTIIKDNSKSQVFLKMNSLQTDDTAIYYCAKHYYYGGSYAMDYWGQ GTSVTVSS | FMC63 VH |
| **42** | DIQMTQTTSSLSASLGDRVTISCRASQDISKYLNWYQQKPDGTVKLLIYHTSRLHS GVPSRFSGSGSGTDYSLTISNLEQEDIATYFCQQGNTLPYTFGGGTKLEIT | FMC63 VL |
| **43** | DIQMTQTTSSLSASLGDRVTISCRASQDISKYLNWYQQKPDGTVKLLIYHTSRLHS GVPSRFSGSGSGTDYSLTISNLEQEDIATYFCQQGNTLPYTFGGGTKLEITGSTSG SGKPGSGEGSTKGEVKLQESGPGLVAPSQSLSVTCTVSGVSLPDYGVSWIRQPPRK GLEWLGVIWGSETTYYNSALKSRLTIIKDNSKSQVFLKMNSLQTDDTAIYYCAKHY YYGGSYAMDYWGQGTSVTVSS | FMC63 scFv |
| **44** | KASQNVGTNVA | SJ25C1 CDR L1 |
| **45** | SATYRNS | SJ25C1 CDR L2 |
| **46** | QQYNRYPYT | SJ25C1 CDR L3 |
| **47** | SYWMN | SJ25C1 CDR H1 |
| **48** | QIYPGDGDTNYNGKFKG | SJ25C1 CDR H2 |
| **49** | KTISSWDFYFDY | SJ25C1 CDR H3 |
| **50** | EVKLQQSGAELVRPGSSVKISCKASGYAFSSYWMNWVKQRPGQGLEWIGQIYPGDG DTNYNGKFKGQATLTADKSSSTAYMQLSGLTSEDSAVYFCARKTISSVVDFYFDYW | SJ25C1 VH |
| | GQGTTVTVSS | |
| **51** | DIELTQSPKFMSTSVGDRVSVTCKASQNVGTNVAWYQQKPGQSPKPLIYSATYRNS GVPDRFTGSGSGTDFTLTITNVQSKDLADYFCQQYNRYPYTSGGGTKLEIKR | SJ25C1 VL |
| **52** | GGGGSGGGGSGGGGS | Linker |
| **53** | EVKLQQSGAELVRPGSSVKISCKASGYAFSSYWMNWVKQRPGQGLEWIGQIYPGDG DTNYNGKFKGQATLTADKSSSTAYMQLSGLTSEDSAVYFCARKTISSVVDFYFDYW GQGTTVTVSSGGGGSGGGGSGGGGSDIELTQSPKFMSTSVGDRVSVTCKASQNVGT NVAWYQQKPGQSPKPLIYSATYRNSGVPDRFTGSGSGTDFTLTITNVQSKDLADYF CQQYNRYPYTSGGGTKLEIKR | SJ25C1 scFv |
| **54** | HYYYGGSYAMDY | FMC63 HC-CDR3 |
| **55** | HTSRLHS | FMC63 LC-CDR2 |
| **56** | GSTSGSGKPGSGEGSTKG | Linker |
| **57** | gacatccagatgacccagaccacctccagcctgagcgccagcctgggcgaccgggt gaccatcagctgccgggccagccaggacatcagcaagtacctgaactggtatcagc agaagcccgacggcaccgtcaagctgctgatctaccacaccagccggctgcacagc ggcgtgcccagccggtttagcggcagcggctccggcaccgactacagcctgaccat ctccaacctggaacaggaagatatcgccacctacttttgccagcagggcaacacac tgccctacacctttggcggcggaacaaagctggaaatcaccggcagcacctccggc agcggcaagcctggcagcggcgagggcagcaccaagggcgaggtgaagctgcagga aagcggccctggcctggtggcccccagccagagcctgagcgtgacctgcaccgtga gcggcgtgagcctgcccgactacggcgtgagctggatccggcagccccccaggaag ggcctggaatggctgggcgtgatctggggcagcgagaccacctactacaacagcgc cctgaagagccggctgaccatcatcaaggacaacagcaagagccaggtgttcctga agatgaacagcctgcagaccgacgacaccgccatctactactgcgccaagcactac tactacggcggcagctacgccatggactactggggccagggcaccagcgtgaccgt gagcagc | Sequence encoding scFv |
| 58 | X1PPX2P | Hinge |
| | X1 is glycine, cysteine or arginine | |
| | X2 is cysteine or threonine | |
| 59 | Glu Pro Lys Ser Cys Asp Lys Thr His Thr Cys Pro Pro Cys Pro | Hinge |

### Numbered embodiments:

1. A method for genetically engineering T cells, the method comprising:
   (a) incubating an input composition, under stimulating conditions, for between 2 and 6 days, said input composition comprising a population of T cells comprising naive-like T cells and non-naive-like T cells, wherein the stimulating conditions comprises a stimulatory reagent capable of activating one or more intracellular signaling domains of one or more components of a TCR complex and/or one or more intracellular signaling domains of one or more costimulatory molecules, thereby generating a stimulated composition; and
   (b) introducing a nucleic acid encoding a genetically engineered recombinant receptor into the stimulated composition of T cells, wherein the introducing is carried out during at least a portion of the incubating.
2. A method for genetically engineering T cells, the method comprising incubating an input composition, under stimulating conditions, for between 2 and 6 days, said input composition comprising a population of T cells comprising naive-like T cells and non-naive-like T cells, wherein:
   the stimulating conditions comprises the presence of a stimulatory reagent capable of activating one or more intracellular signaling domains of one or more components of a TCR complex and/or one or more intracellular signaling domains of one or more costimulatory molecules, thereby generating a stimulated composition; and
   the incubating the input composition under stimulating conditions is performed prior to, during and/or subsequent to introducing a nucleic acid encoding a genetically engineered recombinant receptor.
3. The method of embodiment 1 or embodiment 2, wherein the incubating is carried out for at least 3 days.
4. The method of embodiment 1 or embodiment 2, wherein the incubating is carried out for at least 4 days.
5. The method of embodiment 1 or embodiment 2, wherein the incubating is carried out for at least 5 days.
6. The method of embodiment 1 or embodiment 2, wherein the incubating is carried out for at least 6 days.
7. A method for stimulating T cells, the method comprising:
   (a) incubating, under stimulating conditions, an input composition comprising T cells comprising a culture-initiating amount of naive-like T cells or a CD8+ T cell subset thereof, thereby producing a stimulated composition, wherein the stimulating conditions comprise the presence of a stimulatory reagent capable of activating one or more intracellular signaling domains of one or more components of a TCR complex and/or one or more intracellular signaling domains of one or more costimulatory molecules, thereby generating a stimulated composition; and
   (b) introducing into the stimulated cell composition a nucleic acid encoding a genetically engineered recombinant receptor, wherein the method thereby generates an output composition comprising T cells expressing the genetically engineered recombinant receptor.
8. The method of embodiment 7, wherein the T cells comprise naive-like T cells and non-naive-like T cells, wherein the stimulating conditions preferentially induces expansion or proliferation of the naive-like T cells compared to the non-naive like T cells in the stimulated composition.
9. The method of embodiment 7 or embodiment 8, wherein the introducing is carried out during at least a portion of the incubating or is carried out subsequent to the incubating.
10. The method of any of embodiments 7-9, wherein the culture-initiating amount of naive-like T cells or a CD8+ T cell subset thereof is from or from about 0.1 × 10⁸ to 5 × 10⁸, from or from about 0.1 × 10⁸ to 4 × 10⁸, from or from about 0.1 × 10⁸ to 2 × 10⁸, from or from about 0.1 × 10⁸ to 1 × 10⁸, from or from about 1 × 10⁸ to 5 × 10⁸ from or from about 1 × 10⁸ to 4 × 10⁸, from or from about 1 × 10⁸ to 2 × 10⁸, from or from about 2 × 10⁸ to 5 × 10⁸, from or from about 2 × 10⁸ to 4 × 10⁸of the naive-like T cells or a CD8+ T cell subset thereof.
11. The method of any of embodiments 7-10, wherein the culture-initiating amount of naive-like T cells or a CD8+ T cell subset thereof is at least or at least about or is or is about 0.5 × 10⁸, 0.75 × 10⁸,1 × 10⁸, 1.5 × 10⁸, 2 × 10⁸, or 4 × 10⁸ of the naive-like T cells or a CD8+ T cell subset thereof
12. The method of any of embodiments 7-11, wherein the culture-initiating amount of naive-like T cells or a CD8+ T cell subset thereof is at least or at least about or is or is about 2 × 10⁸ of the naive-like T cells or a CD8+ T cell subset thereof
13. A method for stimulating T cells, the method comprising incubating, under stimulating conditions, an input composition comprising T cells comprising a culture-initiating amount of naive-like T cells or a CD8+ T cell subset thereof of from or from about 1 × 10⁸ to 4 × 10⁸ naive-like T cells or a CD8+ T cell subset thereof, thereby producing a stimulated composition, wherein the stimulating conditions comprises the presence of a stimulatory reagent capable of activating one or more intracellular signaling domains of one or more components of a TCR complex and/or one or more intracellular signaling domains of one or more costimulatory molecules, thereby generating a stimulated composition.
14. The method of embodiment 13, wherein the T cells comprise naive-like T cells and non-naive-like T cells, wherein the stimulating conditions preferentially induces expansion or proliferation of the naive-like T cells compared to the non-naive like T cells in the stimulated composition.
15. The method of embodiment 13 or embodiment 14, wherein the culture-initiating amount of naive-like T cells or a CD8+ T cell subset thereof is at least or at least about or is or is about 2 × 10⁸ of the naive-like T cells or a CD8+ T cell subset thereof
16. The method of any of embodiments 7-15, wherein the culture initiating amount is an amount of naive-like CD8+ T cells.
17. The method of any of embodiments 1-16, wherein the naive-like T cells or naive-like CD8+ T cells:
   are surface positive for a T cell activation marker selected from the group consisting of CD45RA, CD27, CD28, and CCR7; and/or
   are surface negative for a marker selected from the group consisting of CD25, CD45RO, CD56, CD62L, KLRG1; and/or
   have low expression of CD95; and/or
   are negative for intracellular expression of a cytokine selected from the group consisting of IL-2, IFN-γ, IL-4, IL-10.
18. The method of any of embodiments 1-16, wherein the naive-like cells or the naive-like CD8+ cells:
   are surface positive for a T cell activation marker selected from the group consisting of CD45RA, CD27, CD28, and CCR7; and/or
   are surface negative for a marker selected from the group consisting of CD45RO, CD56, KLRG1; and/or
   have low expression of CD95.
19. The method of any of embodiments 1-18, wherein the naive-like T cells or the naive-like CD8+ cells are CD45RA+, CD27+, CCR7+, and/or CD45RO-.
20. The method of any of embodiments 1-7, 8-12 and 14-19, wherein the non-naive-like T cells:
   are surface negative for a T cell activation marker selected from the group consisting of CD45RA, CD27, CD28, and CCR7; and/or
   are surface positive for a marker selected from the group consisting of CD25, CD45RO, CD56, CD62L, KLRG1, and perforin; and/or
   are positive intracellular expression of a cytokine selected from the group consisting of IL-2, IFN-γ, IL-4, IL-10; and/or
   have high expression of CD95.
21. The method of any of embodiments 1-7, 8-12 and 14-20, wherein the non-naive-like T cells are CD45RA-, CD27-, CCR7-, and/or CD45RO+.
22. The method of any of embodiments 1-21, wherein the cells of the input composition have not been and are not, prior to the incubation, subjected to a selection step based on an endogenous T cell surface marker that differentiates between naive-like and non-naive-like T cells.
23. The method of any of embodiments 13-22, further comprising introducing a genetically engineered recombinant receptor into the stimulated cells,
   wherein the method thereby generates an output composition comprising T cells expressing the genetically engineered recombinant receptor.
24. The method of embodiment 23, wherein incubating the composition under stimulating conditions is performed prior to, during and/or subsequent to introducing a nucleic acid encoding a genetically engineered recombinant receptor.
25. The method of any of embodiments 1-12 and 23-24, wherein the recombinant receptor is capable of binding to a target antigen that is associated with, specific to, and/or expressed on a cell or tissue of a disease, disorder or condition.
26. The method of embodiment 25, wherein the disease, disorder or condition is an infectious disease or disorder, an autoimmune disease, an inflammatory disease, or a tumor or a cancer.
27. The method of embodiment 25 or embodiment 26, wherein the target antigen is a tumor antigen.
28. The method of any of embodiments 25-27, wherein the target antigen is selected from among ανβ6 integrin (avb6 integrin),, B cell maturation antigen (BCMA), carbonic anhydrase 9 (CAIX), Her2/neu (receptor tyrosine kinase erbB2), L1-CAM, B7-H3, B7-H6, carbonic anhydrase 9 (CA9, also known as CAIX or G250), a cancer-testis antigen, cancer/testis antisen
   1B (CTAG, also known as NY-ESO-1 and LAGE-2), carcinoembryonic antigen (CEA), and hepatitis B surface antigen, anti-folate receptor, a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), epithelial glycoprotein 2 (EPG-2), epithelial glycoprotein 40 (EPG-40), ephrinB2, ephrin receptor A2 (EPHa2), erb-B2, erb-B3, erb-B4, erbB dimers, type III epidermal growth factor receptor mutation (EGFR vIII), folate binding protein (FBP), Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glypican-3 (GPC3), G Protein Coupled Receptor 5D (GPRC5D), Her2/neu (receptor tyrosine kinase erbB2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha (IL-22R-alpha), IL-13R-alpha2 (IL-13Rα2), kinase insert domain receptor (kdr), kappa light chain, Lewis Y, L1-cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), folate receptor-a, 8H9, dual antigen, glycoprotein 100 (gp100), cular endothelial growth factor receptor (VEGFR), vascular endothelial growth factor receptor 2 (VEGF-R2), estrogen receptor, progesterone receptor, Wilms Tumor 1 (WT-1), a pathogen-specific or pathogen-expressed antigen and an antigen associated with a universal tag.
29. The method of any of embodiments 1-12 and 23-28, wherein the recombinant receptor is or comprises a functional non-TCR antigen receptor or a TCR or antigen-binding fragment thereof.
30. The method of any of emodiments 1-12 and 23-29, wherein the recombinant receptor is a chimeric antigen receptor (CAR).
31. The method of any of embodiments 1-12 and 23-30, wherein the recombinant receptor comprises an extracellular domain comprising an antigen-binding domain that specifically binds a target antigen and an intracellular signaling domain comprising an ITAM.
32. The method of embodiment 31, wherein the antigen-binding domain is or comprises an antibody or an antibody fragment thereof, which optionally is a single chain fragment.
33. The method of embodiment 31 or embodiment 32, wherein the intracellular signaling domain is or comprises an intracellular signaling domain of a CD3 chain, optionally a CD3-zeta (CD3ζ) chain, or a signaling portion thereof.
34. The method of any of embodiments 31-33, wherein the intracellular signaling region further comprises a costimulatory signaling region.
35. The method of embodiment 34, wherein the costimulatory signaling region comprises an intracellular signaling domain of a T cell costimulatory molecule or a signaling portion thereof.
36. The method of embodiment 34 or embodiment 35, wherein the costimulatory signaling region comprises an intracellular signaling domain of a CD28, a 4-1BB or an ICOS or a signaling portion thereof.
37. The method of any of embodiments 30-36, wherein:
   the CAR comprises an scFv specific for the antigen, a transmembrane domain, a cytoplasmic signaling domain derived from a costimulatory molecule, which optionally is or comprises a 4-1BB, and a cvtoolasmic signaling domain derived from a primary signaling ITAM-containing molecule, which optionally is or comprises a CD3zeta signaling domain and optionally further comprises a spacer between the transmembrane domain and the scFv;
   the CAR comprises, in order, an scFv specific for the antigen, a transmembrane domain, a cytoplasmic signaling domain derived from a costimulatory molecule, which optionally is or comprises a 4-1BB signaling domain, and a cytoplasmic signaling domain derived from a primary signaling ITAM-containing molecule, which optionally is a CD3zeta signaling domain; or
   the CAR comprises, in order, an scFv specific for the antigen, a spacer, a transmembrane domain, a cytoplasmic signaling domain derived from a costimulatory molecule, which optionally is a 4-1BB signaling domain, and a cytoplasmic signaling domain derived from a primary signaling ITAM-containing molecule, which optionally is or comprises a CD3zeta signaling domain.
38. The method of any of embodiments 1-37, wherein the stimulatory reagent comprises a primary agent that specifically binds to a member of a TCR complex.
39. The method of any of embodiments 1-38, wherein the stimulatory reagent comprises a primary reagent that specifically binds to CD3.
40. The method of embodiment 38 or embodiment 39, wherein the primary agent is an antibody or an antigen-binding fragment.
41. The method of embodiment 38 or embodiment 39, wherein the stimulatory agent further comprises a secondary agent that specifically binds to a T cell costimulatory molecule, optionally wherein the costimulatory molecule is selected from the group consisting of CD28, CD137 (4-1-BB), OX40, or ICOS.
42. The method of embodiment 41, wherein the primary agent is an antibody or an antigen-binding fragment.
43. The method of any of embodiments 1-40, wherein the stimulatory reagent comprises a primary agent that is an anti-CD3 antibody and a secondary agent that is an anti-CD28 antibody.
44. The method of any of embodiments 38-43, wherein the primary and/or secondary are present on the surface of a solid support.
45. The method of embodiment 44, wherein the solid support is or comprises a bead.
46. The method of embodiment 45, wherein the bead comprises a diameter of greater than
   or greater than about 3.5 µm but no more than about 9 µm or no more than about 8 µm or no more than about 7 µm or no more than about 6 µm or no more than about 5 µm.
47. The method of embodiment 45 or embodiments 46, wherein the bead comprises a diameter of or about 4.5 µm.
48. The method of any of embodiments 45-47, wherein the bead comprises a diameter that is or is about the same size as a lymphocyte or an antigen presenting cell.
49. The method of any of embodiments 45-48, wherein the bead is inert.
50. The method of any of embodiments 45-49, wherein the bead is or comprises a polystyrene surface.
51. The method of any of embodiments 45-50, wherein the bead comprises a magnetic or superparamagnetic core.
52. The method of any of embodiments 45-51, wherein the stimulating condition comprises incubating the cells with a ratio of beads to cells that is from or from about 1:1 to 10:1, from or from about 1:1 to 8:1, from or from about 1:1 to 6:1, from or from about 1:1 to 4:1, from or from about 1:1 to 3:1, from or from about 2:1 to 4:1, from or from about 2:1 to 3:1, from or from about 1:1 to 2:1, from or from about 4:1 to 10:1, from or from about 4:1 to 8:1, from or from about 4:1 to 6:1, from or from about 6:1 to 10:1, from or from about 6:1 to 8:1, from or from about 8:1 to 10:1, from or from about 1:1 to 1:10, from or from about 1:1 to 1:8, from or from about 1:1 to 1:6, from or from about 1:1 to 1:4, from or from about 1:2 to 1:3.
53. A method for genetically engineering T cells, the method comprising:
   (a) incubating an input composition, under stimulating conditions, for between 2 and 6 days, said input composition comprising a population of T cells comprising naive-like T cells and non-naive-like T cells, wherein the stimulating conditions comprises a stimulatory reagent comprising an anti-CD3 antibody and a secondary agent that is an anti-CD28 antibody that is attached to a bead, wherein the ratio of beads to cells during the incubating is from or from about 1:1 to 4:1; and
   (b) introducing a nucleic acid encoding a genetically engineered recombinant receptor into the stimulated composition of T cells, wherein the introducing is carried out during at least a portion of the incubating.
54. A method for genetically engineering T cells, the method comprising incubating an input composition, under stimulating conditions, for between 2 and 6 days, said input composition comprising a population of T cells comprising naïve-like T cells and non-naive-like T cells, wherein:
   the stimulating conditions comprises a stimulatory reagent comprising an anti-CD3 antibody and a secondary agent that is an anti-CD28 antibody that is attached to a bead, wherein the ratio of beads to cells during the incubating is from or from about 1:1 to 4:1; and
   the incubating the input composition under stimulating conditions is performed prior to, during and/or subsequent to introducing a nucleic acid encoding a genetically engineered recombinant receptor.
55. The method of any of embodiments 52-54, wherein the ratio of beads to cells is from or from about 3:1.
56. The method of any of embodiments 52-54, wherein the ratio of beads to cells is from or from about 1:1.
57. The method of any of embodiments 1-56, wherein the T cells are from a biological sample, optionally wherein the biological sample is from a human subject.
58. The method of embodiment 57, wherein the biological sample is or comprises a whole blood sample, a buffy coat sample, a peripheral blood mononuclear cells (PBMC) sample, an unfractionated T cell sample, a lymphocyte sample, a white blood cell sample, an apheresis product, or a leukapheresis product
59. The method of any of embodiments 1-58, wherein the T cells comprise CD4+ and/or CD8+ cells.
60. The method of any of embodiments 1-59, wherein the T cells comprise CD4+ and CD8+ T cells and the ratio of CD4+ to CD8+ T cells is between at or about 2:1 and at or about 1:5.
61. The method of any of embodiments 1-60, wherein the T cells comprise CD4+ and CD8+ T cells and the ratio of the CD4+ cells to the CD8+ cells is or is about 1:1, 1:2, 2:1, 1:3, or 3:1.
62. The method of any of embodiments 1-61, wherein the naive-like T cells comprise naive-like CD4+ T cells and/or naive-like CD8+ T cells.
63. The method of any of embodiments 1-62, wherein the naive-like T cells are polyclonal.
64. The method of any of embodiments 1-63, wherein the stimulating condition comprise N-acetylcysteine (NAC).
   does not
65. The method of any of embodiments 1-63, wherein the stimulating condition does not comprise IL-15 and/or IL-7.
66. The method of any of embodiments 1-65, wherein the stimulating condition results in or induces death or the non-naive like T cells or a subpopulation thereof.
67. The method of any of embodiments 1-66, wherein the stimulation condition results in activation-induced cell death (AICD) of non-naive like T cells or a subpopulation thereof.
68. The method of any of embodiments 1-67, further comprising adding DNAase during the incubation and/or to the stimulated composition.
69. The method of any of embodiments 7-65, wherein the incubation is carried out for greater than or about 1, 2, 3, 4, 5, 6, 7, 8, 9, 10, 11, 12, 13, 14, 15, or 16 days.
70. The method of any of embodiments 1-69, wherein the percent of cells, in the stimulated composition, derived from the naïve-like T cells is increased greater than or greater than about 1.5-fold, 2-fold, 3-fold, 4-fold, 5-fold, 6-fold , 7-fold, 8-fold, 9-fold, 10-fold, 50-fold, 100-fold compared to the percent of naïve-like cells in the input composition.
71. The method of any of embodiments 1-70, wherein the ratio, in the stimulated composition, of cells derived from the naïve-like T cells compared to cells derived from the non-naïve-like T cells is increased greater than or greater than about 1.5-fold, 2-fold, 3-fold, 4-fold, 5-fold, 6-fold , 7-fold, 8-fold, 9-fold, 10-fold, 50-fold, 100-fold compared to the ratio of the naïve-like T cells compared to non-naïve-like T cells in the input composition.
72. The method of any of embodiments 1-71, wherein the stimulated composition comprises greater than 75%, 85%, 90%, 91%, 92%, 93%, 94%, 95%, 96%, 97%, 98%, 99% of cells that are derived from naïve-like T cells of the input composition.
73. The method of any of embodiments 1-72, wherein the stimulated composition comprises less than 10% of cells derived from the non-naïve like T cells.
74. The method of any of embodiments 1-73, wherein the stimulated composition comprises less than 9%, 8%, 7%, 6%, 5%, 4%, 3%, 2%, 1%, 0.5% or 0.1% cells derived from the non-naïve T cells.
75. The method of any of embodiments 1-74, wherein, of the cells in the input composition, a greater percentage of the naïve-like T cells, as compared to the non-naive-like T cells, are induced to proliferate and/or become activated.
76. The method of any of embodiments 1-75, wherein a greater percentage of the T cells that were naive-like in the input composition, as compared to the percentage of the T cells that were non-naive-like in the input composition, are dividing at day 1, 2, 3, 4, 5, 6, 7, 8, 9, or 10 following initiation of said incubation.
77. The method of any of embodiments 1-76, wherein the stimulating conditions are capable of inducing proliferation of a greater percentage of cells of a human naive-like T cell population, as compared to human non-naive-like T cells, at day 1, 2, 3, 4, 5, 6, 7, 8, 9, or 10 following initiation of incubation under the conditions.
78. The method of any of embodiments 1-77, wherein: (i) the non-naive-like T cells are selected from the group consisting of effector T (T_{EFF}) cells, memory T cells, central memory T cells (T_{CM}), effector memory T (T_{EM}) cells, and combinations thereof or (ii) the non-naive-like T cells are a plurality of T cells comprising or consisting of effector T (T_{EFF}) cells and/or memory T cells, the memory T cells optionally comprising central memory T cells (T_{CM}) and/or effector memory T (T_{EM}) cells.
79. The method of any of embodiments 1-78, wherein:
   the percentage of naive-like T cells in the input composition is less than the percentage of engineered cells in the stimulated composition derived from naive-like T cells in the input composition.
80. The method of any of embodiments 1-12 and 23-79, wherein a greater percentage of the cells introduced with the nucleic acid are, or are derived from the proliferation of, naive-like T cells in the input composition, compared to non-naive-like T cells in the input composition.
81. The method of any of embodiments 1-12 and 23-80, wherein the introduction is by transduction with a viral vector comprising a nucleic acid encoding the recombinant receptor.
82. The method of embodiment 81, wherein the viral vector is a retroviral vector.
83. The method of embodiment 81 or embodiment 82, wherein the viral vector is a lentiviral vector or a gammaretroviral vector.
84. The method of embodiment 1-83, wherein the ratio of naïve-like T cells compared to non-naïve-like T cells in the stimulated composition is increased greater than or greater than about 1.5-fold, 2-fold, 3-fold, 4-fold, 5-fold, 6-fold, 7-fold, 8-fold, 9-fold, 10-fold, 50-fold, 100-fold compared to the ratio of the naïve-like T cells compared to non-naïve-like T cells in the input composition.
85. The method of any of embodiments 1-84, wherein the stimulated composition is more polyclonal or multiclonal compared to the input composition.
86. The method of any of embodiments 1-85 that is performed in vitro or ex vivo.
87. An output composition produced by the method of any of embodiments 1-86.
88. A pharmaceutical composition comprising the output composition of embodiment 87.
89. The pharmaceutical composition of embodiment 88, further comprising a pharmaceutical carrier.
90. A method of treatment, comprising administering to a mammalian subject an output composition produced by the method of any of embodiments 1-6 or a pharmaceutical composition of embodiment 88 or embodiment 89.
91. The method of embodiment 90, wherein the cells are derived from the subject to which the cells are administered.

## Claims

1. A method for genetically engineering T cells, the method comprising:
(a) incubating an input composition, under stimulating conditions, for between 2 and 6 days to generate a stimulated composition, wherein:
the stimulating conditions comprises a stimulatory reagent capable of activating one or more intracellular signaling domains of one or more components of a TCR complex and/or one or more intracellular signaling domains of one or more costimulatory molecules, and wherein the stimulatory reagent comprises a primary agent that is an anti-CD3 antibody and a secondary agent that is an anti-CD28 antibody present on the surface of a bead, wherein the cells are incubated at a ratio of beads to cells from 1:1 to 10:1;
the input composition comprises a population of T cells comprising naïve-like T cells and non-naïve-like T cells, wherein the input composition contains a culture-initiating amount of the naïve-like T cells or a CD8+ T cell subset thereof is at least 2 x 10⁸ cells , and wherein the method does not comprise positive selection based on a marker that is characteristic of naïve-like or non-naïve-like cells, or distinguishes one from the other; and
(b) introducing a nucleic acid encoding a genetically engineered recombinant receptor into the stimulated composition of T cells, wherein the introducing is carried out during at least a portion of the incubating.

2. The method of claim 1, wherein the incubating is carried out for at least 3 days, at least 4 days, at least 5 days, or at least 6 days.

3. The method of claim 1 or claim 2, wherein the naïve-like T cells or the naïve-like CD8+ cells are CD45RA+, CD27+, CCR7+, and/or CD45RO-.

4. The method of any of claims 1-3, wherein the naïve-like T cells or the naïve-like CD8+ cells are CCR7+CD27+.

5. The method of any of claims 1-4, wherein the recombinant receptor is or comprises a functional non-TCR antigen receptor or a TCR or antigen-binding fragment thereof.

6. The method of any of claims 1-5, wherein the recombinant receptor is a chimeric antigen receptor (CAR).

7. The method of any of claims 1-6, wherein the bead has a diameter of greater than about 3.5 µm but no more than about 9 µm or no more than about 8 µm or no more than about 7 µm or no more than about 6 µm or no more than about 5 µm.

8. The method of any of claims 1-7, wherein the bead has a diameter of about 4.5 µm.

9. The method of any of claims 1-8, wherein the ratio of beads to cells during the incubating is from about 1:1 to about 4:1.

10. The method of any of claims 1-9, wherein the ratio of beads to cells during the incubating is about 3:1.

11. The method of any of claims 1-10, wherein the ratio of beads to cells during the incubating is about 1:1.

12. The method of any of claims 1-11, the sample from which the cells are derived or isolated is blood or a blood-derived sample, or is or is derived from an apheresis or leukapheresis product.

13. The method of any of claims 1-12, wherein the T cells comprise CD4+ and CD8+ T cells and the ratio of CD4+ to CD8+ T cells in the input composition is between about 2:1 and about 1:5 and/or the ratio of the CD4+ cells to the CD8+ cells in the input composition is about 1:1, about 1:2, about 2:1, about 1:3, or about 3:1.

14. The method of any of claims 1-13, wherein the culture-initiating amount of naïve-like T cells or a CD8+ T cell subset thereof is from or from about 2 x 10⁸ to 5 x 10⁸ of the naïve-like T cells or a CD8+ T cell subset thereof
